# EUROPÄISCHE PATENTSCHRIFT

(11) **EP 1 153 016 B1**
(45) Veröffentlichungstag und Bekanntmachung des Hinweises auf die Patenterteilung: **05.09.2007**
(21) Anmeldenummer: 00901606.4
(22) Anmeldetag: 29.01.2000
(51) Int. Cl.: C07D 251/46, C07C 311/39, C07C 311/16, C07C 309/89, A01N 47/36, C07D 239/42, C07D 239/46, C07D 239/52, C07D 251/16, C07D 403/12

(54) **PHENYLSULFONYLHARNSTOFFE, VERFAHREN ZU IHRER HERSTELLUNG UND IHRE VERWENDUNG ALS HERBIZIDE UND PFLANZENWACHSTUMSREGULATOREN**
PHENYLSULFONYL UREAS, METHODS FOR PRODUCING THEM AND THEIR USE AS HERBICIDES AND PLANT GROWTH REGULATORS
PHENYLSULFONYL-UREES, LEUR PROCEDE DE FABRICATION ET LEUR UTILISATION COMME HERBICIDES ET REGULATEURS DE LA CROISSANCE VEGETALE

(30) Priorität: 10.02.1999 DE 19905453
(43) Veröffentlichungstag der Anmeldung: 14.11.2001
(73) Patentinhaber: Bayer CropScience AG, 40789 Monheim (DE)
(72) Erfinder: DIETRICH, Hansjörg, D-65830 Kriftel (DE); WALDRAFF, Christian, D-60318 Frankfurt (DE); WILLMS, Lothar, D-65719 Hofheim (DE); BIERINGER, Hermann, D-65817 Eppstein (DE); ROSINGER, Christopher, D-65719 Hofheim (DE); THÜRWÄCHTER, Felix, D-21465 Reinbeck (DE); AULER, Thomas, D-65812 Bad Soden (DE)
(86) Internationale Anmeldenummer: PCT/EP2000/000718
(87) Internationale Veröffentlichungsnummer: WO 2000/047566

(56) Entgegenhaltungen:
- EP-A- 0 030 138
- EP-A- 0 116 518
- WO-A-94/06778
- CHEMICAL ABSTRACTS, vol. 62, no. 9, 1965 Columbus, Ohio, US; abstract no. 10362e, G.H. HAMOR; M. JANFAZA: "Synthesis of alkyl esters of 4-amino-2-sulfamoylbenzoic acid" Seite 10361; XP002137748 & J. PHARM. SCI., Bd. 52, Nr. 1, 1963, Seiten 102-103,
- DATABASE CAOLD [Online] CHEMICAL ABSTRACTS SERVICE, COLUMBUS, OHIO, US MARKMAN, A. L. ET AL: "polarographic behavior of vanillin" retrieved from STN Database accession no. 56:6669b XP002137750 -& CHEMICAL ABSTRACTS, vol. 56, no. 7, 1962 Columbus, Ohio, US; abstract no. 6669b, Seite 6669; XP002137749 & UZBEK. KHIM. ZHUR., Nr. 4, 1961, Seiten 64-66,
- CLARE BW SUPURAN CT: "Carbonic anhydrase inhibitors. Part 41. Quantitative structure-activity correlations involving kinetic rate constants of 20 sulfonamide inhibitors from a non-congeneric series" EUROPEAN JOURNAL OF MEDICINAL CHEMISTRY.CHIMICA THERAPEUTICA,FR,EDITIONS SCIENTIFIQUE ELSEVIER, PARIS, Bd. 32, Nr. 4, 1. Januar 1997 (1997-01-01), Seiten 311-319, XP004086654 ISSN: 0223-5234
- MANIA D ET AL: "EIN NEUER WEG ZU 2-CYANOBENZOLSULFONAMIDDERIVATEN UND RINGSCHLUSSREAKTIONEN MIT 2-CARBOXAMIDOBENZOLSULFONAMIDEN A NEW APPROACH TO 2-CYANOBENZENESULFONAMIDE DERIVATIVES AND CYCLIZATIONS WITH 2-CARBOXAMIDOBENZENESULFONAMIDES" ARCHIV DER PHARMAZIE,DE,VCH VERLAGSGESELLSCHAFT MBH, WEINHEIM, Bd. 316, 1. Januar 1983 (1983-01-01), Seiten 464-469, XP000613953 ISSN: 0365-6233

## Beschreibung

Phenylsulfonylhamstoffe, Verfahren zu ihrer Herstellung und ihre Verwendung als Herbizide und Pflanzenwachstumsregulatoren

Es ist bekannt, daß substituierte Phenylsulfonylhamstoffe herbizide Eigenschaften besitzen. Dabei handelt es sich um Benzoesäurederivate der allgemeinen Formel A (EP-A-7 687 und EP-A-30 138). Weitere ähnliche Strukturen werden in EP-A-0 116 518 und WO 9 406 778 beschrieben.

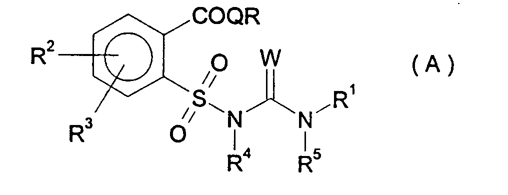

Weiter ist aus DE-4 322 067 bekannt, daß acylaminosubstituierte Phenylsulfonylhamstoffe herbizide Eigenschaften aufweisen.

Überraschenderweise wurden nun spezielle 1,2,3-substituierte Phenylsulfonylhamstoffe gefunden, welche sich besonders gut als Herbizide oder Pflanzenwachstumsregulatoren eignen.

Gegenstand der vorliegenden Erfindung sind somit Verbindungen der Formel (I) oder deren Salze,

Verbindung der Formel (I) oder deren Salze, 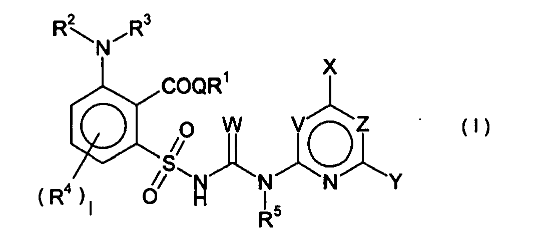 worin
- R¹: H, (C₁-C₆)Alkyl, (C₃-C₆)Alkenyl, (C₃-C₆)Alkinyl, wobei jeder der drei letztgenannten Reste unsubstituiert oder durch einen oder mehrere Reste aus der Gruppe Halogen, unsubstituiertes und substituiertes Phenyl, unsubstituiertes und substituiertes Heterocyclyl mit 3 bis 6 Ringatomen, unsubstituiertes und substituiertes (C₃-C₆)Cycloalkyl, (C₁-C₄)Alkoxy, (C₁-C₄)Alkylthio, [(C₁-C₄)Alkoxy]carbonyl und [(C₁-C₄)Haloalkoxy]carbonyl, substituiert ist, oder unsubstituiertes oder substituiertes (C₃-C₆)Cycloalkyl, substituiertes oder unsubstituiertes (C₃-C₆)Cycloalkenyl, unsubstituiertes oder substituiertes Phenyl, unsubstituiertes oder substituiertes Heterocyclyl mit 3 bis 6 Ringatomen,
wobei substituiertes Phenyl, substituiertes Heterocyclyl, substituiertes Cycloalkyl oder substituiertes Cycloalkenyl als Substituenten einen oder mehrere Reste aus der Gruppe Halogen, (C₁-C₄)Alkyl, (C₁-C₄)Haloalkyl, (C₁-C₄)Alkoxy (C₁-C₄)alkyl, Di[(C₁-C₄)alkoxy](C₁-C₄)alkyl, (C₁-C₄)Haloalkoxy, (C₁-C₄) Alkylthio, (C₁-C₄)Alkylsulfinyl, (C₁-C₄)Haloalkylsulfinyl, (C₁-C₄)Alkylsulfonyl, (C₁-C₄)Haloalkylsulfonyl, NR⁸R⁹, [(C₁-C₄)Alkoxy]carbonyl, [(C₁-C₄)Haloalkoxy]carbonyl, [(C₁-C₄)Alkyl]carbonyl, OH, Phenyl, CN und NO₂ trägt und
- R²: eine Gruppe der Formel R⁰-Q⁰-,
worin R⁰ ein Wasserstoffatom, (C₁-C₁₂)Alkyl, (C₂-C₁₂)Alkenyl oder (C₂-C₁₂)Alkinyl, wobei jeder der letztgenannten drei Reste unsubstituiert oder durch einen oder mehrere Reste aus der Gruppe Halogen, (C₁-C₆)Alkoxy, (C₁-C₆)Haloalkoxy, (C₁-C₆)Alkylthio, (C₁-C₆)Haloalkylthio, (C₁-C₆)Alkylsulfinyl, (C₁-C₆)Haloalkylsulfinyl, (C₁-C₆)Alkylsulfonyl, (C₁-C₆)Haloalkylsulfonyl, [(C₁-C₆)Alkoxy]carbonyl, [(C₁-C₆)Haloalkoxy]carbonyl, CONR⁶R⁷, SO₂NR⁶R⁷, CN, OH, SH, (C₃-C₆)Cycloalkyl, NR⁸R⁹, unsubstituiertes oder substituiertes Phenyl, unsubstituiertes oder substituiertes Heterocyclyl substituiert ist, oder
(C₃-C₆)Cycloalkyl, (C₃-C₆)Cycloalkenyl, Phenyl oder Heterocyclyl, vorzugsweise mit 3 bis 6 Ringatomen, wobei die vier zuletzt genannten Reste unsubstituiert oder substituiert sein können, bedeutet und
worin Q⁰ eine direkte Bindung oder eine divalente Gruppe der Formel -O- oder -N(R^{#})- bedeutet, wobei R^{#} ein Wasserstoffatom, ein Acylrest oder (C₁-C₁₂)Alkyl, (C₂-C₁₂)Alkenyl oder (C₂-C₁₂)Alkinyl, wobei jeder der letztgenannten 3 Reste unsubstituiert oder durch einen oder mehrere Reste aus der Gruppe Halogen, (C₁-C₆)Alkoxy, (C₁-C₆)Haloalkoxy, (C₁-C₆) Alkylthio, (C₁-C₆) Haloalkylthio, CN, OH, (C₃-C₆)Cycloalkyl,
unsubstituiertes oder substituiertes Phenyl, unsubstituiertes oder substituiertes Heterocyclyl substituiert ist, oder unsubstituiertes oder substituiertes (C₃-C₆)Cycloalkyl, unsubstituiertes oder substituiertes (C₃-C₆)Cycloalkenyl, oder
unsubstituiertes oder substituiertes Phenyl bedeutet, und R⁰ und R^{#} gemeinsam mit dem N-Atom der NR^{#}R°-Gruppe einen Heterocyclylrest, vorzugsweise mit 3 bis 6 Ringatomen bilden können, der unsubstituiert oder substituiert ist, vorzugsweise durch einen oder mehrere Reste aus der Gruppe Halogen, (C₁-C₆)Alkyl, (C₁-C₆)Alkoxy, [(C₁-C₆)Alkoxy]carbonyl, (C₁-C₆)Haloalkyl und Oxo,
- R³: ein Wasserstoffatom, (C₁-C₁₂)Alkyl, (C₂-C₁₂)Alkenyl oder (C₂-C₁₂)Alkinyl, wobei jeder der letztgenannten 3 Reste unsubstituiert oder durch einen oder mehrere Reste aus der Gruppe Halogen, (C₁-C₆)Alkoxy, (C₁-C₆)Haloalkoxy, (C₁-C₆)-Alkylthio, (C₁-C₆)-Haloalkylthio, (C₁-C₆)Alkylsulfinyl, (C₁-C₆)Haloalkylsulfinyl, (C₁-C₆)Alkylsulfonyl, (C₁-C₆)Haloalkylsulfonyl,
[(C₁-C₆)Alkoxy]carbonyl, [(C₁-C₆)Haloalkoxy]carbonyl, CONR⁶R⁷ SO₂NR⁶R⁷, CN, OH, (C₃-C₆)Cycloalkyl, NR⁸R⁹, unsubstituiertes oder substituiertes Phenyl, unsubstituiertes oder substituiertes Heterocyclyl substituiert ist, oder unsubstituiertes oder substituiertes (C₃-C₆)Cycloalkyl, unsubstituiertes oder substituiertes (C₃-C₆)Cycloalkenyl, unsubstituiertes oder substituiertes Heterocyclyl, vorzugsweise mit 3 bis 6 Ringatomen, oder unsubstituiertes oder substituiertes Phenyl bedeutet, und
- R² und R³: gemeinsam mit dem N-Atom der NR²R³-Gruppe (N¹) einen Heterocyclylrest, vorzugsweise mit 3 bis 6 Ringatomen bilden können, der unsubstituiert oder substituiert ist, vorzugsweise durch einen oder mehrere Reste aus der Gruppe Halogen, (C₁-C₆)Alkyl, (C₁-C₆)Alkoxy, [(C₁-C₆)Alkoxy]carbonyl, (C₁-C₆)Haloalkyl und Oxo, wobei sich der Oxo-Rest vorzugsweise nicht in Nachbarposition zum N-Atom (N¹) befindet, und
- R⁴: unabhängig voneinander Halogen, CN, (C₁-C₄)Alkyl, (C₁-C₄)Alkoxy, (C₂-C₆)Alkenyl, (C₂-C₆)Alkinyl, (C₃-C₆)Alkenyloxy, (C₃-C₆)Alkinyloxy, wobei jeder der letztgenannten 6 Reste unsubstituiert oder durch einen oder mehrere Reste aus der Gruppe Halogen, (C₁-C₆)Alkoxy, (C₁-C₆)Haloalkoxy, (C₁-C₆)-Alkylthio, (C₁-C₆)-Haloalkylthio, (C₁-C₆)Alkylsulfinyl, (C₁-C₆)Haloalkylsulfinyl, (C₁-C₆)Alkylsulfonyl, (C₁-C₆)Haloalkylsulfonyl,
[(C₁-C₆)Alkoxy]carbonyl, [(C₁-C₆)Haloalkoxy]carbonyl, CONR⁶R⁷, SO₂NR⁶R⁷, CN, OH, (C₃-C₆)Cycloalkyl, NR⁸R⁹, unsubstituiertes oder substituiertes Phenyl oder unsubstituiertes oder substituiertes Heterocyclyl, substituiert ist, oder
unsubstituiertes oder substituiertes (C₃-C₆)Cycloalkyl, unsubstituiertes oder substituiertes (C₃-C₆)Cycloalkenyl, unsubstituiertes oder substituiertes Heterocyclyl, vorzugsweise mit 3 bis 6 Ringatomen, unsubstituiertes oder substituiertes Phenyl, [(C₁-C₄)Alkyl]carbonyl oder [(C₁-C₄)Alkoxy]carbonyl bedeuten, wobei jeder der letztgenannten beiden Reste unsubstituiert oder im Alkylteil durch ein oder mehrere Halogenatome substituiert ist, oder ein Rest der Formel C(O)-NR'-R", C(S)-NR'-R", CR'=N-Q¹-R", S(O)ₘ-Q¹-R"", P(O)ₙ(-Q¹-R""Q²-R"), NR'-Q¹-R" oder NR"'-N=CR'-R" bedeuten, wobei R', R" und R"' unabhängig voneinander ein Wasserstoffatom, einen Acylrest oder einen unsubstituierten oder substituierten (C₁-C₁₀)Kohlenstoffwasserstoffrest, R"" einen kohlenstoffhaltigen Acylrest oder einen unsubstituierten oder substituierten (C₁-C₁₀)Kohlenstoffwasserstoffrest, und Q¹ und Q² unabhängig voneinander eine direkte Bindung oder eine divalente Gruppe der Formel -O- oder -N(R⁺)- bedeuten, wobei R⁺ ein Wasserstoffatom, ein Acylrest oder ein unsubstituierter oder substituierter (C₁-C₁₀)Kohlenwasserstoffrest bedeutet, und m = 0, 1, 2 oder 3, sowie n = 0, 1 oder 2 sein kann, und R' mit R", R⁺ mit R', R⁺ mit R" oder R⁺ mit R"" jeweils einen Heterocyclylrest, vorzugsweise mit 3 bis 6 Ringatomen bilden kann,
der unsubstituiert oder substituiert ist, vorzugsweise durch einen oder mehrere Reste aus der Gruppe Halogen, (C₁-C₆)Alkyl, (C₁-C₆)Alkoxy, [(C₁-C₆)Alkoxy]carbonyl, (C₁-C₆)Haloalkyl und Oxo,
- I: gleich 0, 1 oder 2, vorzugsweise 0 oder 1 ist,
- R⁵: H oder C₁-C₄(Alkyl) ist, das unsubstituiert oder substituiert ist,
- R⁶ und R⁷: unabhängig voneinander H, (C₁-C₆)Alkyl, (C₃-C₆)Alkenyl, (C₃-C₆)Alkinyl, unsubstituiertes oder substituiertes Phenyl oder unsubstituiertes oder substituiertes Heterocyclyl bedeuten oder R⁶ und R⁷ gemeinsam mit dem N-Atom der NR⁶R⁷-Gruppe einen Heterocyclylrest mit 5 oder 6 Ringatomen bilden können, der gegebenenfalls eines oder mehrere zusätzliche Heteroatome aus der Gruppe N, O und S enthalten kann und unsubstituiert oder ein- oder mehrfach durch Reste aus der Gruppe (C₁-C₄)Alkyl und Oxo substituiert ist, und
- R⁸ und R⁹: unabhängig voneinander (C₁-C₄)Alkylcarbonyl, (C₁-C₄)Haloalkylcarbonyl, (C₁-C₄)Alkoxycarbonyl oder (C₁-C₄)Alkylsulfonyl bedeuten oder gemeinsam mit dem N-Atom der NR⁸R⁹-Gruppe einen Heterocyclylrest mit 5 oder 6 Ringatomen bilden können, der gegebenenfalls eines oder mehrere zusätzliche Heteroatome aus der Gruppe N, O und S enthalten kann und unsubstituiert oder ein- oder mehrfach durch Reste aus der Gruppe (C₁-C₄)Alkyl und Oxo substituiert ist, und
- Q: O, S oder NR* ist,
- R*: (C₁-C₄)Alkyl, (C₃-C₄)Alkenyl oder (C₃-C₄)Alkinyl bedeutet, wobei jeder der drei letztgenannten Reste unsubstituiert oder durch einen oder mehrere Reste aus der Gruppe Halogen, (C₁-C₄)Alkoxy und (C₁-C₄)Alkylthio substituiert ist, und R* und R¹ gemeinsam mit dem N-Atom der NR*R¹-Gruppe einen Heterocyclylrest bilden können, der unsubstituiert oder substituiert ist, vorzugsweise durch oder mehrere Reste aus der Gruppe Halogen, (C₁-C₆)Alkyl, (C₁-C₆)Alkoxy, [(C₁-C₆)Alkoxy]carbonyl, (C₁-C₆)Haloalkyl und Oxo,
- X, Y: unabhängig voneinander H, Halogen, (C₁-C₄)Alkyl, (C₁-C₄)Alkoxy, (C₁-C₄)Alkylthio, wobei jeder der drei letztgenannten Reste unsubstituiert oder durch einen oder mehrere Reste aus der Gruppe Halogen, (C₁-C₄)Alkoxy und (C₁-C₄)Alkylthio substituiert ist, Mono- oder Di[(C₁-C₄)alkyl]amino, (C₃-C₅)Alkenyl, (C₃-C₅)Alkenyloxy, (C₃-C₅)Alkinyl oder (C₃-C₅)Alkinyloxy bedeuten, und
wobei die Reste R¹, R², R³ und R⁴ inklusive Substituenten bis zu 20 C-Atomen aufweisen.
- W: ein Sauerstoff oder Schwefelatom
- V, Z: anabhängig voneinander CH oder N bedeuten

Die Verbindungen der Formel (I) können Salze bilden, bei denen der Wasserstoff der -SO₂-NH-Gruppe durch ein für die Landwirtschaft geeignetes Kation ersetzt ist. Diese Salze sind beispielsweise Metallsalze, insbesondere Alkalimetallsalze oder Erdalkalimetallsalze, insbesondere Natrium- und Kaliumsalze, oder auch Ammoniumsalze oder Salze mit organischen Aminen. Ebenso kann Salzbildung durch Anlagerung einer Säure an basischen Gruppen, wie z.B. Amino und Alkylamino, erfolgen. Geeignete Säuren hierfür sind starke anorganische und organische Säuren, beispielsweise HCl, HBr, H₂SO₄ oder HNO₃.

In Formel (I) können jeweils die beiden Reste R⁰ und R^{#}, R² und R³ und R* und R¹ miteinander einen stickstoffhaltigen heterocyclischen Ring bilden.

Kohlenstoffhaltige Reste sind organische Reste, die mindestens ein Kohlenstoffatom, vorzugsweise 1 bis 30 C-Atome, besonders bevorzugt 1 bis 20 C-Atome und außerdem mindestens ein Atom eines oder mehrerer anderer Elemente des Periodensystems der Elemente wie H, Si, N, P, O, S, F, Cl, Br oder J enthalten. Beispiele für kohlenstoffhaltige Reste sind unsubstituierte oder substituierte Kohlenwasserstoffreste, die direkt oder über ein Heteroatom wie N, S, P oder O an den Grundkörper gebunden sein können, unsubstituierte oder substituierte Heterocyclylreste, kohlenstoffhaltige Acylreste oder Cyano.

In Formel (I) und allen nachfolgenden Formeln können die kohlenstoffhaltigen Reste wie Alkyl, Alkoxy, Haloalkyl, Haloalkoxy, Alkylamino und Alkylthio sowie die entsprechenden ungesättigten und/oder substituierten Reste im Kohlenstoffgerüst jeweils geradkettig oder verzweigt sein. Wenn nicht speziell angegeben, sind bei diesen Resten die niederen Kohlenstoffgerüste, z.B. mit 1 bis 6 C-Atomen bzw. bei ungesättigten Gruppen mit 2 bis 6 C-Atomen, bevorzugt. Alkylreste, auch in den zusammengesetzten Bedeutungen wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste; Alkenyl bedeutet z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl; Alkinyl bedeutet z.B. Propargyl, But-2-in-1-yl, But-3-in-1-yl, 1-Methyl-but-3-in-1-yl.

Alkenyl in der Form (C₃-C₄)Alkenyl, (C₃-C₅)Alkenyl, (C₃-C₆)Alkenyl, (C₃-C₈)Alkenyl oder (C₃-C₁₂)-Alkenyl bedeutet vorzugsweise einen Alkenylrest mit 3 bis 4, 3 bis 5, 3 bis 6, 3 bis 8 bzw. 3 bis 12 C-Atomen, bei dem die Doppelbindung nicht an dem C-Atom liegt, das mit dem übrigen Molekülteil der Verbindung (I) verbunden ist ("yl"-Position). Entsprechendes gilt für (C₃-C₄)Alkinyl etc., (C₃-C₄)Alkenyloxy etc. und (C₃-C₄)Alkinyloxy etc.

Cycloalkyl bedeutet ein carbocyclisches, gesättigtes Ringsystem mit 3-8 C-Atomen, z.B. Cyclopropyl, Cyclobutyl, Cyclopentyl oder Cyclohexyl.

Kohlenstofffreie stickstoffhaltige Reste sind Reste die vorzugsweise 1 bis 10 N-Atome, besonders bevorzugt 1 oder 2 N-Atome enthalten und außerdem vorzugsweise eines oder mehrere Atome eines oder mehrerer von Kohlenstoff verschiedener Elemente des Periodensystems der Elemente wie H, O oder S enthalten. Beispiele für kohlenstofffreie stickstoffhaltige Reste sind NH₂, NO₂, NHOH, NO, NH-NH₂ oder N₃.

Halogen bedeutet beispielsweise Fluor, Chlor, Brom oder Iod. Haloalkyl, -alkenyl und -alkinyl bedeuten durch Halogen, vorzugsweise durch Fluor, Chlor und/oder Brom, insbesondere durch Fluor oder Chlor, teilweise oder vollständig substituiertes Alkyl, Alkenyl bzw. Alkinyl, z.B. CF₃, CHF₂, CH₂F, CF₃CF₂, CH₂FCHCl, CCl₃, CHCl₂, CH₂CH₂Cl; Haloalkoxy ist z.B. OCF₃, OCHF₂, OCH₂F, CF₃CF₂O, OCH₂CF₃ und OCH₂CH₂Cl; entsprechendes gilt für Haloalkenyl und andere durch Halogen substituierte Reste.

Ein Kohlenwasserstoffrest ist ein geradkettiger, verzweigter oder cyclischer und gesättigter oder ungesättigter aliphatischer oder aromatischer Kohlenwasserstoffrest, z.B. Alkyl, Alkenyl, Alkinyl, Cycloalkyl, Cycloalkenyl oder Aryl; Aryl bedeutet dabei ein mono-, bi- oder polycyclisches aromatisches System, beispielsweise Phenyl, Naphthyl, Tetrahydronaphthyl, Indenyl, Indanyl, Pentalenyl, Fluorenyl und ähnliches, vorzugsweise Phenyl;
vorzugsweise bedeutet ein Kohlenwasserstoffrest Alkyl, Alkenyl oder Alkinyl mit bis zu 12 C-Atomen oder Cycloalkyl mit 3, 4, 5, 6 oder 7 Ringatomen oder Phenyl.

Ein heterocyclischer Rest oder Ring (Heterocyclyl) kann gesättigt, ungesättigt oder heteroaromatisch und unsubstituiert oder substituiert sein; er enthält vorzugsweise ein oder mehrere Heteroatome im Ring, vorzugsweise aus der Gruppe N, O und S; vorzugsweise ist er ein aliphatischer Heterocyclylrest mit 3 bis 7 Ringatomen oder ein heteroaromatischer Rest mit 5 oder 6 Ringatomen und enthält 1, 2 oder 3 Heteroatome. Der heterocyclische Rest kann z.B. ein heteroaromatischer Rest oder Ring (Heteroaryl) sein, wie z.B. ein mono-, bi- oder polycyclisches aromatisches System, in dem mindestens 1 Ring ein oder mehrere Heteroatome enthält, beispielsweise Pyridyl, Pyrimidinyl, Pyridazinyl, Pyrazinyl, Triazinyl, Thienyl, Thiazolyl, Oxazolyl, Furyl, Pyrrolyl, Pyrazolyl und Imidazolyl, oder ist ein partiell oder vollständig hydrierter Rest wie Oxiranyl, Oxetanyl, Pyrrolidyl, Piperidyl, Piperazinyl, Dioxolanyl, Morpholinyl, Tetrahydrofuryl. Als Substituenten für einen substituierten heterocyclischen Rest kommen die weiter unten genannten Substituenten in Frage, zusätzlich auch Oxo. Die Oxogruppe kann auch an den Heteroringatomen, die in verschiedenen Oxidationsstufen existieren können, z.B. bei N und S, auftreten.

Substituierte Reste, wie substituierte Kohlenwasserstoffreste, z.B. substituiertes Alkyl, Alkenyl, Alkinyl, Aryl, Phenyl und Benzyl, oder substituiertes Heterocyclyl oder Heteroaryl, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Haloalkoxy, Alkylthio, Hydroxy, Amino, Nitro, Carboxy, Cyano, Azido, Alkoxycarbonyl, Alkylcarbonyl, Formyl, Carbamoyl, Mono- und Dialkylaminocarbonyl, substituiertes Amino, wie Acylamino, Mono- und Dialkylamino, und Alkylsulfinyl, Haloalkylsulfinyl, Alkylsulfonyl, Haloalkylsulfonyl und, im Falle cyclischer Reste, auch Alkyl und Haloalkyl sowie den genannten gesättigten kohlenwasserstoffhaltigen Resten entsprechende ungesättigte aliphatische Reste, wie Alkenyl, Alkinyl, Alkenyloxy, Alkinyloxy etc. bedeuten. Bei Resten mit C-Atomen sind solche mit 1 bis 4 C-Atomen, insbesondere 1 oder 2 C-Atomen, bevorzugt. Bevorzugt sind in der Regel Substituenten aus der Gruppe Halogen, z.B. Fluor und Chlor, (C₁-C₄)Alkyl, vorzugsweise Methyl oder Ethyl, (C₁-C₄)Haloalkyl, vorzugsweise Trifluormethyl, (C₁-C₄)Alkoxy, vorzugsweise Methoxy oder Ethoxy, (C₁-C₄)Haloalkoxy, Nitro und Cyano. Besonders bevorzugt sind dabei die Substituenten Methyl, Methoxy und Chlor.

Gegebenenfalls substituiertes Phenyl ist vorzugsweise Phenyl, das unsubstituiert oder ein- oder mehrfach, vorzugsweise bis zu dreifach durch gleiche oder verschiedene Reste aus der Gruppe Halogen, (C₁-C₄)Alkyl, (C₁-C₄)Alkoxy, (C₁-C₄)Halogenalkyl, (C₁-C₄)Halogenalkoxy und Nitro substituiert ist, z.B. o-, m- und p-Tolyl, Dimethylphenyle, 2-, 3- und 4-Chlorphenyl, 2-, 3- und 4-Trifluor- und -Trichlorphenyl, 2,4-, 3,5-, 2,5- und 2,3-Dichlorphenyl, o-, m- und p-Methoxyphenyl.

Mono- oder disubstituiertes Amino bedeutet einen chemisch stabilen Rest aus der Gruppe der substituierten Aminoreste, welche beispielsweise durch einen bzw. zwei gleiche oder verschiedene Reste aus der Gruppe Alkyl, Alkoxy, Acyl und Aryl N-substituiert sind; vorzugsweise Monoalkylamino, Dialkylamino, Acylamino, Arylamino, N-Alkyl-N-arylamino sowie N-Heterocyclen; dabei sind Alkylreste mit 1 bis 4 C-Atomen bevorzugt; Aryl ist dabei vorzugsweise Phenyl oder substituiertes Phenyl; für Acyl gilt dabei die weiter unten genannte Definition, vorzugsweise Formyl, (C₁-C₄)Alkylcarbonyl oder (C₁-C₄)Alkylsulfonyl. Entsprechenes gilt für substituiertes Hydroxylamino oder Hydrazino.

Ein Acylrest bedeutet den Rest einer organischen Säure, der formal durch Abspaltung einer OH-Gruppe aus der organischen Säure entsteht, z.B. der Rest einer Carbonsäure und Reste davon abgeleiteter Säuren wie der Thiocarbonsäure, gegebenenfalls N-substituierten Iminocarbonsäuren oder die Reste von Kohlensäuremonoestern, gegebenenfalls N-substituierter Carbaminsäuren, Sulfonsäuren, Sulfinsäuren, Phosphonsäuren, Phosphinsäuren.

Ein Acylrest ist bevorzugt Formyl oder aliphatisches Acyl aus der Gruppe CO-R^{x}, CS-R^{x}, CO-OR^{x}, CS-OR^{x}, CS-SR^{x}, SOR^{Y} oder SO₂R^{Y}, wobei R^{x} und R^{Y} jeweils einen C₁-C₁₀-Kohlenwasserstoffrest bedeutet, der unsubstituiert oder substituiert ist, oder Aminocarbonyl oder Aminosulfonyl, wobei die beiden letztgenannten Reste unsubstituiert, N-monosubstituiert oder N,N-disubstituiert sind. Acyl bedeutet beispielsweise Formyl, Halogenalkylcarbonyl, Alkylcarbonyl wie (C₁-C₄)Alkylcarbonyl, Phenylcarbonyl, wobei der Phenylring substituiert sein kann, z.B. wie oben für Phenyl angegeben, oder Alkyloxycarbonyl, Phenyloxycarbonyl, Benzyloxycarbonyl, Alkylsulfonyl, Alkylsulfinyl, N-Alkyl-1-iminoalkyl und andere Reste von organischen Säuren.

Gegenstand der Erfindung sind auch alle Stereoisomeren, die von Formel (I) umfaßt sind, und deren Gemische. Solche Verbindungen der Formel (I) enthalten ein oder mehrere asymmetrische C-Atome oder auch Doppelbindungen, die in der allgemeinen Formel (1) nicht gesondert angegeben sind. Die durch ihre spezifische Raumform definierten möglichen Stereoisomeren, wie Enantiomere, Diastereomere, Z- und E-Isomere sind alle von der Formel (I) umfaßt und können nach üblichen Methoden aus Gemischen der Stereoisomeren erhalten oder auch durch stereoselektive Reaktionen in Kombination mit dem Einsatz von stereochemisch reinen Ausgangsstoffen hergestellt werden.
Die vorstehenden Beispiele für Reste oder Restebereiche, die unter die allgemeinen Begriffe wie "Alkyl", "Acyl", "substituierten Reste" etc., fallen, bedeuten keine vollständige Aufzählung. Die allgemeinen Begriffe umfassen auch die weiter unten angeführten Definitionen für Restebereiche in Gruppen bevorzugter Verbindungen, insbesondere Restebereiche, welche spezifische Reste aus den Tabellenbeispielen umfassen.

Vor allem aus Gründen der höheren herbiziden Wirkung, besseren Selektivität und/oder besseren Herstellbarkeit sind erfindungsgemäße Verbindungen der Formel (I) oder deren Salze von besonderem Interesse,
worin
- R¹: H, (C₁-C₆)Alkyl, (C₃-C₆)Alkenyl oder (C₃-C₆)Alkinyl, wobei jeder der drei letztgenannten Reste unsubstituiert oder durch einen oder mehrere Reste aus der Gruppe Halogen, Phenyl, (C₁-C₄)Alkoxy, (C₁-C₄)Alkylthio (C₃-C₆)Cycloalkyl, Heterocyclyl mit 3 bis 6 Ringatomen, und [(C₁-C₄)Alkoxy]-carbonyl substituiert ist, oder (C₃-C₆)Cycloalkyl oder Heterocyclyl mit 3 bis 6 Ringatomen bedeutet, wobei jeder der letztgenannten beiden Reste unsubstituiert oder durch einen oder mehrere Reste aus der Gruppe Halogen, (C₁-C₄)Alkyl und (C₁-C₄)Alkoxy substituiert ist,
- R²: eine Gruppe der Formel R⁰-Q⁰-, worin
R⁰ ein Wasserstoffatom, (C₁-C₈)Alkyl, (C₃-C₈)Alkenyl oder (C₃-C₈)Alkinyl, wobei jeder der letztgenannten 3 Reste unsubstituiert oder durch einen oder mehrere Reste aus der Gruppe Halogen (C₁-C₄)Alkoxy, (C₁-C₄)Haloalkoxy, (C₁-C₄)Alkylthio, (C₁-C₄)Haloalkylthio, (C₁-C₄)Alkylsulfinyl, (C₁-C₄)Haloalkylsulfinyl, (C₁-C₄)Alkylsulfonyl, (C₁-C₄)Haloalkylsulfonyl, [(C₁-C₆)Alkoxy]carbonyl, CONR⁶R⁷, SO₂NR⁶R⁷, CN, OH, (C₃-C₆)Cycloalkyl, NR⁸R⁹ und Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylsulfinyl, (C₁-C₄)Alkylsulfonyl, NR⁸R⁹, [(C₁-C₄)Alkoxy]carbonyl, [(C₁-C₄)Alkyl]carbonyl, Phenyl, [(C₁-C₄)Alkyl]-carbonyl, CN und NO₂ substituiert ist, substituiert ist, oder Heterocyclyl mit 3 bis 6 Ringatomen, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylsulfinyl, (C₁-C₄)Alkylsulfonyl, NR⁸R⁹, [(C₁-C₄)Alkoxy]carbonyl, [(C₁-C₄)Alkyl]carbonyl, Phenyl, CN und NO₂ substituiert ist, oder (C₃-C₆)Cycloalkyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)-Haloalkoxy, [(C₁-C₄)Alkoxy]carbonyl, CN, OH und Phenyl substituiert ist, oder
(C₃-C₆)Cycloalkenyl, das unsubstituiert oder durch einen oder mehrere Reste aus der Gruppe Halogen, (C₁-C₄)Alkyl, (C₁-C₄)Haloalkyl, (C₁-C₄)Alkoxy und [(C₁-C₄)Alkoxy]carbonyl substituiert ist, oder
Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylsulfinyl, (C₁-C₄)Alkylsulfonyl, NR⁸R⁹, [(C₁-C₄)Alkoxy]carbonyl, [(C₁-C₄)Alkyl]carbonyl, Phenyl, CN und NO₂ substituiert ist, und
Q⁰ eine direkte Bindung oder eine divalente Gruppe der Formel -O- oder -NR^{#}, worin R^{#} ein Wasserstoffatom oder unsubstituiertes oder substituiertes (C₁-C₄)Alkyl ist, bedeutet,
- R³: ein Wasserstoffatom, (C₁-C₈)Alkyl, (C₃-C₈)Alkenyl oder (C₃-C₈)Alkinyl, wobei jeder der letztgenannten 3 Reste unsubstituiert oder durch einen oder mehrere Reste aus der Gruppe Halogen (C₁-C₄)Alkoxy, (C₁-C₄)Haloalkoxy, (C₁-C₄)Alkylthio, (C₁-C₄)Haloalkylthio, (C₁-C₄)Alkylsulfinyl, (C₁-C₄)Haloalkylsulfinyl, (C₁-C₄)Alkylsulfonyl, (C₁-C₄)Haloalkylsulfonyl, [(C₁-C₆)Alkoxy]carbonyl, CONR⁶R⁷, SO₂NR⁶R⁷, CN, OH, (C₃-C₆)Cycloalkyl, NR⁸R⁹ und Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylsulfinyl, (C₁-C₄)Alkylsulfonyl, NR⁸R⁹, [(C₁-C₄)Alkoxy]carbonyl, [(C₁-C₄)Alkyl]carbonyl, Phenyl, [(C₁-C₄)Alkyl]carbonyl, CN und NO₂ substituiert ist, substituiert ist, oder Heterocyclyl, vorzugsweise mit 3 bis 6 Ringatomen, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylsulfinyl, (C₁-C₄)Alkylsulfonyl, NR⁸R⁹, [(C₁-C₄)Alkoxy]carbonyl, [(C₁-C₄)Alkyl]carbonyl, Phenyl, CN und NO₂ substituiert ist, substituiert ist, oder (C₃-C₆)Cycloalkyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, [(C₁-C₄)Alkoxy]carbonyl, CN, OH und Phenyl substituiert ist, oder
(C₃-C₆)Cycloalkenyl, das unsubstituiert oder durch einen oder mehrere Reste aus der Gruppe Halogen, (C₁-C₄)Alkyl, (C₁-C₄)Haloalkyl, (C₁-C₄)Alkoxy und [(C₁-C₄)Alkoxy]carbonyl substituiert ist, oder
Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylsulfinyl, (C₁-C₄)Alkylsulfonyl, NR⁸R⁹, [(C₁-C₄)Alkoxy]carbonyl, [(C₁-C₄)Alkyl]carbonyl, Phenyl, CN und NO₂ substituiert ist, und
- R² und R³: gemeinsam mit dem N-Atom (N¹) einen Heterocyclylrest mit 3-6 Ringatomen bilden können, der gesättigt, ungesättigt oder heteroaromatisch ist und zusätzlich zum N-Atom (N¹) ein oder zwei Heteroatome aus der Gruppe N, O und S enthalten kann und unsubstituiert oder durch einen oder mehrere Reste aus der Gruppe (C₁-C₆)Alkyl, vorzugsweise (C₁-C₃)Alkyl, (C₁-C₃)Alkoxy, Halogen, [(C₁-C₃)Alkoxy]carbonyl, (C₁-C₃)Haloalkyl und Oxo, wobei sich der Oxo-Rest vorzugsweise nicht in Nachbarposition zum N-Atom (N¹) befindet, substituiert ist,
- R⁴: Halogen, CN, (C₁-C₄)Alkyl, (C₁-C₄)Alkoxy, (C₂-C₆)Alkenyl, (C₂-C₆)-Alkinyl, (C₃-C₆)Alkenyloxy, (C₃-C₆)Alkinyloxy,
wobei jeder der letztgenannten 6 Reste unsubstituiert oder durch einen oder mehrere Reste aus der Gruppe Halogen, (C₁-C₆)Alkoxy, (C₁-C₆)Haloalkoxy, (C₁-C₆)Alkylthio, (C₁-C₆)Haloalkylthio, (C₁-C₆)Alkylsulfinyl, (C₁-C₆)Haloalkylsulfinyl, (C₁-C₆)Alkylsulfonyl, (C₁-C₆)Haloalkylsulfonyl, [(C₁-C₆)Alkoxy]carbonyl, [(C₁-C₆)Haloalkoxy]carbonyl, CONR⁶R⁷, SO₂NR⁶R⁷, CN, OH, (C₃-C₆)Cycloalkyl, NR⁸R⁹, unsubstituiertes oder substituiertes Phenyl, unsubstituiertes oder substituiertes Heterocyclyl substituiert ist, oder
unsubstituiertes oder substituiertes (C₃-C₆)Cycloalkyl, unsubstituiertes oder substituiertes (C₃-C₆)Cycloalkenyl, unsubstituiertes oder substituiertes Heterocyclyl mit 3 bis 6 Ringatomen, unsubstituiertes oder substituiertes Phenyl oder [(C₁-C₄)Alkyl]carbonyl oder [(C₁-C₄)Alkoxy]carbonyl bedeuten, wobei jeder der letztgenannten beiden Reste unsubstituiert oder im Alkylteil durch ein oder mehrere Halogenatome substituiert ist, oder ein Rest der Formel C(O)-NR'-R", C(S)-NR'-R", CR'=N-Q¹-R", NR'-Q¹-R" oder NR"'-N=CR'-R" bedeuten, wobei R', R" und R'" unabhängig voneinander ein Wasserstoffatom, einen Acylrest oder einen unsubstituierten oder substituierten (C₁-C₁₀)Kohlenstoffwasserstoffrest und Q¹ und Q² unabhängig voneinander eine direkte Bindung oder eine divalente Gruppe der Formel -O- oder -N(R⁺)- bedeuten, wobei R⁺ ein Wasserstoffatom, ein Acylrest oder ein unsubstituierter oder substituierter (C₁-C₄)Alkylrest bedeutet, und R' mit R" oder R⁺ mit R' oder R⁺ mit R" jeweils einen Heterocyclylrest mit 3 bis 6 Ringatomen bilden kann, der unsubstituiert oder substituiert ist, vorzugsweise durch einen oder mehrere Reste aus der Gruppe Halogen, (C₁-C₆)Alkyl, (C₁-C₆)Alkoxy, [(C₁-C₆)Alkoxy]carbonyl, (C₁-C₆)Haloalkyl und Oxo,
- I: gleich 0 oder 1 ist,
- R⁶ und R⁷: unabhängig voneinander H, (C₁-C₄)Alkyl, (C₃-C₄)Alkenyl, (C₃-C₄)Alkinyl oder Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Alkylthio, (C₁-C₄)Alkylsulfonyl, [(C₁-C₄)Alkoxy]carbonyl, CN und NO₂ substituiert ist, oder
R⁶ und R⁷ gemeinsam mit dem N-Atom der NR⁶R⁷-Gruppe einen Heterocyclylrest mit 5 oder 6 Ringgliedern bilden können, der gegebenenfalls eines oder mehrere Heteroatome aus der Gruppe N, O und S enthalten kann und unsubstituiert oder ein- oder mehrfach durch Reste aus der Gruppe (C₁-C₄)Alkyl und Oxo substituiert ist,
- R⁸ und R⁹: (C₁-C₄)Alkylcarbonyl, (C₁-C₄)Haloalkylcarbonyl, (C₁-C₄)Alkoxycarbonyl oder (C₁-C₄)Alkylsulfonyl sind, oder gemeinsam mit dem N-Atom der NR⁸R⁹-Gruppe einen Heterocyclylrest mit 5 oder 6 Ringatomen bilden können, der gegebenenfalls eines oder mehrere Heteroatome aus der Gruppe N, O und S enthalten kann und unsubstituiert oder ein- oder mehrfach durch Reste aus der Gruppe (C₁-C₄)Alkyl und Oxo substituiert ist.

Von besonderem Interesse sind auch erfindungsgemäße Verbindungen der Formel (I) und deren Salze, worin
- R¹: (C₁-C₆)Alkyl, das unsubstituiert oder durch einen oder mehrere Reste aus der Gruppe Halogen und (C₁-C₄)Alkoxy substituiert ist, oder 3-Oxetanyl, (C₃-C₄)Alkenyl oder (C₃-C₄)Alkinyl,
- R²: H, (C₁-C₆)Alkyl, (C₃-C₆)Alkenyl, (C₃-C₆)Alkinyl, wobei jeder der letztgenannten drei Reste unsubstituiert oder durch einen oder mehrere Reste aus der Gruppe Halogen, (C₁-C₄)Alkoxy, (C₁-C₄)Alkylthio, (C₁-C₄)Alkylsulfonyl, [(C₁-C₄)Alkoxy]carbonyl, (C₃-C₆)Cycloalkyl, CN und OH substituiert ist, oder (C₃-C₆)Cycloalkyl, das unsubstituiert oder durch einen oder mehrere Reste aus der Gruppe Halogen, (C₁-C₄)Alkyl, (C₁-C₄)Alkoxy, [(C₁-C₄)Alkoxy]carbonyl, CN und OH substituiert ist, oder (C₃-C₆)Cycloalkenyl, (C₁-C₄)Alkoxy, (C₁-C₄)Alkenyloxy, (C₁-C₄)Alkylamino oder Di[(C₁-C₄)alkyl]amino und
- R³: H, (C₁-C₆)Alkyl, (C₃-C₆)Alkenyl, (C₃-C₆)Alkinyl, wobei jeder der letztgenannten drei Reste unsubstituiert oder durch einen oder mehrere Reste aus der Gruppe Halogen, (C₁-C₄)Alkoxy, (C₁-C₄)Alkylthio, (C₁-C₄)Alkylsulfonyl, [(C₁-C₄)Alkoxy]carbonyl, (C₃-C₆)Cycloalkyl, CN und OH substituiert ist, oder (C₃-C₆)Cycloalkyl, das unsubstituiert oder durch einen oder mehrere Reste aus der Gruppe Halogen, (C₁-C₄)Alkyl, (C₁-C₄)Alkoxy, [(C₁-C₄)Alkoxy]carbonyl, CN und OH substituiert ist, oder (C₃-C₆)Cycloalkenyl oder
- R² und R³: gemeinsam mit dem N-Atom (N¹) einen Heterocyclylrest mit 3-6, vorzugsweise 5 oder 6 Ringatomen bilden können, der gesättigt, ungesättigt oder heteroaromatisch ist, zusätzlich zu dem N-Atom (N¹) ein oder zwei Heteroatome aus der Gruppe N, O und S enthalten kann und unsubstituiert oder durch einen oder mehrere Reste aus der Gruppe Halogen, (C₁-C₆)Alkyl, vorzugsweise (C₁-C₃)Alkyl, (C₁-C₃)Alkoxy, [(C₁-C₃)Alkoxy]carbonyl und Oxo, wobei sich der Oxo-Rest vorzugsweise nicht in Nachbarposition zum N-Atom (N¹) befindet, substituiert ist, und
- R⁴: (C₁-C₄)Alkyl, (C₁-C₄)Haloalkyl, (C₁-C₄)Alkoxy oder Halogen,
- I: gleich 0 oder 1 ist, vorzugsweise 0 ist,
- R⁵: H oder Methyl,
- Q: O oder NR*,
- R*: H oder (C₁-C₄)Alkyl,
- X und Y: unabhängig voneinander (C₁-C₄)Alkyl, (C₁-C₄)Alkoxy, wobei jeder der letztgenannten zwei Reste unsubstituiert oder durch einen oder mehrere Halogenatome substituiert ist, oder (C₁-C₄)Alkylthio, Halogen oder Mono- oder Dil(C₁-C₂)alkyl]amino bedeuten, und
- W: ein Sauerstoffatom bedeutet.

Bevorzugte erfindungsgemäße Verbindungen der Formel (I) oder deren Salze sind solche, worin
- R¹: (C₁-C₃)Alkyl, Allyl oder Propargyl,
- R²: H, (C₁-C₄)Alkyl, (C₃-C₅)Alkenyl, (C₃-C₅)Alkinyl, (C₃-C₆)Cycloalkyl oder (C₃-C₆)Cycloalkenyl,
- R³: H, (C₁-C₄)Alkyl, (C₃-C₅)Alkenyl, (C₃-C₅)Alkinyl, (C₃-C₆)Cycloalkyl oder (C₃-C₆)Cycloalkenyl, oder
- R² und: R³ gemeinsam mit dem N-Atom (N¹) einen Heterocyclylrest mit 3-6, vorzugsweise 5 oder 6 Ringatomen bilden können, der gesättigt, ungesättigt oder heteroaromatisch ist, zusätzlich zu dem N-Atom (N¹) ein oder zwei Heteroatome aus der Gruppe N, O und S enthalten kann und unsubstituiert oder durch einen oder mehrere (C₁-C₆)Alkylreste substituiert ist,
- R⁴: (C₁-C₃)Alkyl oder Halogen,
- I: gleich 0 oder 1, vorzugsweise 0 ist,
- Q: O oder NR*,
- R*: (C₁-C₃)Alkyl,
- X: (C₁-C₂)Alkyl, (C₁-C₂)Alkoxy, (C₁-C₂)Alkylthio, (C₁-C₂)Haloalkyl oder (C₁-C₂)Haloalkoxy,
- Y: (C₁-C₂)Alkyl, (C₁-C₂)Alkoxy, Halogen, NHCH₃ oder N(CH₃)₂,
- V: CH oder N, vorzugsweise N und
- Z: CH oder N bedeuten.

Besonders bevorzugte erfindungsgemäße Verbindungen der Formel (I) oder deren Salze sind solche, worin
- R¹: (C₁-C₃)Alkyl, Allyl oder Propargyl und/oder
- Q: ein Sauerstoffatom bedeutet.

Besonders bevorzugt sind auch erfindungsgemäße Verbindungen der Formel (I) und deren Salze, welche eine Kombination von Resten aus den obengenannten Verbindungen von besonderem Interesse bzw. den bevorzugten Verbindungen enthalten, sowie solche, welche einzelne oder mehrere Reste aus den in den Tabellen 1 und 2 (s. u.) aufgeführten Verbindungen enthalten. Ebenfalls bevorzugt sind Verbindungen der Formel (I) in denen V=N ist.

Gegenstand der vorliegenden Erfindung sind auch Verfahren zur Herstellung der erfindungsgemäßen Verbindungen der allgemeinen Formel (I) oder deren Salze, wobei man
a) eine Verbindung der Formel (II) 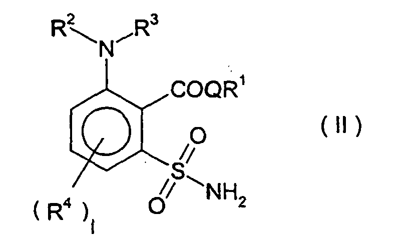 mit einem heterocyclischen Carbamat der Formel (III), 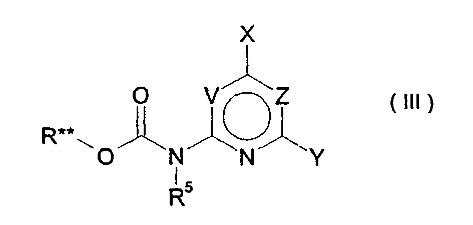 worin R** ein substituierter oder unsubstituierter C₁-C₂₀-Kohlenwasserstoffrest wie Aryl oder Alkyl, vorzugsweise gegebenenfalls substituiertes Phenyl oder gegebenenfalls substituiertes (C₁-C₄)Alkyl bedeutet, umsetzt oder
b) ein Sulfonylcarbamat der Formel (IV), 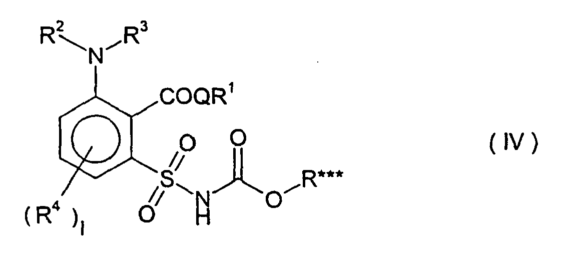 worin R*** ein substituierter oder unsubstituierter C₁-C₂₀-Kohlenwasserstoffrest wie gebenenfalls substituiertes Phenyl oder gegebenenfalls substituiertes (C₁-C₄)Alkyl bedeutet, mit einem Aminoheterocyclus der Formel (V) 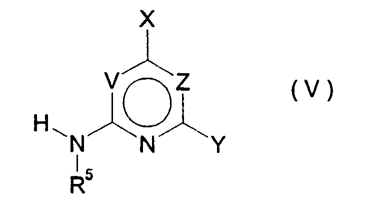 umsetzt oder
c) ein Sulfonylisocyanat der Formel (VI) 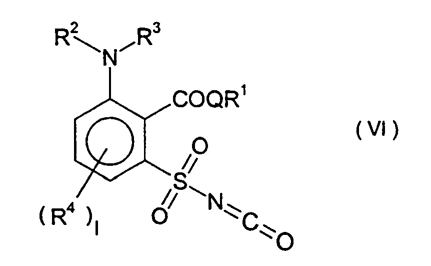 mit einem Aminoheterocyclus der Formel (V) umsetzt oder
d) ein Sulfonamid der Formel (II) mit einem (Thio)-Isocyanat der Formel (VII) 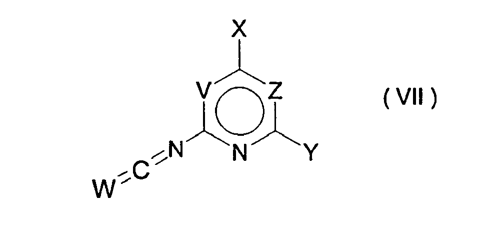 in Gegenwart einer Base umsetzt oder
e) einen Aminoheterocyclus der Formel (V) zunächst basenkatalysiert mit einem Kohlensäureester, z.B. Diphenylcarbonat, umsetzt und das gebildete Intermediat in einer Eintopfreaktion mit einem Sulfonamid der Formel (II) (siehe Variante a) umsetzt, oder
f) einen Phenylsulfonylharnstoff der Formel (VIII) 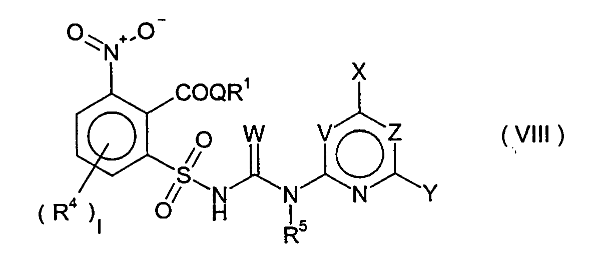 durch Reduktion der Nitrogruppe, und gegebenenfalls weiterer Umsetzung der freigesetzten Hydroxylamino- oder Aminofunktion, zu einem Sulfonylharnstoff der Formel (I) umsetzt,
wobei in den Formeln (II)-(VIII) die Reste, Gruppen bzw. Indizes R¹-R⁵, Q, V, W, X, Y, Z und I wie in Formel (I) definiert sind. In Verfahrensvarianten a) bis c), e) und f) werden zunächst Verbindungen der Formel (I) mit W = O erhalten.

Die Umsetzung der Verbindungen der Formeln (II) und (III) erfolgt vorzugsweise basenkatalysiert in einem inerten organischen Lösungsmittel, wie z.B. Dichlormethan, Acetonitril, Dioxan oder THF bei Temperaturen zwischen 0 °C, vorzugsweise 20 °C, und dem Siedepunkt des Lösungsmittels. Als Base werden dabei beispielsweise organische Aminbasen, wie 1,8-Diazabicyclo[5.4.0]undec-7-en (DBU), Alkali-tert.-butoxide, wie z.B. NaO-t-C₄H₉, oder Alkalihydroxide, wie z.B. NaOH, insbesondere bei R** = (subst.) Phenyl (vgl. EP-A-44 807), oder Trimethylaluminium oder Triethylaluminium, letztere insbesondere bei R** = Alkyl (vgl. EP-A-166 516) verwendet. Die jeweilige Base wird dabei beispielsweise im Bereich von 1 bis 3 Moläquivalenten, bezogen auf die Verbindung der Formel (II) eingesetzt.

Die Sulfonamide (II) und damit strukturell verwandte Verbindungen der Formeln (IV) und (VI) sowie die Phenylsulfonylhamstoffe (VIII) sind neue Verbindungen. Sie und ihre Herstellung sind ebenfalls Gegenstand dieser Erfindung.

Man erhält die Verbindungen der Formel (II) wie in Schema 1 gezeigt beispielsweise ausgehend von Verbindungen der Formel (IX)

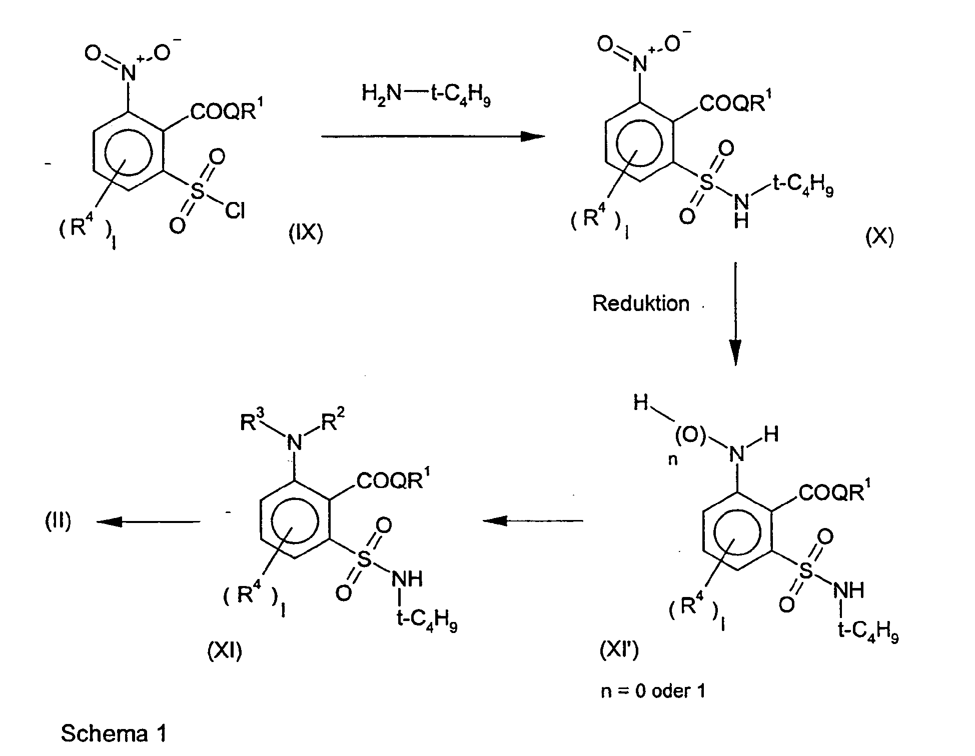

Die Behandlung von Sulfochloriden der Formel (IX) mit tert.-Butylamin ergibt Sulfonamide der Formel (X). Durch Reduktion der Nitrogruppe in den Verbindungen der Formel (X) gelangt man zu den entsprechenden Hydroxylaminen bzw. Aminen (XI') wobei die freigesetzte Funktionalität gegebenenfalls derivatisiert z.B. alkyliert werden kann. Die so erhaltenen Zwischenstufen der Formel (XI) werden schließlich durch Säurebehandlung in die Verbindungen (II) übergeführt. Die Reste R¹ bis R⁴, Q und 1 sind in Schema 1 wie in Formel (I) definiert.
Die Sulfonamidbildung wird beispielsweise in inerten Lösungsmitteln wie z.B. Dichlormethan, Tetrahydrofuran (THF), Dioxan, Toluol oder Dimethylformamid (DMF) bei Temperaturen von -70 °C bis zum Siedepunkt des Lösungsmittels, vorzugsweise bis 25 °C durchgeführt. Dabei kommt bevorzugt eine Aminmenge von 1.5 - 2.5 Äquivalenten bezogen auf Sulfochlorid zum Einsatz.

Die Reduktion der Nitrofunktion in (X) zu Aminen bzw. Hydroxylaminen der allgemeinen Formel (XI') erfolgt analog zu bekannten Methoden (vgl. hierzu Houben-Weyl, "Methoden der Organischen Chemie", 4. Aufl. Bd. Xl/1 S. 360 ff, Thieme Verlag Stuttgart, 1957 sowie Bd. X/1 S. 1138 ff, Thieme Verlag Stuttgart, 1971). Verbindungen der allgemeinen Formel (XI') (wobei n = 0) lassen sich mit Carbonylverbindungen reduktiv alkylieren (vgl. hierzu Houben-Weyl, "Methoden der Organischen Chemie", 4. Aufl. Bd. Xl/1 S. 618-643, Thieme Verlag Stuttgart, 1957; J. Am. Chem. Soc. 96 (1974) 7812-7814; J. Med. Chem. 38 (1995) 3132-3137). Durch nachfolgende tert.-Butylspaltung gelingt so beispielsweise die Darstellung sekundärer und tertiärer Aniline der allgemeinen Formel (II).
Die Abspaltung der tert.-Butyl-Schutzgruppe in den Verbindungen (XI) erfolgt durch Behandlung mit einer starken Säure (siehe WO 89/10921). Als starke Säuren kommen z.B. Mineralsäuren, wie H₂SO₄ oder HCl, oder starke organische Säuren, wie Trifluoressigsäure in Frage. Die Reaktion erfolgt beispielsweise bei Temperaturen von -20 °C bis zur jeweiligen Rückflußtemperatur des Reaktionsgemisches, vorzugsweise bei 0 °C bis 40 °C. Die Umsetzung kann in Substanz oder auch in einem inerten Solvens, wie z.B. Dichlormethan oder Trichlormethan, durchgeführt werden.
Für I = 0, Q = O und R¹ = H, Me, Et und Allyl sind die Verbindungen (IX) bekannt (siehe US-4 647 588, US-4 694 020, EP-A-197 386 und US-4 603 133). Die erfindungsgemäßen neuen Verbindungen der Formel (IX) können ausgehend von kernsubstituierten Phthalsäuren (für I = 0 beispielsweise aus käuflicher 3-Nitrophthalsäure), analog zu bekannten Methoden hergestellt werden (siehe J. Heterocyclic Chem. 23 (1986) 1253-1255).

Die Carbamate der Formel (III) können nach Methoden hergestellt werden, wie sie in den südafrikanischen Patentanmeldungen 82/5671 und 82/5045 bzw. EP-A-70 804 (US-A-4 480 101) oder RD 275 056 beschrieben sind.

Die Umsetzung der Verbindungen (IV) mit den Aminoheterocyclen (V) führt man vorzugsweise in inerten, aprotischen Lösungsmitteln wie z.B. Dioxan, Acetonitril oder Tetrahydrofuran bei Temperaturen zwischen 0 °C und der Siedetemperatur des Lösungsmittels durch. Die Phenylsulfonylcarbamate der Formel (IV) und (XII') erhält man aus Verbindungen der Formel (II) bzw. (XII) analog US-A-4 684 393 oder US-A-4 743 290. Die benötigten Ausgangsmaterialien (V) sind literaturbekannt oder können nach literaturbekannten Verfahren hergestellt werden (siehe hierzu DE-A-38 42 621; Dudley J. R. et al. J. Am. Chem. Soc. 73 (1951) 1968-2990; Braker et al. J. Am. Chem. Soc. 69 (1947) 3072, 3075; Rose et al. J. Chem. Soc. (1946) 81, 84; Huffman, K. R.; Schaefer, F. C. J. Org. Chem. 28 (1963) 1816-1821; Gabriel et al. Chem.Ber. 32 (1899) 2924).

Die Phenylsulfonylisocyanate der Formel (VI) lassen sich analog US-A-4 481 029 herstellen und mit den Aminoheterocyclen (V) umsetzen.

Die (Thio)-Isocyanate der Formel (VII) sind nach literaturbekannten Verfahren erhältlich (EP-A-232 067, EP-A-166 516). Die Umsetzung der (Thio)-Isocyanate (VII) mit Verbindungen (II) erfolgt beispielsweise bei -10 °C bis 100 °C, vorzugsweise bei 20 °C bis 100 °C, in einem inerten aprotischen Lösungsmittel, wie z.B. Aceton oder Acetonitril, in Gegenwart einer geeigneten Base, z.B. Triethylamin oder Kaliumcarbonat.

Die Zwischenprodukte der Formel (VIII) lassen sich beispielsweise unter Umgehung der Isolierung der intermediären Sulfonylisocyanate direkt aus den Sulfochloriden (IX) und den Aminoheterocyclen (V) in Gegenwart eines Alkali- oder Ammoniumcyanats oder -thiocyanats und Pyridin herstellen (vgl. hierzu US-A-5 157 119). Alternativ dazu können die Verbindungen (VIII) auch durch Umsetzung eines Sulfonamids (XII) mit Carbamaten der Formel (III) entsprechend dem unter a) beschriebenen Verfahren gewonnen werden. Ausgehend von Sulfonamiden (XII) können die Zwischenprodukte der Formel (VIII) auch analog zu den unter d) und e) beschriebenen Verfahren hergestellt werden. Außerdem können die Zwischenprodukte der Formel (VIII) auch von Verbindungen der Formel (X*), wobei Z** = NHCOOR*** ist, analog zu dem unter b) beschriebenen Verfahren hergestellt werden.

Die Sulfonamide (XII) können durch Umsetzung der Verbindungen (IX) mit Ammoniak erhalten werden (Schema 2).

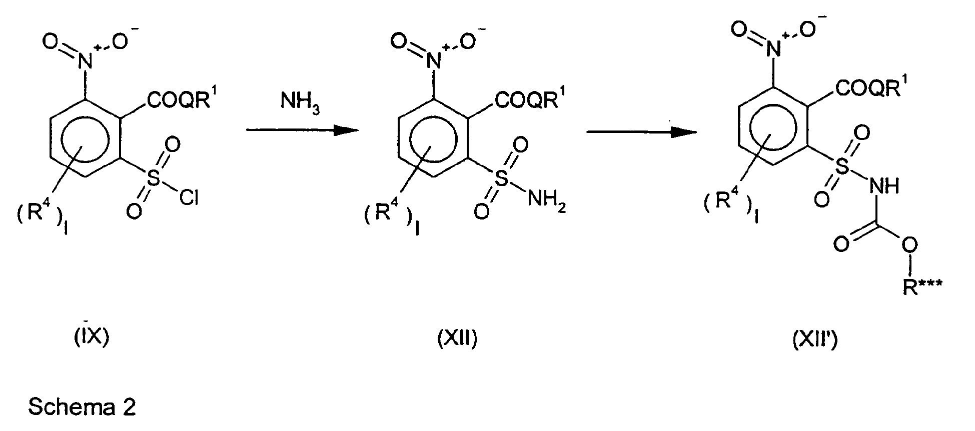

Die Sulfonamidbildung wird beispielsweise in wässrigem Medium oder inerten Lösungsmitteln wie z.B. Essigsäureethylester, Dichlormethan, Tetrahydrofuran (THF), Dioxan, Toluol oder Dimethylformamid (DMF) bei Temperaturen von -70 °C bis zum Siedepunkt des Lösungsmittels, vorzugsweise bis 25 °C durchgeführt. Dabei kommt bevorzugt eine Ammoniakmenge von 1.5 - 2.5 Äquivalenten bezogen auf Sulfochlorid zum Einsatz.

Die Umsetzung eines Aminoheterocyclus der Formel (V) mit Diphenylcarbonat und einem Sulfonamid der Formel (II) in einer Eintopfreaktion kann gemäß EP-A-562 575 durchgeführt werden.

Die genannten Verbindungen der Formel (II), (IV), (VI), (XI) und (XI)' sind strukturverwandte neue Zwischenprodukte der allgemeinen Formel (II*)

Verbindung der Formel (II*) 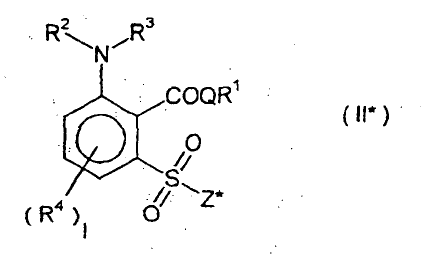 worin Z*= NH₂, NHCOOR***, NCO oder NH-tert.-Butyl bedeutet und R¹-R⁴, I und Q wie in Formel (I) nach Anspruch 1 und R*** wie in Formel (IV) nach Anspruch 5 definiert sind und wobei folgende Verbindungen a), b) und c) ausgenommen sind:
a) 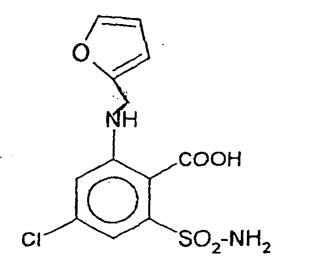
b) 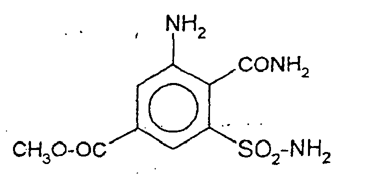
c)

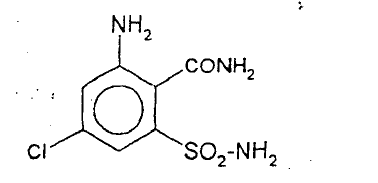

Die genannten Verbindungen der Formel (IX), (X), (XII) und (XII`) sind strukturverwandte neue Zwischenprodukte der allgemeinen Formel (X*) 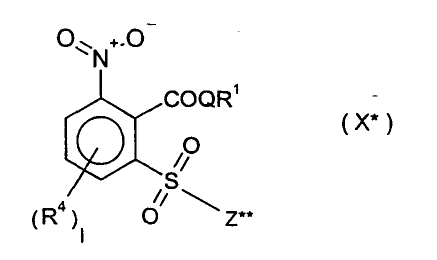 worin Z**= NH₂, NHCOOR***, NH-tert.-Butyl oder Cl bedeutet und R¹, R⁴, I und Q wie in Formel (I) definiert sind und R*** wie in Formel (IV) definiert ist, und wobei Verbindungen ausgenommen sind, in denen Z** = Cl, I = 0, Q = O und R¹ = H, Methyl, Ethyl oder Allyl ist, sowie verbindungen in denen Z** = NH₂, I=O, Q = O und R¹ = H ist.

Ebenfalls neu sind die Zwischenprodukte der allgemeinen Formel (VIII) 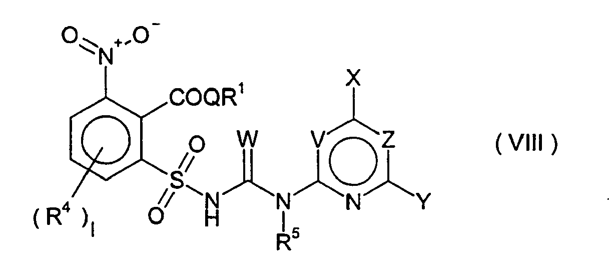 worin R¹, R⁴, R⁵, Q, V, W, X, Y, Z und I wie in Formel (I) definiert sind.

Die Salze der Verbindungen der Formel (I) werden vorzugsweise in inerten polaren Lösungsmitteln wie z.B. Wasser, Methanol oder Aceton bei Temperaturen von 0 °C bis 100 °C hergestellt. Geeignete Basen zur Herstellung der erfindungsgemäßen Salze sind beispielsweise Alkalicarbonate, wie Kaliumcarbonat, Alkali- und Erdalkalihydroxide, z.B. NaOH oder KOH, oder Alkalialkoholate, wie Natriummethanolat oder Natrium-tert.-butanolat, oder Ammoniak oder Ethanolamin.

Mit den in den vorstehenden Verfahrensvarianten bezeichneten "inerten Lösungsmitteln" sind jeweils Lösungsmittel gemeint, die unter den jeweiligen Reaktionsbedingungen inert sind, jedoch nicht unter beliebigen Reaktionsbedingungen inert sein müssen.

Kollektionen aus Verbindungen der Formel (I) und deren Salzen, die nach oben genannten Schemata synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch S.H. DeWitt in "Annual Reports in Combinatorial Chemistry and Molecular Diversity: Automated Synthesis", Band 1, Verlag Escom 1997, Seite 69 bis 77 beschrieben ist.

Zur parallelisierten Reaktionsdurchführung und Aufarbeitung können eine Reihe von im Handel erhältlichen Geräten verwendet werden, wie sie beispielsweise von den Firmen Stem Corporation, Woodrolfe road, Tollesbury, Essex, England, H+P Labortechnik GmbH, Bruckmannring 28, 85764 Oberschleißheim, Deutschland oder der Firma Radleys, Shirehill, Saffron Walden, Essex, CB 11 3AZ, England angeboten werden. Für die parallelisierte Aufreinigung von Verbindungen der allgemeinen Formel (I) und deren Salzen beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständige integrierten Automationssystemen umgangen werden, bei denen die jeweiligen Automationsmodule beispielsweise durch Roboter bedient werden. Derartige Automationssysteme können zum Beispiel von der Firma Zymark Corporation, Zymark Center, Hopkinton, MA 01748, USA bezogen werden.

Neben den hier beschriebenen Methoden kann die Herstellung von Verbindungen der allgemeinen Formel (I) und deren Salzen vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepaßten Synthese an ein Syntheseharz gebunden. Festphasen- unterstützte -Synthesemethoden sind in der Fachliteratur hinreichend beschrieben, z.B. Barry A. Bunin in "The Combinatorial Index", Verlag Academic Press, 1998.
Die Verwendung von Festphasen- unterstützten -Synthesemethoden erlaubt eine Reihe von literaturbekannten Protokollen, die wiederum manuell oder automatisierten ausgeführt werden können. Zum Beispiel kann die "Teebeutelmethode" (Houghten, US 4,631,211; Houghten et al., Proc. Natl. Acad. Sci, 1985, 82, 5131-5135) mit Produkten der Firma IRORI, 11149 North Torrey Pines Road, La Jolla, CA 92037, USA teilweise automatisiert werden. Die Automatisierung von Festphasen unterstützten Parallelsynthesen gelingt beispielsweise durch Apparaturen der Firmen Argonaut Technologies, Inc., 887 Industrial Road, San Carlos, CA 94070, USA oder MultiSynTech GmbH, Wullener Feld 4, 58454 Witten, Deutschland.

Die Herstellung gemäß der hier beschriebenen Verfahren liefert Verbindungen der Formel (I) und deren Salze in Form von Substanzkollektionen, die Bibliotheken genannt werden. Gegenstand der vorliegenden Erfindung sind auch Bibliotheken, die mindestens zwei Verbindungen der Formel (I) und deren Salzen enthalten.

Die erfindungsgemäßen Verbindungen der Formel (I) und deren Salze, im folgenden zusammen als (erfindungsgemäße) Verbindungen der Formel (I) bezeichnet, weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll.
Auf der Seite der monokotylen Unkrautarten werden z.B. Avena, Lolium, Alopecurus, Phalaris, Echinochloa, Digitaria, Setaria sowie Cyperusarten aus der annuellen Gruppe und auf seiten der perennierenden Spezies Agropyron, Cynodon, Imperata sowie Sorghum und auch ausdauernde Cyperusarten gut erfaßt.
Bei dikotylen Unkrautarten erstreckt sich das Wirkungsspektrum auf Arten wie z.B. Galium, Viola, Veronica, Lamium, Stellaria, Amaranthus, Sinapis, Ipomoea, Matricaria, Abutilon und Sida auf der annuellen Seite sowie Convolvulus, Cirsium, Rumex und Artemisia bei den perennierenden Unkräutern.
Unter den spezifischen Kulturbedingungen im Reis vorkommende Schadpflanzen wie z.B. Echinochloa, Sagittaria, Alisma, Eleocharis, Scirpus und Cyperus werden von den erfindungsgemäßen Wirkstoffen ebenfalls hervorragend bekämpft.
Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. zweikeimblättriger Kulturen wie Soja, Baumwolle, Raps, Zuckerrüben, oder Gramineen-Kulturen wie Weizen, Gerste, Roggen, Reis oder Mais, insbesondere Soja, nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen oder in Zierpflanzungen.

Darüberhinaus weisen die erfindungsgemäßen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da die Lagerbarkeit hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt ist die Anwendung der erfindungsgemäßen Verbindungen der Formel (I) oder deren Salze in wirtschaftlich bedeutenden transgenen Kulturen von Nutz-und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.
Vorzugsweise können die Verbindungen der Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044 und EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827 und WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924 und EP-A-0193259).
- transgene Kulturpflanzen mit modifizierter Fettsäurezusammensetzung (WO 91/13972).

Zahlreiche molekularbiologische Techniken, mit denen neue transgene Pflanzen mit veränderten Eigenschaften hergestellt werden können, sind im Prinzip bekannt (siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996 oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe der obengenannten Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden.

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106).

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h. sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen (I) in transgenen Kulturen eingesetzt werden, welche gegen Herbizide aus der Gruppe der Sulfonylharnstoffe, Glufosinate-ammonium oder Glyphosate-isopropylammonium und analoge Wirkstoffe resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen (I) als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die Verbindungen der Formel (I) enthalten.

Die Verbindungen der Formel (I) können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse. Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenem, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischem und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, Wirkstoff der Formel (I).
In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Als Kombinationspartner für die erfindungsgemäßen Wirkstoffe in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. aus Weed Research 26 (1986) 441-445 oder "The Pesticide Manual", 11th edition, The British Crop Protection Council and the Royal Soc. of Chemistry, 1997 und dort zitierter Literatur beschrieben sind. Als bekannte Herbizide, die mit den Verbindungen der Formel (I) kombiniert werden können, sind z.B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet):
acetochlor; acifluorfen(-sodium); aclonifen; AKH 7088, d.h. [[[1-[5-[2-Chloro-4-(trifluoromethyl)-phenoxy]-2-nitrophenyl]-2-methoxyethylidene]-amino]-oxy]-essigsäure und -essigsäuremethylester, alachlor; alloxydim(-sodium); ametryn; amicarbazone; amidosulfuron; amitrol; AMS, d.h. Ammoniumsulfamat; anilofos; asulam; atrazin; azafenidin; azimsulfuron; aziprotryn; barban; BAS 516 H, d.h. 5-Fluor-2-phenyl-4H-3,1-benzoxazin-4-on; beflubutamid; benazolin(-ethyl); benfluralin; benfuresate; bensulfuron(-methyl); bensulide; bentazone; benzobicyclon; benzofenap; benzofluor; benzoylprop(-ethyl); benzthiazuron; bialaphos; bifenox; bispyribac(-sodium); bromacil; bromobutide; bromofenoxim; bromoxynil; bromuron; buminafos; busoxinone; butachlor; butafenacil; butamifos; butenachlor; buthidazole; butralin; butroxydim; butylate; cafenstrole; caloxydim; carbetamide; carfentrazone(-ethyl); CDAA, d.h. 2-Chlor-N,N-di-2-propenylacetamid; CDEC, d.h. Diethyldithiocarbaminsäure-2-chlorallylester; chlomethoxyfen; chloramben; chlorazifop-butyl, chlorbromuron; chlorbufam; chlorfenac; chlorflurecol-methyl; chloridazon; chlorimuron(-ethyl); chlormesulam (ICI-A0051); chlornitrofen; chlorotoluron; chloroxuron; chlorpropham; chlorsulfuron; chlorthal(-dimethyl); chlorthiamid; cinidon(-ethyl und -methyl); cinmethylin; cinosulfuron; clefoxydim; clethodim; clodinafop und dessen Esterderivate (z.B. clodinafop-propargyl); clomazone; clomeprop; cloproxydim; clopyralid; clopyrasulfuron(-methyl); cloransulam(-methyl); cumyluron; cyanazine; cycloate; cyclosulfamuron; cycloxydim; cycluron; cyhalofop und dessen Esterderivate (z.B. cyhalofop-butyl); cyperquat; cyprazine; cyprazole; daimuron; 2,4-D; 2,4-DB; dalapon; desmedipham; desmetryn; di-allate; dicamba; dichlobenil; dichlorprop; diclofop und dessen Ester wie diclofop-methyl; diclosulam, d.h. N-(2,6-Dichlorphenyl)-5-ethoxy-7-fluor-[1,2,4]triazolo[1,5-c]pyrimidin-2-sulfonamid; diethatyl(-ethyl); difenoxuron; difenzoquat; diflufenican; diflufenzopyr(-sodium), dimefuron; dimepiperate; dimethachlor; dimethametryn; dimethenamid ; dimidazon; dimethipin; dinitramine; dinoseb; dinoterb; diphenamid; dipropetryn; diquat; dithiopyr; diuron; DNOC; eglinazine-ethyl; EL 77, d.h. 5-Cyano-1-(1,1-dimethylethyl)-N-methyl-1 H-pyrazole-4-carboxamid; endothal; EPTC; esprocarb; ethalfluralin; ethametsulfuron(-methyl); ethidimuron; ethiozin; ethofumesate; ethoxyfen und dessen Ester (z.B. Ethylester, HN-252); ethoxysulfuron; etobenzanid; F5231, d.h. N-[2-Chlor-4-fluor-5-[4-(3-fluorpropyl)-4,5-dihydro-5-oxo-1H-tetrazol-1-yl]-phenyl]-ethansulfonamid; fenoprop; fenoxan, fenoxaprop und fenoxaprop-P sowie deren Ester, z.B. fenoxaprop-P-ethyl und fenoxaprop-ethyl; fenoxydim; fentrazamide; fenuron; flamprop(-methyl oder-isopropyl oder-isopropyl-L); flazasulfuron; florasulam; fluazifop und fluazifop-P und deren Ester (z.B. fluazifop-butyl und fluazifop-P-butyl); fluazolate(-isopropazol); flucarbazone(-sodium); fluchloralin; flufenacet; flumetsulam; flumeturon; flumiclorac und dessen Ester (z.B. flumidoracpentyl); flumioxazin; flumipropyn; fluometuron; flupoxam (KNW-739); fluorodifen; fluoroglycofen(-ethyl); flupropacil; flupyrsulfuron(-methyl, -sodium); flurenol(-butyl); fluridone; flurochloridone; fluroxypyr(-meptyl); flurtamone; fluthiacet(-methyl); fluthiamide; fomesafen; foramsulfuron; fosamine; furyloxyfen; glufosinate(-ammonium); glyphosate(-isopropylammonium); halosafen; halosulfuron(-methyl); haloxyfop und dessen Ester; haloxyfop-P (= R-haloxyfop) und dessen Ester; hexazinone; imazamethabenz; imazamox; imazapic; imazapyr; imazaquin(-ammonium); ; imazethamethapyr; imazethapyr; imazosulfuron; indanofan; iodosulfuron; ioxynil; isocarbamid; isopropalin; isoproturon; isouron; isoxaben; isoxachlortole; isoxaflutole; isoxapyrifop; karbutilate; lactofen; lenacil; linuron; MCPA; MCPB; mecoprop; mefenacet; mefluidid; mesotrione; metamitron; metazachlor; methabenzthiazuron; metham; methazole; methoxyphenone; methyldymron; metobenzuron; metobromuron; (alpha-)metolachlor; metosulam); metoxuron; metribuzin; metsulfuron(-methyl); MH; molinate; monalide; monocarbamide dihydrogensulfate; monolinuron; monuron; MT 128, d.h. 6-Chlor-N-(3-chlor 2-propenyl)-5-methyl-N-phenyl-3-pyridazinamin; MT 5950, d.h. N-[3-Chlor-4-(1-methylethyl)-phenyl]-2-methylpentanamid; naproanilide; napropamide; naptalam; NC 310, d.h. 4-(2,4-dichlorbenzoyl)-1-methyl-5-benzyloxypyrazol; neburon; nicosulfuron; nipyraclophen; nitralin; nitrofen; nitrofluorfen; norflurazon; orbencarb; oryzalin; oxaciclomefone; oxadiargyl; oxadiazone; oxasulfuron; oxyfluorfen; paraquat; pebulate; pelargonic acid; pendimethalin; pentoxazone; perfluidone; phenisopham; phenmedipham; picloram; picolinafen; piperophos; piributicarb; pirifenop(-butyl); pretilachlor; primisulfuron(-methyl); procarbazone(-sodium); procyazine; prodiamine; profluralin; proglinazine(-ethyl); prometon; prometryn; propachlor; propanil; propaquizafop und dessen Ester; propazine; propham; propisochlor; propyzamide; prosulfalin; prosulfocarb; prosulfuron; prynachlor; pyroflufen(-ethyl); pyrazolinate; pyrazon; pyrazosulfuron(-ethyl); pyrazoxyfen; pyribenzoxim; pyributicarb; pyridafol; pyridate; pyriminobac(-methyl); pyrithiobac(-sodium); pyroxofop und dessen Ester (z.B. pyroxofop-propargyl); quinclorac; quinmerac; quinofop und dessen Esterderivate; quinoclamine; quizalofop und quizalofop-P und deren Esterderivate (z.B. quizalofop-ethyl; quizalofop-p-tefuryl und -ethyl); renriduron; rimsulfuron; S 275, d.h. 2-[4-Chlor-2-fluor-5-(2-propynyloxy)-phenyl]-4,5,6,7-tetrahydro-2H-indazol; secbumeton; sethoxydim; siduron; simazine; simetryn; SN 106279, d.h. 2-[[7-[2-Chlor-4-(trifluor-methyl)-phenoxy]-2-naphthalenyl]-oxy]-propansäure und -methylester; sulcotrione; sulfentrazone; sulfazurone; sulfometuron(-methyl); sulfosate (ICI-A0224); sulfosulfuron; TCA(-sodium); tebutam; tebuthiuron; tepraloxydim; terbacil; terbucarb; terbuchlor; terbumeton; terbuthylazine; terbutryn; TFH 450, d.h. N,N-Diethyl-3-[(2-ethyl-6-methylphenyl)-sulfonyl]-1 H-1,2,4-triazol-1-carboxamid; thenylchlor; thiafluamide; thiazafluron; thiazopyr; thidiazimin (SN-24085); thifensulfuron(-methyl); thiobencarb; tiocarbazil; tralkoxydim; tri-allate; triasulfuron; triaziflam, triazofenamide; tribenuron(-methyl); triclopyr, tridiphane; trietazine; trifluralin; triflusulfuron(-methyl); tritosulfuron; trimeturon; tsitodef; vernolate; WL 110547, d.h. 5-Phenoxy-1-[3-(trifluormethyl)-phenyl]-1H-tetrazol; AMS-13668; D-489; BH-60229; BH-71712; DK-8910; DOWCO-535; DPX-N8189; JTC-101; KH-218; KPP-300; KPP-421; LS 82-556; MBH-001; MT-146; MT-147; NC-324; NC-330; OK-9403; OK-9604; OK-9701; PP-600; SC-0774 und UBH-509.

Die erfindungsgemäßen Wirkstoffe können auch in Kombination mit einem oder mehreren als Safener wirkenden Verbindungen eingesetzt werden. Beispiele für Safener sind:
a) Verbindungen der Formeln (XIII) bis (XV), 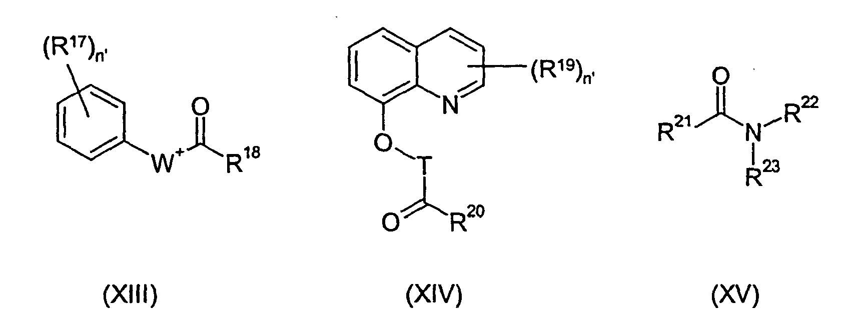 wobei die Symbole und Indizes folgende Bedeutungen haben:
   - n': ist eine natürliche Zahl von 0 bis 5, vorzugsweise 0 bis 3;
   - T: ist eine (C₁ oder C₂)Alkandiylkette, die unsubstituiert oder mit einem oder zwei (C₁-C₄)Alkylresten oder mit [(C₁-C₃)Alkoxy]carbonyl substituiert ist;
   - W⁺: ist ein unsubstituierter oder substituierter divalenter heterocyclischer Rest aus der Gruppe der teilungesättigten oder aromatischen Fünfring-Heterocyclen mit 1 bis 3 Heteroringatomen des Typs N oder O, wobei mindestens ein N-Atom und höchstens ein O-Atom im Ring enthalten ist, vorzugsweise ein Rest aus der Gruppe (W⁺1) bis (W⁺4), 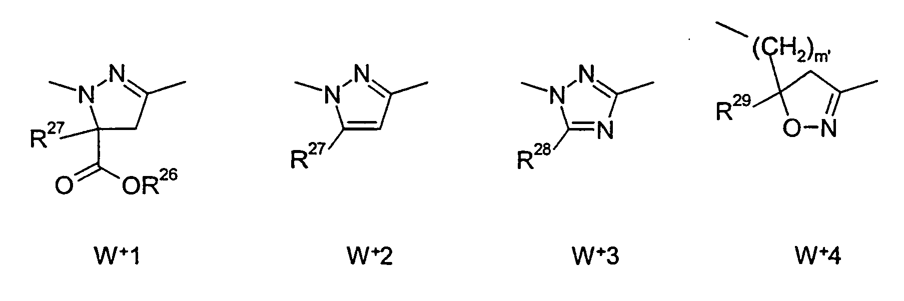
   - m': ist 0 oder 1;
   - R¹⁷, R¹⁹: sind gleich oder verschieden Wasserstoff, Halogen, (C₁-C₄)Alkyl, (C₁-C₄)Alkoxy, Nitro oder (C₁-C₄)Haloalkyl;
   - R¹⁸, R²⁰: sind gleich oder verschieden OR²⁴, SR²⁴ oder NR²⁴R²⁵ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (XIII) bzw. (XIV) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR²⁴, NHR²⁵ oder N(CH₃)₂, insbesondere der Formel OR²⁴;
   - R²⁴: ist Wasserstoff oder ein unsubstituierter oder substituierter aliphatischer Kohlenwasserstoffrest, vorzugsweise mit insgesamt 1 bis 18 C-Atomen;
   - R²⁵: ist Wasserstoff, (C₁-C₆)Alkyl, (C₁-C₆)Alkoxy oder substituiertes oder unsubstituiertes Phenyl;
   - R²⁶: ist Wasserstoff, (C₁-C₈)Alkyl, (C₁-C₈)Haloalkyl, (C₁-C₄)Alkoxy-(C₁-C₄)alkyl, (C₁-C₆)Hydroxyalkyl, (C₃-C₁₂)Cycloalkyl oder Tri- (C₁-C₄)alkylsilyl;
   - R²⁷, R²⁸, R²⁹: sind gleich oder verschieden Wasserstoff, (C₁-C₈)Alkyl, (C₁-C₈)Haloalkyl, (C₃-C₁₂)Cycloalkyl oder substituiertes oder unsubstituiertes Phenyl;
   - R²¹: ist (C₁-C₄)Alkyl, (C₁-C₄)Haloalkyl, (C₂-C₄)Alkenyl, (C₂-C₄)Haloalkenyl, (C₃-C₇)Cycloalkyl, vorzugsweise Dichlormethyl;
   - R²², R²³: ist gleich oder verschieden Wasserstoff, (C₁-C₄)Alkyl, (C₂-C₄)Alkenyl, (C₂-C₄)Alkinyl, (C₁-C₄)Haloalkyl, (C₂-C₄)Haloalkenyl, (C₁-C₄)Alkylcarbamoyl-(C₁-C₄)alkyl, (C₂-C₄)Alkenylcarbamoyl-(C₁-C₄)alkyl, (C₁-C₄)Alkoxy-(C₁-C₄)alkyl, Dioxolanyl-(C₁-C₄)alkyl, Thiazolyl, Furyl, Furylalkyl, Thienyl, Piperidyl, substituiertes oder unsubstituiertes Phenyl, oder R²² und R²³ bilden zusammen einen substituierten oder unsubstituierten heterocyclischen Ring, vorzugsweise einen Oxazolidin-, Thiazolidin-, Piperidin-, Morpholin-, Dihydropyrimidin- oder Benzoxazinring;
   oder
b) eine oder mehreren Verbindungen aus Gruppe:
   1,8-Naphthalsäureanhydrid,
   Methyl-diphenylmethoxyacetat,
   Cyanomethoxyimino(phenyl)acetonitril (Cyometrinil),
   1,3-Dioxolan-2-ylmethoxyimino(phenyl)acetonitril (Oxabetrinil),
   4'-Chlor-2,2,2-trifluoracetophenon O-1,3-dioxolan-2-ylmethyloxim (Fluxofenim),
   4,6-Dichlor-2-phenylpyrimidin (Fenclorim),
   Benzyl-2-chlor-4-trifluormethyl-1,3-thiazol-5-carboxylat (Flurazole),
   2-Dichlormethyl-2-methyl-1,3-dioxolan (MG-191),
   N-(4-Methylphenyl)-N'-(1-methyl-1-phenylethyl)harnstoff (Dymron),
   1-[4-(N-2-Methoxybenzoylsulfamoyl)phenyl]-3-methylharnstoff,
   1-[4-(N-2-Methoxybenzoyisulfamoyl)phenyl]-3,3-dimethylhamstoff,
   1-[4-(N-4,5-Dimethylbenzoylsulfamoyl)phenyl]-3-methylhamstoff,
   1-[4-(N-Naphthoylsulfamoyl)phenyl]-3,3-dimethylhamstoff,
   (2,4-Dichlorphenoxy)essigsäure (2,4-D),
   (4-Chlorphenoxy)essigsäure,
   (R,S)-2-(4-Chlor-o-tolyloxy)propionsäure (Mecoprop),
   4-(2,4-Dichlorphenoxy)buttersäure (2,4-DB),
   (4-Chlor-o-tolyloxy)essigsäure (MCPA),
   4-(4-Chlor-o-tolyloxy)buttersäure,
   4-(4-Chlorphenoxy)buttersäure,
   3,6-Dichlor-2-methoxybenzoesäure (Dicamba),
   1-(Ethoxycarbonyl)ethyl 3,6-dichlor-2-methoxybenzoat (Lactidichlor)
   sowie deren Salze und Ester, vorzugsweise (C₁-C₈)-Ester;
c) N-Acylsulfonamide der Formel (XVI) und ihre Salze, 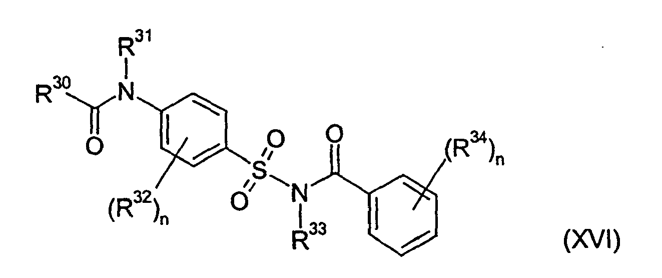 worin
   - R³⁰: Wasserstoff, einen Kohlenwasserstoffrest, einen Kohlenwasserstoffoxyrest, einen Kohlenwasserstoffthiorest oder einen Heterocyclylrest, wobei jeder der letztgenannten 4 Reste unsubstituiert oder durch einen oder mehrere gleiche oder verschiedene Reste aus der Gruppe Halogen, Cyano, Nitro, Amino, Hydroxy, Carboxy, Formyl, Carbonamid, Sulfonamid und Reste der Formel -Z^{a}-R^{a} substituiert ist,
   wobei jeder Kohlenwasserstoffteil vorzugsweise 1 bis 20 C-Atome aufweist
   und ein C-haltiger Rest R³⁰ inklusive Substituenten vorzugsweise 1 bis 30 C-Atome aufweist;
   - R³¹: Wasserstoff oder (C₁-C₄)Alkyl, vorzugsweise Wasserstoff, oder
   - R³⁰ und R³¹: zusammen mit der Gruppe der Formel -CO-N- den Rest eines 3- bis 8-gliedrigen gesättigten oder ungesättigten Rings;
   - R³²: gleich oder verschieden Halogen, Cyano, Nitro, Amino, Hydroxy, Carboxy, Formyl, CONH₂, SO₂NH₂ oder einen Rest der Formel -Z^{b}-R^{b} ;
   - R³³: Wasserstoff oder (C₁-C₄)Alkyl, vorzugsweise H;
   - R³⁴: gleich oder verschieden Halogen, Cyano, Nitro, Amino, Hydroxy, Carboxy, CHO, CONH₂, SO₂NH₂ oder einen Rest der Formel -Z^{c}-R^{c} ;
   - R^{a}: einen Kohlenwasserstoffrest oder einen Heterocyclylrest, wobei jeder der beiden letztgenannten Reste unsubstituiert oder durch einen oder mehrere gleiche oder verschiedene Reste aus der Gruppe Halogen, Cyano, Nitro, Amino, Hydroxy, Mono- und Di-[(C₁-C₄)alkyl]amino substituiert ist, oder einen Alkylrest, in dem mehrere, vorzugsweise 2 oder 3, nicht benachbarte CH₂-Gruppen jeweils durch ein Sauerstoffatom ersetzt sind;
   - R^{b}, R^{c}: gleich oder verschieden einen Kohlenwasserstoffrest oder einen Heterocyclylrest, wobei jeder der beiden letztgenannten Reste unsubstituiert oder durch einen oder mehrere gleiche oder verschiedene Reste aus der Gruppe Halogen, Cyano, Nitro, Amino, Hydroxy, Phosphoryl, Halogen-(C₁-C₄)alkoxy, Mono- und Di-[(C₁-C₄)alkyl]amino substituiert ist, oder einen Alkylrest, in dem mehrere, vorzugsweise 2 oder 3, nicht benachbarte CH₂-Gruppen jeweils durch ein Sauerstoffatom ersetzt sind;
   - Z^{a}: eine divalente Gruppe der Formel -O-, -S-, -CO-, -CS-, -CO-O-, -CO-S-, -O-CO-, -S-CO-, -SO-, -SO₂-, -NR⁺-, -CO-NR⁺-, -NR⁺-CO-, -SO₂-NR⁺- oder -NR⁺-SO₂-, wobei die rechts angegebene Bindung der jeweiligen divalenten Gruppe die Bindung zum Rest R^{a} ist und wobei die R⁺ in den letztgenannten 5 Resten unabhängig voneinander jeweils H, (C₁-C₄)Alkyl oder Halo-(C₁-C₄)-alkyl bedeuten;
   - Z^{b}, Z^{c}: unabhängig voneinander eine direkte Bindung oder eine divalente Gruppe der Formel -O-, -S-, -CO-, -CS-, -CO-O-, -CO-S-, -O-CO-, -S-CO-, -SO-, -SO₂-, -NR⁺-, -SO₂-NR⁺-, -NR⁺-SO₂-, -CO-NR⁺- oder -NR⁺-CO-, wobei die rechts angegebene Bindung der jeweiligen divalenten Gruppe die Bindung zum Rest R^{b} bzw. R^{c} ist und wobei die R⁺ in den letztgenannten 5 Resten unabhängig voneinander jeweils H, (C₁-C₄)Alkyl oder Halo-(C₁-C₄)alkyl bedeuten;
   - n: eine ganze Zahl von 0 bis 4, vorzugsweise 0, 1 oder 2, insbesondere 0 oder 1, und
   - m: eine ganze Zahl von 0 bis 5, vorzugsweise 0, 1, 2 oder 3, insbesondere 0, 1 oder 2;
   bedeuten;
d) Acylsulfamoylbenzoesäureamide der allgemeinen Formel (XVII), gegebenenfalls auch in Salzform, 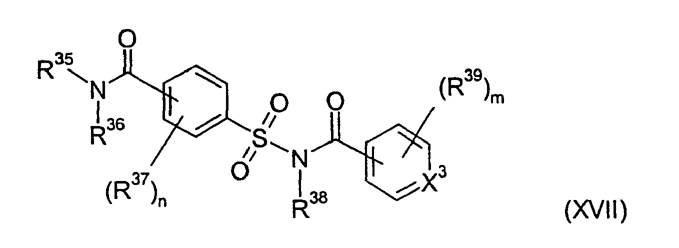 worin
   - X³: CH oder N;
   - R³⁵: Wasserstoff, Heterocyclyl oder einen Kohlenwasserstoffrest, wobei die beiden letztgenannten Reste gegebenenfalls durch einen oder mehrere, gleiche oder verschiedene Reste aus der Gruppe Halogen, Cyano, Nitro, Amino, Hydroxy, Carboxy, CHO, CONH₂, SO₂NH₂ und Z^{a}-R^{a} substituiert sind;
   - R³⁶: Wasserstoff, Hydroxy, (C₁-C₆)Alkyl, (C₂-C₆)Alkenyl, (C₂-C₆)Alkinyl, (C₁-C₆)Alkoxy, (C₂-C₆)Alkenyloxy, wobei die fünf letztgenannten Reste gegebenenfalls durch einen oder mehrere, gleiche oder verschiedene Reste aus der Gruppe Halogen, Hydroxy, (C₁-C₄)Alkyl, (C₁-C₄)Alkoxy und (C₁-C₄)Alkylthio substituiert sind, oder
   - R³⁵ und R³⁶: zusammen mit dem sie tragenden Stickstoffatom einen 3- bis 8-gliedrigen gesättigten oder ungesättigten Ring;
   - R³⁷: Halogen, Cyano, Nitro, Amino, Hydroxy, Carboxy, CHO, CONH₂, SO₂NH₂ oder Z^{b}-R^{b};
   - R³⁸: Wasserstoff, (C₁-C₄)Alkyl, (C₂-C₄)Alkenyl oder (C₂-C₄)Alkinyl;
   - R³⁹: Halogen, Cyano, Nitro, Amino, Hydroxy, Carboxy, Phosphoryl, CHO, CONH₂, SO₂NH₂ oder Z^{c}-R^{c};
   - R^{a}: einen (C₂-C₂₀)Alkylrest, dessen Kohlenstoffkette ein- oder mehrfach durch Sauerstoffatome unterbrochen ist, Heterocyclyl oder einen Kohlenwasserstoffrest, wobei die zwei letztgenannten Reste gegebenenfalls durch einen oder mehrere, gleiche oder verschiedene Reste aus der Gruppe Halogen, Cyano, Nitro, Amino, Hydroxy, Mono- und Di-[(C₁-C₄)alkyl]amino substituiert sind;
   - R^{b}, R^{c}: gleich oder verschieden einen (C₂-C₂₀)Alkylrest, dessen Kohlenstoffkette ein- oder mehrfach durch Sauerstoffatome unterbrochen ist, Heterocyclyl oder einen Kohlenwasserstoffrest, wobei die zwei letztgenannten Reste gegebenenfalls durch einen oder mehrere, gleiche oder verschiedene Reste aus der Gruppe Halogen, Cyano, Nitro, Amino, Hydroxy, Phosphoryl, (C₁-C₄)-Haloalkoxy, Mono- und Di-[(C₁-C₄)alkyl]amino substituiert sind;
   - Z^{a}: eine divalente Einheit aus der Gruppe O, S, CO, CS, C(O)O, C(O)S, SO, SO₂, NR^{d}, C(O)NR^{d} oder SO₂NR^{d};
   - Z^{b}, Z^{c}: gleich oder verschieden eine direkte Bindung oder eine divalente Einheit aus der Gruppe O, S, CO, CS, C(O)O, C(O)S, SO, SO₂, NR^{d}, SO₂NR^{d} oder C(O)NR^{d};
   - R^{d}: Wasserstoff, (C₁-C₄)Alkyl oder (C₁-C₄)Haloalkyl;
   - n: eine ganze Zahl von 0 bis 4, und
   - m: für den Fall, daß X für CH steht, eine ganze Zahl von 0 bis 5, und für den Fall, daß X für N steht, eine ganze Zahl von 0 bis 4
   bedeuten;
e) Verbindungen der Formel (XVIII), 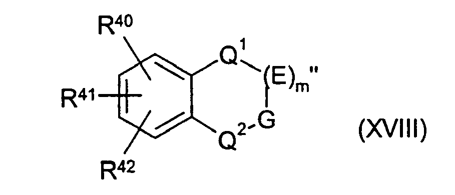
worin die Symbole und Indizes folgende Bedeutungen haben:
- R⁴⁰: ist H, (C₁-C₄)Alkyl, (C₁-C₄)Alkyl substituiert mit (C₁-C₄)Alkyl-X⁴ oder (C₁-C₄)Haloalkyl-X⁴, (C₁-C₄)Haloalkyl, NO₂, CN, -COO-R⁴³, NR₂⁴⁴, SO₂NR₂⁴⁵ oder CONR₂⁴⁶;
- R⁴¹: ist H, Halogen, (C₁-C₄)Alkyl, CF₃, (C₁-C₄)Alkoxy oder (C₁-C₄)Haloalkoxy;
- R⁴²: ist H, Halogen oder (C₁-C₄)Alkyl;
- Q¹, Q², E, G: sind gleich oder verschieden, -O-, -S-, -CR₂⁴⁷-, -CO-, NR⁴⁸- oder eine Gruppe der Formel (XIX), 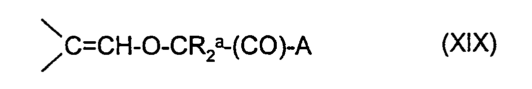 mit der Maßgabe, daß
- a): mindestens eine der Gruppen Q¹, Q², E, G eine Carbonylgruppe ist, daß genau eine dieser Gruppe ein Rest der Formel (XIX) ist und daß die Gruppe der Formel (XIX) einer Carbonylgruppe benachbart ist, und
- b): zwei benachbarte Gruppen Q¹ Q², E und G nicht gleichzeitig Sauerstoff sein können;
- R^{a}: ist gleich oder verschieden H oder (C₁-C₈)Alkyl oder die beiden Reste R^{a} zusammen sind (C₂-C₆)Alkylen;
- A: ist R^{b}-Y³-oder-NR₂⁴⁹;
- X⁴: ist -O- oder -S(O)p-;
- Y³: ist -O- oder -S-;
- R^{b}: ist H, (C₁-C₈)Alkyl, (C₁-C₈)Haloalkyl, (C₁-C₄)Alkoxy-(C₁-C₈)alkyl, (C₃-C₆)-Alkenyloxy-(C₁-C₈)alkyl, oder Phenyl-(C₁-C₈)alkyl, wobei der Phenylring gegebenenfalls durch Halogen, (C₁-C₄)Alkyl, CF₃, Methoxy oder Methyl-S(O)ₚ substituiert ist; (C₃-C₆)Alkenyl, (C₃-C₆)Haloalkenyl, Phenyl-(C₃-C₆)alkenyl, (C₃-C₆)Alkinyl, Phenyl-(C₃-C₆)alkinyl, Oxetanyl, Furfuryl, Tetrahydrofuryl;
- R⁴³: ist H oder (C₁-C₄)Alkyl;
- R⁴⁴: ist gleich oder verschieden H, (C₁-C₄)Alkyl, (C₁-C₄)Alkylcarbonyl oder die beiden Reste R⁴⁴ zusammen sind (C₄-C₅)Alkylen;
- R⁴⁵, R⁴⁶: sind unabhängig voneinander jeweils gleich oder verschieden H, (C₁-C₄)Alkyl, oder die beiden Reste R⁴⁵ und/oder R⁴⁶ zusammen sind (C₄-C₅)Alkylen, wobei eine CH₂-Gruppe durch O oder S oder eine oder zwei CH₂-Gruppen durch -NR^{c}- ersetzt sein können;
- R^{c}: ist H oder (C₁-C₈)Alkyl;
- R⁴⁷: ist gleich oder verschieden H, (C₁-C₈)Alkyl oder die beiden Reste R⁴⁷ zusammen sind (C₂-C₆)Alkylen;
- R⁴⁸: ist H, (C₁-C₈)Alkyl, substituiertes oder unsubstituiertes Phenyl, oder unsubstituiertes oder am Phenylring substituiertes Benzyl;
- R⁴⁹: ist gleich oder verschieden H, (C₁-C₈)Alkyl, Phenyl, Phenyl-(C₁-C₈)alkyl, wobei ein Phenylring durch F, Cl, Br, NO₂, CN, OCH₃, (C₁-C₄)Alkyl oder CH₃SO₂- substituiert sein kann; (C₁-C₄)Alkoxy-(C₁-C₈)alkyl, (C₃-C₆)Alkenyl, (C₃-C₆)Alkinyl, (C₃-C₆)Cycloalkyl oder zwei Reste R⁴⁹ zusammen sind (C₄-C₅)Alkylen, wobei eine CH₂-Gruppe durch O oder S oder eine oder zwei CH₂-Gruppen durch -NR^{d}- ersetzt sein können;
- R^{d}: ist H oder (C₁-C₄)Alkyl;
- m": ist 0 oder 1 und
- p: ist 0, 1 oder 2;
einschließlich der Stereoisomeren und der in der Landwirtschaft gebräuchlichen Salze.

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0.001 und 10.0 kg/ha oder mehr Aktivsubstanz, vorzugsweise liegt sie jedoch zwischen 0.005 und 5 kg/ha.

### Beispiele

### A. Chemische Beispiele

### Beispiel A1

### Methyl 2-[(tert-butylamino)sulfonyl]-6-nitrobenzoat

Zu einer Lösung von 1.00 g (3.58 mmol) Methyl 2-(chlorsulfonyl)-6-nitrobenzoat in 8 ml Dichlormethan tropft man bei 0 °C 1.4 ml (13.3 mmol) tert.-Butylamin und läßt das Reaktionsgemisch dann auf Raumtemperatur kommen. Nach 2 h wird das Reaktionsgemisch auf Eiswasser gegossen, mit 2 N wässriger Salzsäure auf pH 3 eingestellt und mit Dichlormethan extrahiert. Anschließend wird die organische Phase über Natriumsulfat getrocknet, abfiltriert und im Vakuum zur Trockne eingeengt. Der verbleibende Rückstand wird mit Diethylether digeriert und abgesaugt. Man erhält so 1.06 g (91 % d. Th.) Methyl 2-[(*tert*-butylamino)sulfonyl]-6-nitrobenzoat vom Schmp. 89-91 °C.

### Beispiel A2

### Methyl 2-(aminosulfonyl)-6-nitrobenzoat

300 mg (0.948 mmol) Methyl 2-[(*tert*-butylamino)sulfonyl]-6-nitrobenzoat werden in 8 ml Trifluoressigsäure 4 h bei Raumtemperatur gerührt. Anschließend wird im Vakuum zur Trockne eingeengt und der Rückstand wird mit Diethylether digeriert. Nach Absaugen und Trocknen verbleiben 230 mg (93 % d. Th.) Methyl 2-(aminosulfonyl)-6-nitrobenzoat vom Schmp. 227-229 °C.

### Beispiel A3

### Methyl 2-[(tert-butylamino)sulfonyl]-6-(isopropylamino)benzoat

Bei einer Temperatur von 5 - 10 °C werden 925 mg (24.4 mmol) Natriumborhydrid in 14 ml Essigsäure eingerührt. Nach 1 h wird dieses Gemisch erst mit 1.42 g (24.4 mmol) Aceton, dann mit 700 mg (2.44 mmol) Methyl 2-amino-6-[(*tert-*butylamino)sulfonyl]benzoat versetzt und 2.5 h bei 5 - 10 °C gerührt. Zur Aufarbeitung wird das Reaktionsgemisch auf Eiswasser gegeben, mit wässriger Ammoniaklösung neutralisiert und mit Dichlormethan extrahiert. Die organische Phase wird eingeengt und der verbleibende Rückstand mit Diisopropylether verrrieben. Nach Absaugen und Trocknen verbleiben 700 mg (89 % d. Th.) Methyl 2-[(*tert*-butylamino)sulfonyl]-6-(isopropylamino)benzoat als weißer Feststoff vom Schmp. 110 - 112 °C.

### Beispiel A4

### Methyl 2-amino-6-[(tert-butylamino)sulfonyl]benzoat

Eine Lösung von 8.00 g (25.3 mmol) Methyl 2-[(*tert*-butylamino)sulfonyl]-6-nitrobenzoat wird mit 3.2 g Palladium auf Aktivkohle (5 %ig) versetzt und bei Raumtemperatur 6 h unter Normaldruck hydriert. Anschließend filtriert man das Reaktionsgemisch, engt das Filtrat zur Trockne ein und reinigt den Rückstand durch Chromatographie an Kieselgel [DC (SiO₂) Ethylacetat/n-Heptan 1:1; R_{f}= 0.38] mit Ethylacetat/n-Heptan (1:1). Nach Evaporation erhält man 6.23 g (86 % d. Th.) Methyl 2-amino-6-[(*tert*-butylamino)sulfonyl]benzoat als beiger Feststoff vom Schmelzpunkt 132 - 133 °C.

### Beispiel A5

### Methyl 2-(diisobutylamino)-6-[([(4,6-dimethoxy-2-pyrimidinyl)amino]carbonylamino)sulfonyl]benzoat

700 mg (2.04 mmol) Methyl 2-(diisobutylamino)-6-(sulfonylamino)benzoat und 619 mg (2.25 mmol) N-(4,6-Dimethoxypyrimidin-2-yl)carbaminsäurephenylester werden in 10 ml Acetonitril vorgelegt. Bei Raumtemperatur werden dann 375 mg (2.51 mmol) 1,8-Diazabicyclo[5.4.0]undec-7-en (1,5-5) (DBU) zugetropft und das Reaktionsgemisch 3 h bei dieser Temperatur gerührt. Anschließend gießt man auf Eiswasser und stellt die Lösung mit 2 N Salzsäure langsam auf pH 1 ein. Der ausgefallene Feststoff wird abgesaugt, mit Wasser nachgewaschen und getrocknet. Man erhält so 910 mg (91 % d.Th.) Methyl 2-(diisobutylamino)-6-[([(4,6-dimethoxy-2-pyrimidinyl)amino]carbonylamino)sulfonyl]benzoat vom Schmp. 166 - 167 °C.

### Beispiel A6

### Methyl 2-[([(4,6-dimethoxy-1,3,5-triazin-2-yl)amino]carbonylamino)sulfonyl]-6-nitrobenzoat

Zu einer Suspension aus 2.79 g (17.9 mmol) 2-Amino-4,6-dimethoxy-1,3,5-triazin, 1.28 g (19.7 mmol) Natriumcyanat, 1.45 ml (17.9 mmol) Pyridin und 500 mg frisch aktiviertem Molekularsieb (Porengröße 3 Å) in 85 ml trockenem Acetonitril, tropft man bei 55 - 60 °C binnen 2 h unter Argon eine Lösung von 5.0 g (17.9 mmol) 2-Chlorsulfonyl-6-nitrobenzoesäuremethylester in 55 ml trockenem Dichlormethan. Nach 7 h Rühren bei dieser Temperatur läßt man auf Raumtemperatur kommen. Nach weiteren 12 h engt man das Reaktionsgemisch ein, suspendiert den Rückstand in 200 ml Wasser und stellt mit 2 N wässriger Natriumhydroxidlösung auf pH 10 ein. Der ausgefallene Feststoff wird abgesaugt und das Filtrat mit Ethylacetat extrahiert. Die organische Phase wird abgetrennt und die wässrige Phase mit 3 N wässriger Salzsäurelösung auf pH 1.5 eingestellt und 15 min im Eisbad nachgerührt. Anschließend wird der ausgefallene Feststoff abgesaugt, erst mit Wasser dann mit Methanol nachgewaschen und im Vakuum getrocknet. Man erhält so 2.88 g (36 % d. Th.) Methyl 2-[([(4,6-dimethoxy-1,3,5-triazin-2-yl)amino]carbonylamino)sulfonyl]-6-nitrobenzoat vom Schmp. 185 - 195 °C (Zers.).

### Beispiel A7

### Methyl 2-amino-6-[([(4,6-dimethoxy-1,3,5-triazin-2-yl)amino]carbonylamino)sulfonyl]benzoat

50 mg (113 µmol) Methyl 2-[([(4,6-dimethoxy-1,3,5-triazin-2-yl)amino]carbonylamino)sulfonyl]-6-nitrobenzoat werden in einem Gemisch aus 3 ml Methanol/Dimethylformamid (5:1) suspendiert, mit 10 mg Palladium auf Aktivkohle (5 %ig) und 1 mg (8.20 µmol) Natrium(meta)vanadat versetzt und 3 h unter Normaldruck bei Raumtemperatur hydriert. Anschließend wird abfiltriert, das Filtrat eingeengt, der Rückstand in Wasser aufgenommen und mit 3 N wässriger Salzsäurelösung auf pH 1.5 eingestellt. Der ausgefallene Niederschlag wird 10 min im Eisbad nachgerührt, dann abgesaugt und im Vakuum getrocknet. Man erhält 41 mg (87 % d. Th.) Methyl 2-amino-6-[([(4,6-dimethoxy-1,3,5-triazin-2-yl)amino]carbonylamino)sulfonyl]benzoat vom Schmp. 129 - 134 °C (Zers.).

### Beispiel A8

### Methyl 2-(diisobutylamino)-6-[([(4,6-dimethoxy-2-pyrimidinyl)amino]carbonylamino)sulfonyl]benzoat, Natriumsalz

In 5 ml Tetrahydrofuran werden 0.2 g (0.382 mmol) Methyl 2-(diisobutylamino)-6-[([(4,6-dimethoxy-2-pyrimidinyl)amino]carbonylamino)sulfonyl]benzoat eingerührt und anschließend 0.04 g (416 µmol) Natrium-tert.-butylat zugegeben. Nach 6 h wird der entstandene Feststoff abgesaugt und getrocknet. Man erhält 0.19 g (93 % d. Th.) Methyl 2-(diisobutylamino)-6-[([(4,6-dimethoxy-2-pyrimidinyl)amino]carbonylamino)sulfonyl]benzoat, Natriumsalz als farblosen Feststoff vom Schmp. 215-217 °C.

Die in den nachfolgenden Tabellen 1, 2 und 3 beschriebenen Verbindungen werden nach bzw. analog den obigen Beispielen A1- A8 erhalten.

Abkürzungen in den Tabellen 1, 2 und 3:
- Smp. =: Schmelzpunkt in °C
- (Z) =: Schmelzpunkt unter Zersetzung
- Bu =: n-Butyl; (entsprechend Pentyl = n-Pentyl, Hexyl = n-Hexyl)
- Et =: Ethyl
- Me =: Methyl
- Ph - =: Phenyl
- Pr, i-Pr, c-Pr =: n-Propyl, Isopropyl bzw. Cyclopropyl
Ein Diradikal wie Butylen der Formel 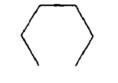 in den Spalten für R², R³ heißt, daß R² und R³ zusammen die Diradikalbrücke bedeuten und mit dem N-Atom der Gruppe R²R³N ein cyclisches Amin bilden.
- Het =: Heterocyclus, wobei Het für einen der Reste T1 bis T20 steht

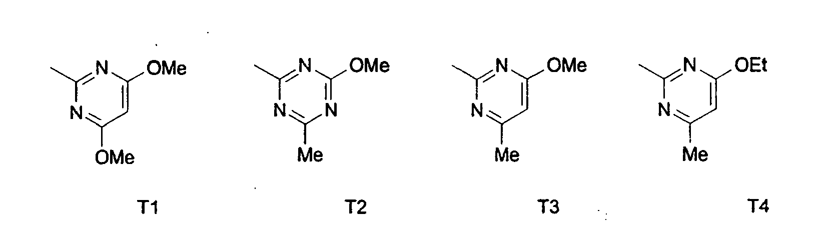

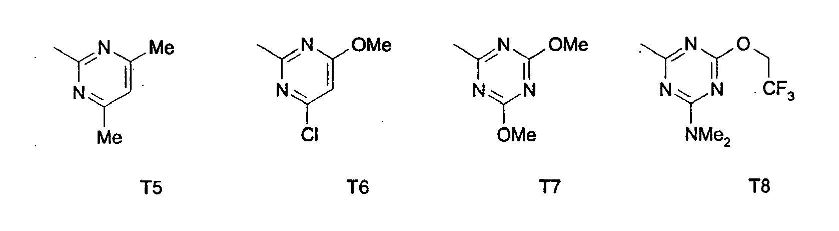

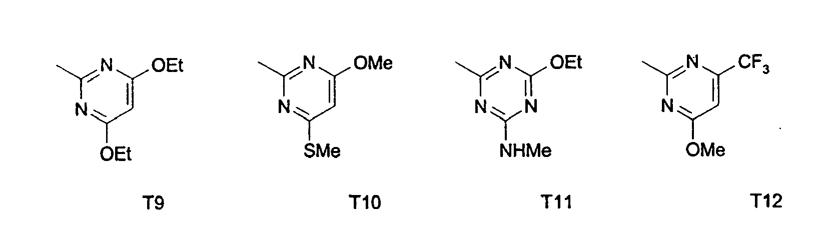

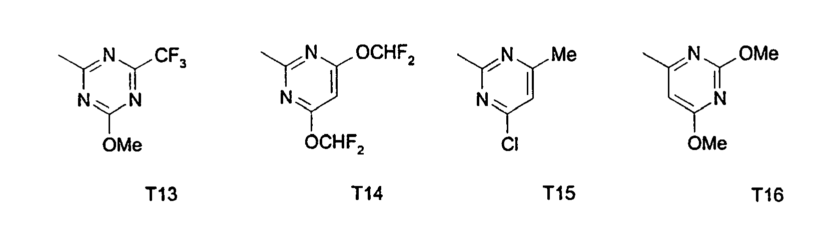

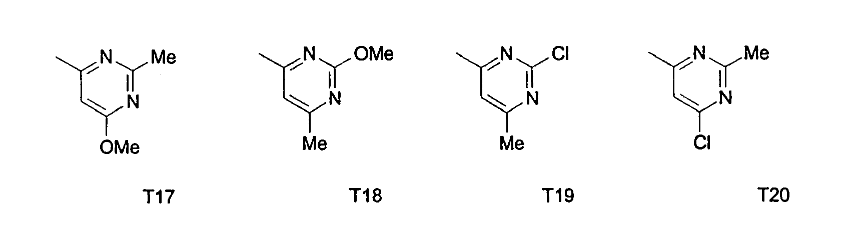

**Tabelle 1: Verbindungen der Formel (la)**

| | | | | | | | | |
|---|---|---|---|---|---|---|---|---|
| 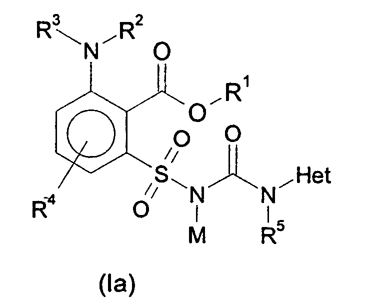 | | | | | | | | |

| **Bsp. Nr.** | **R¹** | **R²** | **R³** | **R⁴** | **R⁵** | **M** | **Het** | **Smp.** |
|---|---|---|---|---|---|---|---|---|
| 1-1. | H | H | H | - | H | H | T1 | |
| 1-2. | H | H | H | - | H | Na | T1 | |
| 1-3. | H | H | H | - | H | H | T2 | |
| 1-4. | H | H | H | - | H | Na | T2 | |
| 1-5. | H | H | H | - | H | H | T5 | |
| 1-6. | H | H | H | - | H | Na | T5 | |
| 1-7. | H | H | H | - | H | H | T6 | |
| 1-8. | H | H | H | - | H | Na | T6 | |
| 1-9. | H | H | H | - | H | H | T7 | 141-142 |
| 1-10. | H | H | H | - | H | Na | T7 | |
| 1-11. | Me | H | H | - | H | H | T1 | 171-173 |
| 1-12. | Me | H | H | - | H | Na | T1 | |
| 1-13. | Me | H | H | - | Me | H | T2 | |
| 1-14. | Me | H | H | - | Me | Na | T2 | |
| 1-15. | Me | H | H | - | H | H | T2 | 126-127 |
| | | | | | | | | (Z) |
| 1-16. | Me | H | H | - | H | Na | T2 | 239-242 |
| 1-17. | Me | H | H | - | H | H | T3 | 137-139 |
| | | | | | | | | (Z) |
| 1-18. | Me | H | H | - | H | Na | T3 | |
| 1-19. | Me | H | H | - | H | H | T4 | |
| 1-20. | Me | H | H | - | H | Na | T4 | |
| 1-21. | Me | H | H | - | H | H | T5 | |
| 1-22. | Me | H | H | - | H | Na | T5 | |
| 1-23. | Me | H | H | - | H | H | T6 | 137-139 |
| 1-24. | Me | H | H | - | H | Na | T6 | |
| 1-25. | Me | H | H | - | H | H | T7 | 129-134 |
| | | | | | | | | (Z) |
| 1-26. | Me | H | H | - | H | Na | T7 | 257-260 |
| | | | | | | | | (Z) |
| 1-27. | Me | H | H | - | Me | H | T7 | |
| 1-28. | Me | H | H | - | Me | Na | T7 | |
| 1-29. | Me | H | H | - | H | H | T8 | |
| 1-30. | Me | H | H | - | H | Na | T8 | |
| 1-31. | Me | H | H | - | H | H | T9 | |
| 1-32. | Me | H | H | - | H | Na | T9 | |
| 1-33. | Me | H | H | - | H | H | T10 | |
| 1-34. | Me | H | H | - | H | Na | T10 | |
| 1-35. | Me | H | H | - | H | H | T11 | |
| 1-36. | Me | H | H | - | H | Na | T11 | |
| 1-37. | Me | H | H | - | H | H | T12 | |
| 1-38. | Me | H | H | - | H | Na | T12 | |
| 1-39. | Me | H | H | - | H | H | T13 | |
| 1-40. | Me | H | H | - | H | Na | T13 | |
| 1-41. | Me | H | H | - | H | H | T14 | 162-164 |
| | | | | | | | | (Z) |
| 1-42. | Me | H | H | - | H | Na | T14 | |
| 1-43. | Me | H | H | - | H | H | T15 | |
| 1-44. | Me | H | H | - | H | Na | T15 | |
| 1-45. | Et | H | H | - | H | H | T1 | 164-166 |
| 1-46. | Et | H | H | - | H | Na | T1 | |
| 1-47. | Et | H | H | - | H | H | T2 | 149-151 |
| 1-48. | Et | H | H | - | H | Na | T2 | |
| 1-49. | Et | H | H | - | H | H | T3 | |
| 1-50. | Et | H | H | - | H | Na | T3 | |
| 1-51. | Et | H | H | - | H | H | T4 | |
| 1-52. | Et | H | H | - | H | Na | T4 | |
| 1-53. | Et | H | H | - | H | H | T5 | |
| 1-54. | Et | H | H | - | H | Na | T5 | |
| 1-55. | Et | H | H | - | H | H | T6 | 157-158 |
| 1-56. | Et | H | H | - | H | Na | T6 | |
| 1-57. | Et | H | H | - | H | H | T7 | 158-160 |
| 1-58. | Et | H | H | - | H | Na | T7 | |
| 1-59. | Et | H | H | - | H | H | T8 | |
| 1-60. | Et | H | H | - | H | Na | T8 | |
| 1-61. | Et | H | H | - | H | H | T9 | |
| 1-62. | Et | H | H | - | H | Na | T9 | |
| 1-63. | Et | H | H | - | H | H | T10 | |
| 1-64. | Et | H | H | - | H | Na | T10 | |
| 1-65. | Et | H | H | - | H | H | T11 | |
| 1-66. | Et | H | H | - | H | Na | T11 | |
| 1-67. | Et | H | H | - | H | H | T12 | |
| 1-68. | Et | H | H | - | H | Na | T12 | |
| 1-69. | Et | H | H | - | H | H | T13 | |
| 1-70. | Et | H | H | - | H | Na | T13 | |
| 1-71. | Et | H | H | - | H | H | T14 | |
| 1-72. | Et | H | H | - | H | Na | T14 | |
| 1-73. | Et | H | H | - | H | H | T15 | |
| 1-74. | Et | H | H | - | H | Na | T15 | |
| 1-75. | i-Pr | H | H | | H | H | T1 | |
| 1-76. | i-Pr | H | H | | H | Na | T1 | |
| 1-77. | i-Pr | H | H | | H | H | T2 | |
| 1-78. | i-Pr | H | H | | H | Na | T2 | |
| 1-79. | i-Pr | H | H | | H | H | T5 | |
| 1-80. | i-Pr | H | H | | H | Na | T5 | |
| 1-81. | i-Pr | H | H | | H | H | T6 | |
| 1-82. | i-Pr | H | H | | H | Na | T6 | |
| 1-83. | i-Pr | H | H | | H | H | T7 | |
| 1-84. | i-Pr | H | H | | H | Na | T7 | |
| 1-85. | Allyl | H | H | | H | H | T1 | |
| 1-86. | Allyl | H | H | | H | Na | T1 | |
| 1-87. | Allyl | H | H | | H | H | T2 | |
| 1-88. | Allyl | H | H | | H | Na | T2 | |
| 1-89. | Allyl | H | H | | H | H | T5 | |
| 1-90. | Allyl | H | H | | H | Na | T5 | |
| 1-91. | Allyl | H | H | | H | H | T6 | |
| 1-92. | Allyl | H | H | | H | Na | T6 | |
| 1-93. | Allyl | H | H | | H | H | T7 | |
| 1-94. | Allyl | H | H | | H | Na | T7 | |
| 1-95. | 3-Oxetanyl | H | H | | H | H | T1 | |
| 1-96. | 3-Oxetanyl | H | H | | H | Na | T1 | |
| 1-97. | 3-Oxetanyl | H | H | | H | H | T2 | |
| 1-98. | 3-Oxetanyl | H | H | | H | Na | T2 | |
| 1-99. | 3-Oxetanyl | H | H | | H | H | T5 | |
| 1-100. | 3-Oxetanyl | H | H | | H | Na | T5 | |
| 1-101. | 3-Oxetanyl | H | H | | H | H | T6 | |
| 1-102. | 3-Oxetanyl | H | H | | H | Na | T6 | |
| 1-103. | 3-Oxetanyl | H | H | | H | H | T7 | |
| 1-104. | 3-Oxetanyl | H | H | | H | Na | T7 | |
| 1-105. | Me | Me | H | | H | H | T1 | 169-171 |
| 1-106. | Me | Me | H | | H | Na | T1 | |
| 1-107. | Me | Me | H | | H | H | T2 | |
| 1-108. | Me | Me | H | | H | Na | T2 | |
| 1-109. | Me | Me | H | | H | H | T6 | |
| 1-110. | Me | Me | H | | H | Na | T6 | |
| 1-111. | Me | Me | H | | H | H | T7 | |
| 1-112. | Me | Me | H | | H | Na | T7 | |
| 1-113. | Et | Me | H | | H | H | T1 | |
| 1-114. | Et | Me | H | | H | Na | T1 | |
| 1-115. | Et | Me | H | | H | H | T2 | |
| 1-116. | Et | Me | H | | H | Na | T2 | |
| 1-117. | i-Pr | Me | H | | H | H | T1 | |
| 1-118. | i-Pr | Me | H | | H | Na | T1 | |
| 1-119. | i-Pr | Me | H | | H | H | T2 | |
| 1-120. | i-Pr | Me | H | | H | Na | T2 | |
| 1-121. | Allyl | Me | H | | H | H | T1 | |
| 1-122. | Allyl | Me | H | | H | Na | T1 | |
| 1-123. | Allyl | Me | H | | H | H | T2 | |
| 1-124. | Allyl | Me | H | | H | Na | T2 | |
| 1-125. | 3-Oxetanyl | Me | H | | H | H | T1 | |
| 1-126. | 3-Oxetanyl | Me | H | | H | Na | T1 | |
| 1-127. | 3-Oxetanyl | Me | H | | H | H | T2 | |
| 1-128. | 3-Oxetanyl | Me | H | | H | Na | T2 | |
| 1-129. | Me | Et | H | | H | H | T1 | 164-166 |
| 1-130. | Me | Et | H | | H | Na | T1 | |
| 1-131. | Me | Et | H | | H | H | T2 | 215-217 |
| 1-132. | Me | Et | H | | H | Na | T2 | |
| 1-133. | Me | Et | H | | H | H | T6 | |
| 1-134. | Me | Et | H | | H | Na | T6 | |
| 1-135. | Me | Et | H | | H | H | T7 | 170-172 |
| 1-136. | Me | Et | H | | H | Na | T7 | |
| 1-137. | Et | Et | H | | H | H | T1 | |
| 1-138. | Et | Et | H | | H | Na | T1 | |
| 1-139. | Et | Et | H | | H | H | T2 | |
| 1-140. | Et | Et | H | | H | Na | T2 | |
| 1-141. | i-Pr | Et | H | | H | H | T1 | |
| 1-142. | i-Pr | Et | H | | H | Na | T1 | |
| 1-143. | i-Pr | Et | H | | H | H | T2 | |
| 1-144. | i-Pr | Et | H | | H | Na | T2 | |
| 1-145. | Allyl | Et | H | | H | H | T1 | |
| 1-146. | Allyl | Et | H | | H | Na | T1 | |
| 1-147. | Allyl | Et | H | | H | H | T2 | |
| 1-148. | Allyl | Et | H | | H | Na | T2 | |
| 1-149. | 3-Oxetanyl | Et | H | | H | H | T1 | |
| 1-150. | 3-Oxetanyl | Et | H | | H | Na | T1 | |
| 1-151. | 3-Oxetanyl | Et | H | | H | H | T2 | |
| 1-152. | 3-Oxetanyl | Et | H | | H | Na | T2 | |
| 1-153. | Me | i-Pr | H | | H | H | T1 | 165-167 |
| 1-154. | Me | i-Pr | H | | H | Na | T1 | 124-126 |
| 1-155. | Me | i-Pr | H | | H | H | T2 | 139-140 |
| 1-156. | Me | i-Pr | H | | H | Na | T2 | (Z) |
| 1-157. | Me | i-Pr | H | | H | H | T6 | |
| 1-158. | Me | i-Pr | H | | H | Na | T6 | |
| 1-159. | Me | i-Pr | H | | H | H | T7 | 161-163 |
| 1-160. | Me | i-Pr | H | | H | Na | T7 | (Z) |
| 1-161. | Et | i-Pr | H | | H | H | T1 | |
| 1-162. | Et | i-Pr | H | | H | Na | T1 | |
| 1-163. | Et | i-Pr | H | | H | H | T2 | |
| 1-164. | Et | i-Pr | H | | H | Na | T2 | |
| 1-165. | i-Pr | i-Pr | H | | H | H | T1 | |
| 1-166. | i-Pr | i-Pr | H | | H | Na | T1 | |
| 1-167. | i-Pr | i-Pr | H | | H | H | T2 | |
| 1-168. | i-Pr | i-Pr | H | | H | Na | T2 | |
| 1-169. | Allyl | i-Pr | H | | H | H | T1 | |
| 1-170. | Allyl | i-Pr | H | | H | Na | T1 | |
| 1-171. | Allyl | i-Pr | H | | H | H | T2 | |
| 1-172. | Allyl | i-Pr | H | | H | Na | T2 | |
| 1-173. | 3-Oxetanyl | i-Pr | H | | H | H | T1 | |
| 1-174. | 3-Oxetanyl | i-Pr | H | | H | Na | T1 | |
| 1-175. | 3-Oxetanyl | i-Pr | H | | H | H | T2 | |
| 1-176. | 3-Oxetanyl | i-Pr | H | | H | Na | T2 | |
| 1-177. | Me | CH₂i-Pr | H | | H | H | T1 | 163-165 |
| 1-178. | Me | CH₂i-Pr | H | | H | Na | T1 | |
| 1-179. | Me | CH₂i-Pr | H | | H | H | T2 | 171-173 |
| | | | | | | | | (Z) |
| 1-180. | Me | CH₂i-Pr | H | | H | Na | T2 | |
| 1-181. | Me | CH₂i-Pr | H | | H | H | T6 | |
| 1-182. | Me | CH₂i-Pr | H | | H | Na | T6 | |
| 1-183. | Me | CH₂i-Pr | H | | H | H | T7 | 174-176 |
| | | | | | | | | (Z) |
| 1-184. | Me | CH₂i-Pr | H | | H | Na | T7 | |
| 1-185. | Et | CH₂i-Pr | H | | H | H | T1 | |
| 1-186. | Et | CH₂i-Pr | H | | H | Na | T1 | |
| 1-187. | Et | CH₂i-Pr | H | | H | H | T2 | |
| 1-188. | Et | CH₂i-Pr | H | | H | Na | T2 | |
| 1-189. | Et | CH₂i-Pr | H | | H | H | T6 | |
| 1-190. | Et | CH₂i-Pr | H | | H | Na | T6 | |
| 1-191. | Et | CH₂i-Pr | H | | H | H | T7 | |
| 1-192. | Et | CH₂i-Pr | H | | H | Na | T7 | |
| 1-193. | i-Pr | CH₂i-Pr | H | | H | H | T1 | |
| 1-194. | i-Pr | CH₂i-Pr | H | | H | Na | T1 | |
| 1-195. | i-Pr | CH₂i-Pr | H | | H | H | T2 | |
| 1-196. | i-Pr | CH₂i-Pr | H | | H | Na | T2 | |
| 1-197. | Allyl | CH₂i-Pr | H | | H | H | T1 | |
| 1-198. | Allyl | CH₂i-Pr | H | | H | Na | T1 | |
| 1-199. | Allyl | CH₂i-Pr | H | | H | H | T2 | |
| 1-200. | Allyl | CH₂i-Pr | H | | H | Na | T2 | |
| 1-201. | 3-Oxetanyl | CH₂i-Pr | H | | H | H | T1 | |
| 1-202. | 3-Oxetanyl | CH₂i-Pr | H | | H | Na | T1 | |
| 1-203. | 3-Oxetanyl | CH₂i-Pr | H | | H | H | T2 | |
| 1-204. | 3-Oxetanyl | CH₂i-Pr | H | | H | Na | T2 | |
| 1-205. | Me | CH₂CH₂F | H | | H | H | T1 | |
| 1-206. | Me | CH₂CH₂F | H | | H | Na | T1 | |
| 1-207. | Me | CH₂CH₂F | H | | H | H | T2 | |
| 1-208. | Me | CH₂CH₂F | H | | H | Na | T2 | |
| 1-209. | Me | CH₂CH₂F | H | | H | H | T6 | |
| 1-210. | Me | CH₂CH₂F | H | | H | Na | T6 | |
| 1-211. | Me | CH₂CH₂F | H | | H | H | T7 | |
| 1-212. | Me | CH₂CH₂F | H | | H | Na | T7 | |
| 1-213. | Et | CH₂CH₂F | H | | H | H | T1 | |
| 1-214. | Et | CH₂CH₂F | H | | H | Na | T1 | |
| 1-215. | Et | CH₂CH₂F | H | | H | H | T2 | |
| 1-216. | Et | CH₂CH₂F | H | | H | Na | T2 | |
| 1-217. | Et | CH₂CH₂F | H | | H | H | T6 | |
| 1-218. | Et | CH₂CH₂F | H | | H | Na | T6 | |
| 1-219. | Et | CH₂CH₂F | H | | H | H | T7 | |
| 1-220. | Et | CH₂CH₂F | H | | H | Na | T7 | |
| 1-221. | i-Pr | CH₂CH₂F | H | | H | H | T1 | |
| 1-222. | i-Pr | CH₂CH₂F | H | | H | Na | T1 | |
| 1-223. | i-Pr | CH₂CH₂F | H | | H | H | T2 | |
| 1-224. | i-Pr | CH₂CH₂F | H | | H | Na | T2 | |
| 1-225. | Allyl | CH₂CH₂F | H | | H | H | T1 | |
| 1-226. | Allyl | CH₂CH₂F | H | | H | Na | T1 | |
| 1-227. | Allyl | CH₂CH₂F | H | | H | H | T2 | |
| 1-228. | Allyl | CH₂CH₂F | H | | H | Na | T2 | |
| 1-229. | 3-Oxetanyl | CH₂CH₂F | H | | H | H | T1 | |
| 1-230. | 3-Oxetanyl | CH₂CH₂F | H | | H | Na | T1 | |
| 1-231. | 3-Oxetanyl | CH₂CH₂F | H | | H | H | T2 | |
| 1-232. | 3-Oxetanyl | CH₂CH₂F | H | | H | Na | T2 | |
| 1-233. | Me | CH₂CH₂CF₃ | H | | H | H | T1 | |
| 1-234. | Me | CH₂CH₂CF₃ | H | | H | Na | T1 | |
| 1-235. | Me | CH₂CH₂CF₃ | H | | H | H | T2 | |
| 1-236. | Me | CH₂CH₂CF₃ | H | | H | Na | T2 | |
| 1-237. | Me | CH₂CH₂CF₃ | H | | H | H | T6 | |
| 1-238. | Me | CH₂CH₂CF₃ | H | | H | Na | T6 | |
| 1-239. | Me | CH₂CH₂CF₃ | H | | H | H | T7 | |
| 1-240. | Me | CH₂CH₂CF₃ | H | | H | Na | T7 | |
| 1-241. | Me | CH₂CH₂CH₂CF₃ | H | | H | H | T1 | 173-175 |
| 1-242. | Me | CH₂CH₂CH₂CF₃ | H | | H | Na | T1 | |
| 1-243. | Me | CH₂CH₂CH₂CF₃ | H | | H | H | T2 | 177-179 |
| 1-244. | Me | CH₂CH₂CH₂CF₃ | H | | H | Na | T2 | |
| 1-245. | Me | CH₂CH₂CH₂CF₃ | H | | H | H | T6 | |
| 1-246. | Me | CH₂CH₂CH₂CF₃ | H | | H | Na | T6 | |
| 1-247. | Me | CH₂CH₂CH₂CF₃ | H | | H | H | T7 | 171-173 |
| 1-248. | Me | CH₂CH₂CH₂CF₃ | H | | H | Na | T7 | |
| 1-249. | Me | CH(Me)CH₂C(O)O | H | | H | H | T1 | |
| | | Me | | | | | | |
| 1-250. | Me | CH(Me)CH₂C(O)O | H | | H | Na | T1 | |
| | | Me | | | | | | |
| 1-251. | Me | CH(Me)CH₂C(O)O | H | | H | H | T2 | |
| | | Me | | | | | | |
| 1-252. | Me | CH(Me)CH₂C(O)O | H | | H | Na | T2 | |
| | | Me | | | | | | |
| 1-253. | Me | CH(Me)CH₂C(O)O | H | | H | H | T6 | |
| | | Me | | | | | | |
| 1-254. | Me | CH(Me)CH₂C(O)O | H | | H | Na | T6 | |
| | | Me | | | | | | |
| 1-255. | Me | CH(Me)CH₂C(O)O | H | | H | H | T7 | |
| | | Me | | | | | | |
| 1-256. | Me | CH(Me)CH₂C(O)O | H | | H | Na | T7 | |
| | | Me | | | | | | |
| 1-257. | Me | CH(Me)CH₂C(O)N | H | | H | H | T1 | |
| | | H₂ | | | | | | |
| 1-258. | Me | CH(Me)CH₂C(O)N | H | | H | Na | T1 | |
| | | H₂ | | | | | | |
| 1-259. | Me | CH(Me)CH₂C(O)N | H | | H | H | T2 | |
| | | H₂ | | | | | | |
| 1-260. | Me | CH(Me)CH₂C(O)N | H | | H | Na | T2 | |
| | | H₂ | | | | | | |
| 1-261. | Me | CH(Me)CH₂C(O)N | H | | H | H | T6 | |
| | | H₂ | | | | | | |
| 1-262. | Me | CH(Me)CH₂C(O)N | H | | H | Na | T6 | |
| | | H₂ | | | | | | |
| 1-263. | Me | CH(Me)CH₂C(O)N | H | | H | H | T7 | |
| | | H₂ | | | | | | |
| 1-264. | Me | CH(Me)CH₂C(O)N | H | | H | Na | T7 | |
| | | H₂ | | | | | | |
| 1-265. | Me | CH(Me)CH₂CH₃ | H | | H | H | T1 | 171-173 |
| 1-266. | Me | CH(Me)CH₂CH₃ | H | | H | Na | T1 | |
| 1-267. | Me | CH(Me)CH₂CH₃ | H | | H | H | T2 | 154-156 |
| 1-268. | Me | CH(Me)CH₂CH₃ | H | | H | Na | T2 | |
| 1-269. | Me | CH(Me)CH₂CH₃ | H | | H | H | T6 | |
| 1-270. | Me | CH(Me)CH₂CH₃ | H | | H | Na | T6 | |
| 1-271. | Me | CH(Me)CH₂CH₃ | H | | H | H | T7 | 172-174 |
| 1-272. | Me | CH(Me)CH₂CH₃ | H | | H | Na | T7 | |
| 1-273. | Me | Allyl | H | | H | H | T1 | |
| 1-274. | Me | Allyl | H | | H | Na | T1 | |
| 1-275. | Me | Allyl | H | | H | H | T2 | |
| 1-276. | Me | Allyl | H | | H | Na | T2 | |
| 1-277. | Me | Allyl | H | | H | H | T6 | |
| 1-278. | Me | Allyl | H | | H | Na | T6 | |
| 1-279. | Me | Allyl | H | | H | H | T7 | |
| 1-280. | Me | Allyl | H | | H | Na | T7 | |
| 1-281. | Et | Allyl | H | | H | H | T1 | |
| 1-282. | Et | Allyl | H | | H | Na | T1 | |
| 1-283. | Et | Allyl | H | | H | H | T2 | |
| 1-284. | Et | Allyl | H | | H | Na | T2 | |
| 1-285. | Et | Allyl | H | | H | H | T6 | |
| 1-286. | Et | Allyl | H | | H | Na | T6 | |
| 1-287. | Et | Allyl | H | | H | H | T7 | |
| 1-288. | Et | Allyl | H | | H | Na | T7 | |
| 1-289. | i-Pr | Allyl | H | | H | H | T1 | |
| 1-290. | i-Pr | Allyl | H | | H | Na | T1 | |
| 1-291. | i-Pr | Allyl | H | | H | H | T2 | |
| 1-292. | i-Pr | Allyl | H | | H | Na | T2 | |
| 1-293. | Allyl | Allyl | H | | H | H | T1 | |
| 1-294. | Allyl | Allyl | H | | H | Na | T1 | |
| 1-295. | Allyl | Allyl | H | | H | H | T2 | |
| 1-296. | Allyl | Allyl | H | | H | Na | T2 | |
| 1-297. | 3-Oxetanyl | Allyl | H | | H | H | T1 | |
| 1-298. | 3-Oxetanyl | Allyl | H | | H | Na | T1 | |
| 1-299. | 3-Oxetanyl | Allyl | H | | H | H | T2 | |
| 1-300. | 3-Oxetanyl | Allyl | H | | H | Na | T2 | |
| 1-301. | Me | Propargyl | H | | H | H | T1 | |
| 1-302. | Me | Propargyl | H | | H | Na | T1 | |
| 1-303. | Me | Propargyl | H | | H | H | T2 | |
| 1-304. | Me | Propargyl | H | | H | Na | T2 | |
| 1-305. | Me | Propargyl | H | | H | H | T6 | |
| 1-306. | Me | Propargyl | H | | H | Na | T6 | |
| 1-307. | Me | Propargyl | H | | H | H | T7 | |
| 1-308. | Me | Propargyl | H | | H | Na | T7 | |
| 1-309. | Et | Propargyl | H | | H | H | T1 | |
| 1-310. | Et | Propargyl | H | | H | Na | T1 | |
| 1-311. | Et | Propargyl | H | | H | H | T2 | |
| 1-312. | Et | Propargyl | H | | H | Na | T2 | |
| 1-313. | Et | Propargyl | H | | H | H | T6 | |
| 1-314. | Et | Propargyl | H | | H | Na | T6 | |
| 1-315. | Et | Propargyl | H | | H | H | T7 | |
| 1-316. | Et | Propargyl | H | | H | Na | T7 | |
| 1-317. | i-Pr | Propargyl | H | | H | H | T1 | |
| 1-318. | i-Pr | Propargyl | H | | H | Na | T1 | |
| 1-319. | i-Pr | Propargyl | H | | H | H | T2 | |
| 1-320. | i-Pr | Propargyl | H | | H | Na | T2 | |
| 1-321. | Allyl | Propargyl | H | | H | H | T1 | |
| 1-322. | Allyl | Propargyl | H | | H | Na | T1 | |
| 1-323. | Allyl | Propargyl | H | | H | H | T2 | |
| 1-324. | Allyl | Propargyl | H | | H | Na | T2 | |
| 1-325. | 3-Oxetanyl | Propargyl | H | | H | H | T1 | |
| 1-326. | 3-Oxetanyl | Propargyl | H | | H | Na | T1 | |
| 1-327. | 3-Oxetanyl | Propargyl | H | | H | H | T2 | |
| 1-328. | 3-Oxetanyl | Propargyl | H | | H | Na | T2 | |
| 1-329. | Me | OMe | H | | H | H | T1 | |
| 1-330. | Me | OMe | H | | H | Na | T1 | |
| 1-331. | Me | OMe | H | | H | H | T2 | |
| 1-332. | Me | OMe | H | | H | Na | T2 | |
| 1-333. | Me | OMe | H | | H | H | T6 | |
| 1-334. | Me | OMe | H | | H | Na | T6 | |
| 1-335. | Me | OMe | H | | H | H | T7 | |
| 1-336. | Me | OMe | H | | H | Na | T7 | |
| 1-337. | Et | OMe | H | | H | H | T1 | |
| 1-338. | Et | OMe | H | | H | Na | T1 | |
| 1-339. | Et | OMe | H | | H | H | T2 | |
| 1-340. | Et | OMe | H | | H | Na | T2 | |
| 1-341. | Et | OMe | H | | H | H | T6 | |
| 1-342. | Et | OMe | H | | H | Na | T6 | |
| 1-343. | Et | OMe | H | | H | H | T7 | |
| 1-344. | Et | OMe | H | | H | Na | T7 | |
| 1-345. | i-Pr | OMe | H | | H | H | T1 | |
| 1-346. | i-Pr | OMe | H | | H | Na | T1 | |
| 1-347. | i-Pr | OMe | H | | H | H | T2 | |
| 1-348. | i-Pr | OMe | H | | H | Na | T2 | |
| 1-349. | Allyl | OMe | H | | H | H | T1 | |
| 1-350. | Allyl | OMe | H | | H | Na | T1 | |
| 1-351. | Allyl | OMe | H | | H | H | T2 | |
| 1-352. | Allyl | OMe | H | | H | Na | T2 | |
| 1-353. | Me | c-Pr | H | | H | H | T1 | 148-150 |
| 1-354. | Me | c-Pr | H | | H | Na | T1 | (Z) |
| 1-355. | Me | c-Pr | H | | H | H | T2 | 147-149 |
| 1-356. | Me | c-Pr | H | | H | Na | T2 | |
| 1-357. | Me | c-Pr | H | | H | H | T6 | |
| 1-358. | Me | c-Pr | H | | H | Na | T6 | |
| 1-359. | Me | c-Pr | H | | H | H | T7 | 177-179 |
| 1-360. | Me | c-Pr | H | | H | Na | T7 | |
| 1-361. | 3-Oxetanyl | OMe | H | | H | H | T1 | |
| 1-362. | 3-Oxetanyl | OMe | H | | H | Na | T1 | |
| 1-363. | 3-Oxetanyl | OMe | H | | H | H | T2 | |
| 1-364. | 3-Oxetanyl | OMe | H | | H | Na | T2 | |
| 1-365. | Me | NMe₂ | H | | H | H | T1 | |
| 1-366. | Me | NMe₂ | H | | H | Na | T1 | |
| 1-367. | Me | NMe₂ | H | | H | H | T2 | |
| 1-368. | Me | NMe₂ | H | | H | Na | T2 | |
| 1-369. | Me | NHCHO | H | | H | H | T1 | |
| 1-370. | Me | NHCHO | H | | H | Na | T1 | |
| 1-371. | Me | NHCHO | H | | H | H | T2 | |
| 1-372. | Me | NHCHO | H | | H | Na | T2 | |
| 1-373. | Me | NHC(O)Me | H | | H | H | T1 | |
| 1-374. | Me | NHC(O)Me | H | | H | Na | T1 | |
| 1-375. | Me | NHC(O)Me | H | | H | H | T2 | |
| 1-376. | Me | NHC(O)Me | H | | H | Na | T2 | |
| 1-377. | Me | NHSO₂Me | H | | H | H | T1 | |
| 1-378. | Me | NHSO₂Me | H | | H | Na | T1 | |
| 1-379. | Me | NHSO₂Me | H | | H | H | T2 | |
| 1-380. | Me | NHSO₂Me | H | | H | Na | T2 | |
| 1-381. | Me | CH₂Ph | H | | H | H | T1 | 189-191 |
| 1-382. | Me | CH₂Ph | H | | H | Na | T1 | |
| 1-383. | Me | CH₂Ph | H | | H | H | T2 | 151-153 |
| 1-384. | Me | CH₂Ph | H | | H | Na | T2 | |
| 1-385. | Me | CH₂Ph | H | | H | H | T6 | |
| 1-386. | Me | CH₂Ph | H | | H | Na | T6 | |
| 1-387. | Me | CH₂Ph | H | | H | H | T7 | 166-168 |
| 1-388. | Me | CH₂Ph | H | | H | Na | T7 | (Z) |
| 1-389. | Me | CH₂OMe | H | | H | H | T1 | |
| 1-390. | Me | CH₂OMe | H | | H | Na | T1 | |
| 1-391. | Me | CH₂OMe | H | | H | H | T2 | |
| 1-392. | Me | CH₂OMe | H | | H | Na | T2 | |
| 1-393. | Me | CH₂C(O)Me | H | | H | H | T1 | |
| 1-394. | Me | CH₂C(O)Me | H | | H | Na | T1 | |
| 1-395. | Me | CH₂C(O)Me | H | | H | H | T2 | |
| 1-396. | Me | CH₂C(O)Me | H | | H | Na | T2 | |
| 1-397. | Me | CH₂C(O)OMe | H | | H | H | T1 | |
| 1-398. | Me | CH₂C(O)OMe | H | | H | Na | T1 | |
| 1-399. | Me | CH₂C(O)OMe | H | | H | H | T2 | |
| 1-400. | Me | CH₂C(O)OMe | H | | H | Na | T2 | |
| 1-401. | Me | Me | Me | | H | H | T1 | 194-196 |
| 1-402. | Me | Me | Me | | H | Na | T1 | |
| 1-403. | Me | Me | Me | | H | H | T2 | |
| 1-404. | Me | Me | Me | | H | Na | T2 | |
| 1-405. | Me | Me | Me | | H | H | T6 | |
| 1-406. | Me | Me | Me | | H | Na | T6 | |
| 1-407. | Me | Me | Me | | H | H | T7 | |
| 1-408. | Me | Me | Me | | H | Na | T7 | |
| 1-409. | Me | Me | Et | | H | H | T1 | |
| 1-410. | Me | Me | Et | | H | Na | T1 | |
| 1-411. | Me | Me | Et | | H | H | T2 | |
| 1-412. | Me | Me | Et | | H | Na | T2 | |
| 1-413. | Me | Me | i-Pr | | H | H | T1 | |
| 1-414. | Me | Me | i-Pr | | H | Na | T1 | |
| 1-415. | Me | Me | i-Pr | | H | H | T2 | |
| 1-416. | Me | Me | i-Pr | | H | Na | T2 | |
| 1-417. | Me | Et | Et | | H | H | T1 | 207-209 |
| 1-418. | Me | Et | Et | | H | Na | T1 | |
| 1-419. | Me | Et | Et | | H | H | T2 | |
| 1-420. | Me | Et | Et | | H | Na | T2 | |
| 1-421. | Me | i-Pr | i-Pr | | H | H | T1 | |
| 1-422. | Me | i-Pr | i-Pr | | H | Na | T1 | |
| 1-423. | Me | i-Pr | i-Pr | | H | H | T2 | |
| 1-424. | Me | i-Pr | i-Pr | | H | Na | T2 | |
| 1-425. | Me | CH₂i-Pr | CH₂i- | | H | H | T1 | 166-167 |
| | | | Pr | | | | | |
| 1-426. | Me | CH₂i-Pr | CH₂i- | | H | Na | T1 | 215-217 |
| | | | Pr | | | | | |
| 1-427. | Me | CH₂i-Pr | CH₂i-Pr | - | H | H | T2 | |
| 1-428. | Me | CH₂i-Pr | CH₂i-Pr | - | H | Na | T2 | |
| 1-429. | Me | 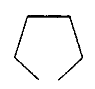 | | - | H | H | T1 | |
| 1-430. | Me | " | | - | H | Na | T1 | |
| 1-431. | Me | " | | - | H | H | T2 | |
| 1-432. | Me | " | | - | H | Na | T2 | |
| 1-433. | Me | 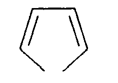 | | - | H | H | T1 | 216-218 |
| 1-434. | Me | " | | - | H | Na | T1 | |
| 1-435. | Me | " | | - | H | H | T2 | 193-195 |
| 1-436. | Me | " | | - | H | Na | T2 | |
| 1-437. | Me | " | | - | H | H | T6 | |
| 1-438. | Me | " | | - | H | Na | T6 | |
| 1-439. | Me | " | | - | H | H | T7 | 200-202 |
| 1-440. | Me | " | | - | H | Na | T7 | |
| 1-441. | Me | 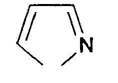 | | - | H | H | T1 | |
| 1-442. | Me | " | | - | H | Na | T1 | |
| 1-443. | Me | " | | - | H | H | T2 | |
| 1-444. | Me | " | | - | H | Na | T2 | |
| 1-445. | Me | 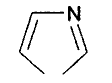 | | - | H | H | T1 | |
| 1-446. | Me | " | | - | H | Na | T1 | |
| 1-447. | Me | " | | - | H | H | T2 | |
| 1-448. | Me | " | | - | H | Na | T2 | |
| 1-449. | Me | 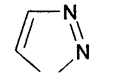 | | - | H | H | T1 | |
| 1-450. | Me | " | | - | H | Na | T1 | |
| 1-451. | Me | " | | - | H | H | T2 | |
| 1-452. | Me | " | | - | H | Na | T2 | |
| 1-453. | Me | 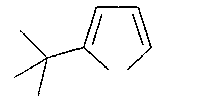 | | - | H | H | T1 | 211-213 |
| | | | | | | | | (Z) |
| 1-454. | Me | " | | - | H | Na | T1 | |
| 1-455. | Me | " | | - | H | H | T2 | 208-209 |
| 1-456. | Me | " | | - | H | Na | T2 | |
| 1-457. | Me | " | | - | H | H | T6 | |
| 1-458. | Me | " | | - | H | Na | T6 | |
| 1-459. | Me | " | | - | H | H | T7 | 201-203 |
| 1-460. | Me | " | | - | H | Na | T7 | |
| 1-461. | Me | 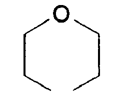 | | - | H | H | T1 | |
| 1-462. | Me | " | | - | H | Na | T1 | |
| 1-463. | Me | " | | - | H | H | T2 | |
| 1-464. | Me | " | | - | H | Na | T2 | |
| 1-465. | Me | H | H | 5-Me | H | H | T1 | |
| 1-466. | Me | H | H | 5-Me | H | Na | T1 | |
| 1-467. | Me | H | H | 5-Me | H | H | T2 | |
| 1-468. | Me | H | H | 5-Me | H | Na | T2 | |
| 1-469. | Me | H | H | 5-F | H | H | T1 | |
| 1-470. | Me | H | H | 5-F | H | Na | T1 | |
| 1-471. | Me | H | H | 5-F | H | H | T2 | |
| 1-472. | Me | H | H | 5-F | H | Na | T2 | |
| 1-473. | Me | H | H | 5-Cl | H | H | T1 | |
| 1-474. | Me | H | H | 5-Cl | H | Na | T1 | |
| 1-475. | Me | H | H | 5-Cl | H | H | T2 | |
| 1-476. | Me | H | H | 5-Cl | H | Na | T2 | |
| 1-477. | Me | H | H | 5-OMe | H | H | T1 | |
| 1-478. | Me | H | H | 5-OMe | H | Na | T1 | |
| 1-479. | Me | H | H | 5-OMe | H | H | T2 | |
| 1-480. | Me | H | H | 5-OMe | H | Na | T2 | |
| 1-481. | Me | H | H | 5-NO₂ | H | H | T1 | |
| 1-482. | Me | H | H | 5-NO₂ | H | Na | T1 | |
| 1-483. | Me | H | H | 5-NO₂ | H | H | T2 | |
| 1-484. | Me | H | H | 5-NO₂ | H | Na | T2 | |
| 1-485. | Me | H | H | 6-Me | H | H | T1 | |
| 1-486. | Me | H | H | 6-Me | H | Na | T1 | |
| 1-487. | Me | H | H | 6-Me | H | H | T2 | |
| 1-488. | Me | H | H | 6-Me | H | Na | T2 | |
| 1-489. | Me | H | H | 6-Cl | H | H | T1 | |
| 1-490. | Me | H | H | 6-Cl | H | Na | T1 | |
| 1-491. | Me | H | H | 6-Cl | H | H | T2 | |
| 1-492. | Me | H | H | 6-Cl | H | Na | T2 | |

**Tabelle 2: Verbindungen der Formel (1b)**

| | | | | | | | | |
|---|---|---|---|---|---|---|---|---|
| 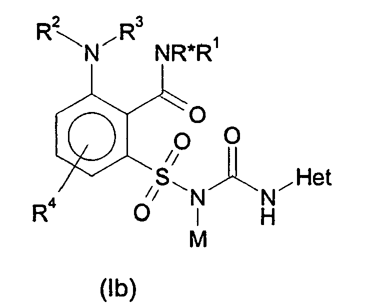 | | | | | | | | |

| **Bsp. Nr.** | **R¹** | **R*** | **R²** | **R³** | **R⁴** | **M** | **Het** | **Smp.** |
|---|---|---|---|---|---|---|---|---|
| 2-1. | H | H | H | H | - | H | T1 | |
| 2-2. | H | H | H | H | - | Na | T1 | |
| 2-3. | H | H | H | H | - | H | T2 | |
| 2-4. | H | H | H | H | - | Na | T2 | |
| 2-5. | Me | Me | H | H | - | H | T1 | |
| 2-6. | Me | Me | H | H | - | Na | T1 | |
| 2-7. | Me | Me | H | H | - | H | T2 | |
| 2-8. | Me | Me | H | H | - | Na | T2 | |
| 2-9. | Me | Me | H | H | - | H | T5 | |
| 2-10. | Me | Me | H | H | - | Na | T5 | |
| 2-11. | Me | Me | H | H | - | H | T6 | |
| 2-12. | Me | Me | H | H | - | Na | T6 | |
| 2-13. | Me | Me | H | H | - | H | T7 | |
| 2-14. | Me | Me | H | H | - | Na | T7 | |
| 2-15. | Me | Me | Me | H | - | H | T1 | |
| 2-16. | Me | Me | Me | H | - | Na | T1 | |
| 2-17. | Me | Me | Me | H | - | H | T2 | |
| 2-18. | Me | Me | Me | H | - | Na | T2 | |
| 2-19. | Me | Me | Me | H | - | H | T5 | |
| 2-20. | Me | Me | Me | H | - | Na | T5 | |
| 2-21. | Me | Me | Me | H | - | H | T6 | |
| 2-22. | Me | Me | Me | H | - | Na | T6 | |
| 2-23. | Me | Me | Me | H | - | H | T7 | |
| 2-24. | Me | Me | Me | H | - | Na | T7 | |
| 2-25. | Me | Me | H | H | 5-Me | H | T1 | |
| 2-26. | Me | Me | H | H | 5-Me | Na | T1 | |
| 2-27. | Me | Me | H | H | 5-Me | H | T2 | |
| 2-28. | Me | Me | H | H | 5-Me | Na | T2 | |
| 2-29. | Me | Me | H | H | 5-F | H | T1 | |
| 2-30. | Me | Me | H | H | 5-F | Na | T1 | |
| 2-31. | Me | Me | H | H | 5-F | H | T2 | |
| 2-32. | Me | Me | H | H | 5-F | Na | T2 | |
| 2-33. | Me | Me | H | H | 5-Cl | H | T1 | |
| 2-34. | Me | Me | H | H | 5-Cl | Na | T1 | |
| 2-35. | Me | Me | H | H | 5-OMe | H | T1 | |
| 2-36. | Me | Me | H | H | 5-OMe | Na | T1 | |
| 2-37. | Me | Me | H | H | 5-NO₂ | H | T1 | |
| 2-38. | Me | Me | H | H | 5-NO₂ | Na | T1 | |
| 2-39. | Me | Me | H | H | 6-Me | H | T1 | |
| 2-40. | Me | Me | H | H | 6-Me | Na | T1 | |
| 2-41. | Me | Me | H | H | 6-Cl | H | T1 | |
| 2-42. | Me | Me | H | H | 6-Cl | Na | T1 | |
| 2-43. | Me | Me | Me | H | 5-Me | H | T1 | |
| 2-44. | Me | Me | Me | H | 5-Me | Na | T1 | |
| 2-45. | Me | Me | Me | H | 5-Cl | H | T1 | |
| 2-46. | Me | Me | Me | H | 5-Cl | Na | T1 | |
| 2-47. | Me | Me | Me | H | 5-OMe | H | T1 | |
| 2-48. | Me | Me | Me | H | 5-OMe | Na | T1 | |
| 2-49. | Me | Me | Me | H | 5-NO₂ | H | T1 | |
| 2-50. | Me | Me | Me | H | 5-NO₂ | Na | T1 | |
| 2-51. | Me | Me | Me | H | 6-Me | H | T1 | |
| 2-52. | Me | Me | Me | H | 6-Me | Na | T1 | |
| 2-53. | Me | Me | Me | H | 6-Cl | H | T1 | |
| 2-54. | Me | Me | Me | H | 6-Cl | Na | T1 | |

**Tabelle 3: Verbindungen der Formel (VIII)**

| | | | | | | |
|---|---|---|---|---|---|---|
| 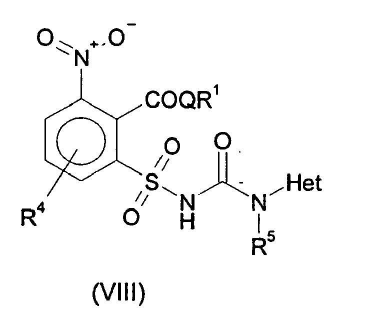 | | | | | | |

| **Bsp. Nr.** | **R¹** | **Q** | **R⁴** | **R⁵** | **Het** | **Smp.** |
|---|---|---|---|---|---|---|
| 3-1. | Me | O | - | H | T1 | |
| 3-2. | Me | O | - | Me | T1 | 161-163 |
| 3-3. | Me | O | - | H | T2 | 212-214 |
| 3-4. | Me | O | - | H | T3 | 218-220 |
| 3-5. | Me | O | - | H | T4 | |
| 3-6. | Me | O | - | H | T5 | |
| 3-7. | Me | O | - | H | T6 | |
| 3-8. | Me | O | - | H | T7 | 211-213 |
| 3-9. | Me | O | - | H | T8 | |
| 3-10. | Me | O | - | H | T9 | |
| 3-11. | Me | O | - | H | T10 | |
| 3-12. | Me | O | - | H | T11 | |
| 3-13. | Me | O | - | H | T12 | |
| 3-14. | Me | O | - | H | T13 | |
| 3-15. | Me | O | - | H | T14 | |
| 3-16. | Me | O | - | H | T15 | |
| 3-17. | Me | O | - | H | T16 | |
| 3-18. | Me | O | - | H | T17 | |
| 3-19. | Me | O | - | H | T18 | |
| 3-20. | Me | O | - | H | T19 | |
| 3-21. | Me | O | - | H | T20 | |
| 3-22. | Et | O | - | H | T1 | 223-225 |
| 3-23. | Et | O | - | H | T2 | 194-196 |
| 3-24. | Et | O | - | H | T6 | |
| 3-25. | Et | O | - | H | T7 | |
| 3-26. | Me | NMe | - | H | T1 | |
| 3-27. | Me | NMe | - | H | T2 | |
| 3-28. | Me | NMe | - | H | T6 | |
| 3-29. | Me | NMe | - | H | T7 | |

### B. Formulierungsbeispiele

a) Ein Stäubemittel wird erhalten, indem man 10 Gew.-Teile einer Verbindung der Formel (I) und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I), 64 Gew.-Teile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gew.-Teile einer Verbindung der Formel (I) mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I), 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man
   75 Gew.-Teile einer Verbindung der Formel (1),
   10 Gew.-Teile ligninsulfonsaures Calcium,
   5 Gew.-Teile Natriumlaurylsulfat,
   3 Gew.-Teile Polyvinylalkohol und
   7 Gew.-Teile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   25 Gew.-Teile einer Verbindung der Formel (I),
   5 Gew.-Teile 2,2'-dinaphthylmethan-6,6'-disulfonsaures Natrium
   2 Gew.-Teile oleoylmethyltaurinsaures Natrium,
   1 Gew.-Teil Polyvinylalkohol,
   17 Gew.-Teile Calciumcarbonat und
   50 Gew.-Teile Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Unkrautwirkung im Vorauflauf

Samen bzw. Rhizomstücke von mono- und dikotylen Unkrautpflanzen wurden in Papptöpfen in sandiger Lehmerde ausgelegt und mit Erde abgedeckt. Die in Form von benetzbaren Pulvern oder Emulsionskonzentraten formulierten erfindungsgemäßen Verbindungen wurden dann als wäßrige Suspensionen bzw. Emulsionen mit einer Wasseraufwandmenge von umgerechnet 600 bis 800 l/ha in unterschiedlichen Dosierungen auf die Oberfläche der Abdeckerde appliziert.

Nach der Behandlung wurden die Töpfe im Gewächshaus aufgestellt und unter guten Wachstumsbedingungen für die Unkräuter gehalten. Die optische Bonitur der Pflanzen- bzw. Auflaufschäden erfolgte nach dem Auflaufen der Versuchspflanzen nach einer Versuchszeit von 3 bis 4 Wochen im Vergleich zu unbehandelten Kontrollen. Wie die Ergebnisse zeigen, weisen erfindungsgemäße Verbindungen eine gute herbizide Vorauflaufwirksamkeit gegen ein breites Spektrum von Ungräsern und Unkräutern auf. Beispielsweise haben die Beispiele Nr. 1-9, 1-11, 1-15, 1-16, 1-17, 1-23, 1-25, 1-26, 1-41, 1-45, 1-47, 1-55, 1-57, 1-105, 1-129, 1-131, 1-135, 1-153, 1-154, 1-155, 1-159, 1-177, 1-179, 1-189, 1-241, 1-243, 1-247, 1-265, 1-267, 1-271, 1-353, 1-355, 1-359, 1-381, 1-383, 1-387, 1-401, 1-417, 1-425, 1-426, 1-433, 1-435, 1-439, 1-453, 1-455, 1-459 und andere Verbindungen aus Tabellen 1 und 2 sehr gute herbizide Wirkung gegen Schadpflanzen wie Sinapis alba, Chrysanthemum segetum, Avena sativa, Stellaria media, Echinochloa crus-galli, Lolium multiflorum, Setaria spp., Abutilon theophrasti, Amaranthus retroflexus und Panicum miliaceum im Vorauflaufverfahren bei einer Aufwandmenge von 0.3 kg und weniger Aktivsubstanz pro Hektar.

### 2. Unkrautwirkung im Nachauflauf

Samen bzw. Rhizomstücke von mono- und dikotylen Unkräutern wurden in Plastiktöpfen in sandigem Lehmboden ausgelegt, mit Erde abgedeckt und im Gewächshaus unter guten Wachstumsbedingungen angezogen. Drei Wochen nach der Aussaat wurden die Versuchspflanzen im Dreiblattstadium behandelt. Die als Spritzpulver bzw. als Emulsionskonzentrate formulierten erfindungsgemäßen Verbindungen wurden in verschiedenen Dosierungen mit einer Wasseraufwandmenge von umgerechnet 600 bis 800 l/ha auf die grünen Pflanzenteile gesprüht. Nach ca. 3 bis 4 Wochen Standzeit der Versuchspflanzen im Gewächshaus unter optimalen Wachstumsbedingungen wurde die Wirkung der Präparate optisch im Vergleich zu unbehandelten Kontrollen bonitiert. Die erfindungsgemäßen Mittel weisen auch im Nachauflauf eine gute herbizide Wirksamkeit gegen ein breites Spektrum wirtschaftlich wichtiger Ungräser und Unkräuter auf. Beispielsweise haben die Beispiele Nr. 1-9, 1-11, 1-15, 1-16, 1-17, 1-23, 1-25, 1-26, 1-41, 1-45, 1-47, 1-55, 1-57, 1-105, 1-129, 1-131, 1-135, 1-153, 1-154, 1-155, 1-159, 1-177, 1-179, 1-189, 1-241, 1-243, 1-247, 1-265, 1-267, 1-271, 1-353, 1-355, 1-359, 1-381, 1-383, 1-387, 1-401, 1-417, 1-425, 1-426, 1-433, 1-435, 1-439, 1-453, 1-455, 1-459 und andere Verbindungen aus Tabellen 1 und 2 sehr gute herbizide Wirkung gegen Schadpflanzen wie Sinapis alba, Echinochloa crus-galli, Lolium multiflorum, Chrysanthemum segetum, Setaria spp., Abutilon theophrasti, Amaranthus retroflexus, Panicum miliaceum und Avena sativa im Nachauflaufverfahren bei einer Aufwandmenge von 0.3 kg und weniger Aktivsubstanz pro Hektar.

### 3. Kulturpflanzenverträglichkeit

In weiteren Versuchen im Gewächshaus wurden Samen einer größeren Anzahl von Kulturpflanzen und Unkräutern in sandigem Lehmboden ausgelegt und mit Erde abgedeckt. Ein Teil der Töpfe wurde sofort wie unter Abschnitt 1 beschrieben behandelt, die übrigen im Gewächshaus aufgestellt, bis die Pflanzen zwei bis drei echte Blätter entwickelt haben und dann wie unter Abschnitt 2 beschrieben mit den erfindungsgemäßen Substanzen der Formel (I) in unterschiedlichen Dosierungen besprüht. Vier bis fünf Wochen nach der Applikation und Standzeit im Gewächshaus wurde mittels optischer Bonitur festgestellt, daß erfindungsgemäße Verbindungen zweikeimblättrige Kulturen wie Soja, Baumwolle, Raps, Zuckerrüben oder Kartoffeln im Vor- und Nachauflaufverfahren selbst bei hohen Wirkstoffdosierungen ungeschädigt ließen. Einige Substanzen schonten darüber hinaus auch Gramineen-Kulturen wie Gerste, Weizen, Roggen, Hirsen, Mais oder Reis. Die Verbindungen der Formel (I) zeigen teilweise eine hohe Selektivität und eignen sich deshalb zur Bekämpfung von unerwünschten Pflanzenwuchs in landwirtschaftlichen Kulturen.

## Patentansprüche

1. Verbindung der Formel (I) oder deren Salze, 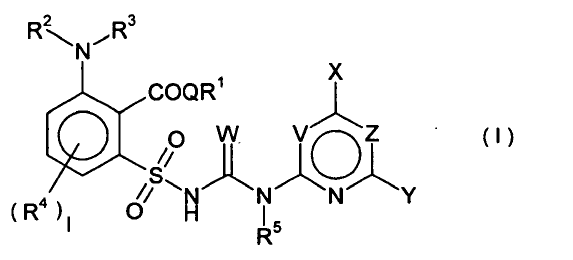 worin
R¹ H, (C₁-C₆)Alkyl, (C₃-C₆)Alkenyl, (C₃-C₆)Alkinyl, wobei jeder der drei letztgenannten Reste unsubstituiert oder durch einen oder mehrere Reste aus der Gruppe Halogen, unsubstituiertes und substituiertes Phenyl, unsubstituiertes und substituiertes Heterocyclyl mit 3 bis 6 Ringatomen, unsubstituiertes und substituiertes (C₃-C₆)Cycloalkyl, (C₁-C₄)Alkoxy, (C₁-C₄)Alkylthio, [(C₁-C₄)Alkoxy]carbonyl und [(C₁-C₄)Haloalkoxy]carbonyl, substituiert ist, oder unsubstituiertes oder substituiertes (C₃-C₆)Cycloalkyl, substituiertes oder unsubstituiertes (C₃-C₆)Cycloalkenyl,unsubstituiertes oder substituiertes Phenyl, unsubstituiertes oder substituiertes Heterocyclyl mit 3 bis 6 Ringatomen,
wobei substituiertes Phenyl, substituiertes Heterocyclyl, substituiertes Cycloalkyl oder substituiertes Cycloalkenyl als Substituenten einen oder mehrere Reste aus der Gruppe Halogen, (C₁-C₄)Alkyl, (C₁-C₄)Haloalkyl, (C₁-C₄)Alkoxy (C₁-C₄)alkyl, Di[(C₁-C₄)alkoxy](C₁-C₄)alkyl, (C₁-C₄)Haloalkoxy, (C₁-C₄) Alkylthio, (C₁-C₄)Alkylsulfinyl, (C₁-C₄)Haloalkylsulfinyl, (C₁-C₄)Alkylsulfonyl, (C₁-C₄)Haloalkylsulfonyl, NR⁸R⁹, [(C₁-C₄)Alkoxy]carbonyl, [(C₁-C₄)Haloalkoxy]carbonyl, [(C₁-C₄)Alkyl]carbonyl, OH, Phenyl, CN und NO₂ trägt und
R² eine Gruppe der Formel R⁰-Q⁰-,
worin R⁰ ein Wasserstoffatom, (C₁-C₁₂)Alkyl, (C₂-C₁₂)Alkenyl oder (C₂-C₁₂)Alkinyl, wobei jeder der letztgenannten drei Reste unsubstituiert oder durch einen oder mehrere Reste aus der Gruppe Halogen, (C₁-C₆)Alkoxy, (C₁-C₆)Haloalkoxy, (C₁-C₆)Alkylthio, (C₁-C₆)Haloalkylthio, (C₁-C₆)Alkylsulfinyl, (C₁-C₆)Haloalkylsutfinyl, (C₁-C₆)Alkylsulfonyl, (C₁-C₆)Haloalkylsulfonyl, [(C₁-C₆)Alkoxy]carbonyl, [(C₁-C₆)Haloalkoxy]carbonyl, CONR⁶R⁷, SO₂NR⁶R⁷, CN, OH, SH, (C₃-C₆)Cycloalkyl, NR⁸R⁹, unsubstituiertes oder substituiertes Phenyl, unsubstituiertes oder substituiertes Heterocyclyl substituiert ist, oder
(C₃-C₆)Cycloalkyl, (C₃-C₆)Cycloalkenyl, Phenyl oder Heterocyclyl, vorzugsweise mit 3 bis 6 Ringatomen, wobei die vier zuletzt genannten Reste unsubstituiert oder substituiert sein können, bedeutet und
worin Q⁰ eine direkte Bindung oder eine divalente Gruppe der Formel -O- oder -N(R^{#})- bedeutet, wobei R^{#} ein Wasserstoffatom, ein Acylrest oder (C₁-C₁₂)Alkyl, (C₂-C₁₂)Alkenyl oder (C₂-C₁₂)Alkinyl, wobei jeder der letztgenannten 3 Reste unsubstituiert oder durch einen oder mehrere Reste aus der Gruppe Halogen, (C₁-C₆)Alkoxy, (C₁-C₆)Haloalkoxy, (C₁-C₆) Alkylthio, (C₁-C₆) Haloalkylthio, CN, OH, (C₃-C₆)Cycloalkyl,
unsubstituiertes oder substituiertes Phenyl, unsubstituiertes oder substituiertes Heterocyclyl substituiert ist, oder unsubstituiertes oder substituiertes (C₃-C₆)Cycloalkyl, unsubstituiertes oder substituiertes (C₃-C₆)Cycloalkenyl, oder
unsubstituiertes oder substituiertes Phenyl bedeutet, und R⁰ und R^{#} gemeinsam mit dem N-Atom der NR#R°-Gruppe einen Heterocyclylrest, vorzugsweise mit 3 bis 6 Ringatomen bilden können, der unsubstituiert oder substituiert ist, vorzugsweise durch einen oder mehrere Reste aus der Gruppe Halogen, (C₁-C₆)Alkyl, (C₁-C₆)Alkoxy, [(C₁-C₆)Alkoxy]carbonyl, (C₁-C₆)Haloalkyl und Oxo,
R³ ein Wasserstoffatom, (C₁-C₁₂)Alkyl, (C₂-C₁₂)Alkenyl oder (C₂-C₁₂)Alkinyl, wobei jeder der letztgenannten 3 Reste unsubstituiert oder durch einen oder mehrere Reste aus der Gruppe Halogen, (C₁-C₈)Alkoxy, (C₁-C₆)Haloalkoxy, (C₁-C₆)-Alkylthio, (C₁-C₈)-Haloalkylthio, (C₁-C₆)Alkylsulfinyl, (C₁-C₆)Haloalkylsulfinyl, (C₁-C₆)Alkylsulfonyl, (C₁-C₆)Haloalkylsulfonyl, [(C₁-C₆)Alkoxy]carbonyl, [(C₁-C₆)Haloalkoxy]carbonyl, CONR⁶R⁷, SO₂NR⁶R⁷, CN, OH, (C₃-C₆)Cycloalkyl, NR⁸R⁹, unsubstituiertes oder substituiertes Phenyl, unsubstituiertes oder substituiertes Heterocyclyl substituiert ist, oder unsubstituiertes oder substituiertes (C₃-C₆)Cycloalkyl, unsubstituiertes oder substituiertes (C₃-C₆)Cycloalkenyl, unsubstituiertes oder substituiertes Heterocyclyl, vorzugsweise mit 3 bis 6 Ringatomen, oder unsubstituiertes oder substituiertes Phenyl bedeutet, und
R² und R³ gemeinsam mit dem N-Atom der NR²R³-Gruppe (N¹) einen Heterocyclylrest, vorzugsweise mit 3 bis 6 Ringatomen bilden können, der unsubstituiert oder substituiert ist, vorzugsweise durch einen oder mehrere Reste aus der Gruppe Halogen, (C₁-C₆)Alkyl, (C₁-C₆)Alkoxy, [(C₁-C₆)Alkoxy]carbonyl, (C₁-C₆)Haloalkyl und Oxo, wobei sich der Oxo-Rest vorzugsweise nicht in Nachbarposition zum N-Atom (N¹) befindet, und
R⁴ unabhängig voneinander Halogen, CN, (C₁-C₄)Alkyl, (C₁-C₄)Alkoxy, (C₂-C₆)Alkenyl, (C₂-C₆)Alkinyl, (C₃-C₆)Alkenyloxy, (C₃-C₆)Alkinyloxy,
wobei jeder der letztgenannten 6 Reste unsubstituiert oder durch einen oder mehrere Reste aus der Gruppe Halogen, (C₁-C₆)Alkoxy, (C₁-C₆)Haloalkoxy, (C₁-C₆)-Alkylthio, (C₁-C₆)-Haloalkylthio, (C₁-C₆)Alkylsulfinyl, (C₁-C₆)Haloalkylsulfinyl, (C₁-C₆)Alkylsulfonyl, (C₁-C₆)Haloalkylsulfonyl, [(C₁-C₆)Alkoxy]carbonyl, [(C₁-C₆)Haloalkoxy]carbonyl, CONR⁶R⁷ SO₂NR⁶R⁷, CN, OH, (C₃-C₆)Cycloalkyl, NR⁸R⁹, unsubstituiertes oder substituiertes Phenyl oder unsubstituiertes oder substituiertes Heterocyclyl, substituiert ist, oder
unsubstituiertes oder substituiertes (C₃-C₆)Cycloalkyl, unsubstituiertes oder substituiertes (C₃-C₆)Cycloalkenyl, unsubstituiertes oder substituiertes Heterocyclyl, vorzugsweise mit 3 bis 6 Ringatomen, unsubstituiertes oder substituiertes Phenyl, [(C₁-C₄)Alkyl]carbonyl oder [(C₁-C₄)Alkoxy]carbonyl bedeuten, wobei jeder der letztgenannten beiden Reste unsubstituiert oder im Alkylteil durch ein oder mehrere Halogenatome substituiert ist, oder ein Rest der Formel C(O)-NR'-R", C(S)-NR'-R", CR'=N-Q¹-R", S(O)ₘ-Q¹-R"", P(O)ₙ(-Q¹-R""Q²-R"), NR'-Q¹-R" oder NR"'-N=CR'-R" bedeuten, wobei R', R" und R'" unabhängig voneinander ein Wasserstoffatom, einen Acylrest oder einen unsubstituierten oder substituierten (C₁-C₁₀)Kohlenstoffwasserstoffrest, R"" einen kohlenstoffhaltigen Acylrest oder einen unsubstituierten oder substituierten (C₁-C₁₀)Kohlenstoffwasserstoffrest, und Q¹ und Q² unabhängig voneinander eine direkte Bindung oder eine divalente Gruppe der Formel -O- oder -N(R⁺)- bedeuten, wobei R⁺ ein Wasserstoffatom, ein Acylrest oder ein unsubstituierter oder substituierter (C₁-C₁₀)Kohlenwasserstoffrest bedeutet, und m = 0, 1, 2 oder 3, sowie n = 0, 1 oder 2 sein kann, und R' mit R", R⁺ mit R', R⁺ mit R" oder R⁺ mit R"" jeweils einen Heterocyclylrest, vorzugsweise mit 3 bis 6 Ringatomen bilden kann,
der unsubstituiert oder substituiert ist, vorzugsweise durch einen oder mehrere Reste aus der Gruppe Halogen, (C₁-C₆)Alkyl, (C₁-C₆)Alkoxy, [(C₁-C₆)Alkoxy]carbonyl, (C₁-C₆)Haloalkyl und Oxo,
I gleich 0, 1 oder 2, vorzugsweise 0 oder 1 ist,
R⁵ H oder C₁-C₄(Alkyl) ist, das unsubstituiert oder substituiert ist,
R⁶ und R⁷ unabhängig voneinander H, (C₁-C₆)Alkyl, (C₃-C₆)Alkenyl, (C₃-C₆)Alkinyl, unsubstituiertes oder substituiertes Phenyl oder unsubstituiertes oder substituiertes Heterocyclyl bedeuten oder R⁶ und R⁷ gemeinsam mit dem N-Atom der NR⁶R⁷-Gruppe einen Heterocyclylrest mit 5 oder 6 Ringatomen bilden können, der gegebenenfalls eines oder mehrere zusätzliche Heteroatome aus der Gruppe N, O und S enthalten kann und unsubstituiert oder ein- oder mehrfach durch Reste aus der Gruppe (C₁-C₄)Alkyl und Oxo substituiert ist, und
R⁸ und R⁹ unabhängig voneinander (C₁-C₄)Alkylcarbonyl, (C₁-C₄)Haloalkylcarbonyl, (C₁-C₄)Alkoxycarbonyl oder (C₁-C₄)Alkylsulfonyl bedeuten oder gemeinsam mit dem N-Atom der NR⁸R⁹-Gruppe einen Heterocyclylrest mit 5 oder 6 Ringatomen bilden können, der gegebenenfalls eines oder mehrere zusätzliche Heteroatome aus der Gruppe N, O und S enthalten kann und unsubstituiert oder ein- oder mehrfach durch Reste aus der Gruppe (C₁-C₄)Alkyl und Oxo substituiert ist, und
Q O, S oder NR* ist,
R* (C₁-C₄)Alkyl, (C₃-C₄)Alkenyl oder (C₃-C₄)Alkinyl bedeutet, wobei jeder der drei letztgenannten Reste unsubstituiert oder durch einen oder mehrere Reste aus der Gruppe Halogen, (C₁-C₄)Alkoxy und (C₁-C₄)Alkylthio substituiert ist, und R* und R¹ gemeinsam mit dem N-Atom der NR*R¹-Gruppe einen Heterocyclylrest bilden können, der unsubstituiert oder substituiert ist, vorzugsweise durch oder mehrere Reste aus der Gruppe Halogen, (C₁-C₆)Alkyl, (C₁-C₆)Alkoxy, [(C₁-C₆)Alkoxy]carbonyl, (C₁-C₆)Haloalkyl und Oxo,
X, Y unabhängig voneinander H, Halogen, (C₁-C₄)Alkyl, (C₁-C₄)Alkoxy, (C₁-C₄)Alkylthio, wobei jeder der drei letztgenannten Reste unsubstituiert oder durch einen oder mehrere Reste aus der Gruppe Halogen, (C₁-C₄)Alkoxy und (C₁-C₄)Alkylthio substituiert ist, Mono- oder Di[(C₁-C₄)alkyl]amino, (C₃-C₅)Alkenyl, (C₃-C₅)Alkenyloxy, (C₃-C₅)Alkinyl oder (C₃-C₅)Alkinyloxy bedeuten, und
wobei die Reste R¹, R², R³ und R⁴ inklusive Substituenten bis zu 20 C-Atomen aufweisen,
W ein sauerstoff-oder Schwefelatom
V, Z unabhängig voneinander CH oder N bedeuten.

2. Verbindung der Formel (I) oder deren Salze nach Anspruch 1 , worin
R¹ H, (C₁-C₆)Alkyl, (C₃-C₆)Alkenyl oder (C₃-C₆)Alkinyl, wobei jeder der drei letztgenannten Reste unsubstituiert oder durch einen oder mehrere Reste aus der Gruppe Halogen, Phenyl, (C₁-C₄)Alkoxy, (C₁-C₄)Alkylthio (C₃-C₆)Cycloalkyl, Heterocyclyl mit 3 bis 6 Ringatomen, und [(C₁-C₄)Alkoxy]-carbonyl substituiert ist, oder (C₃-C₆)Cycloalkyl oder Heterocyclyl mit 3 bis 6 Ringatomen bedeutet, wobei jeder der letztgenannten beiden Reste unsubstituiert oder durch einen oder mehrere Reste aus der Gruppe Halogen, (C₁-C₄)Alkyl und (C₁-C₄)Alkoxy substituiert ist,
R² eine Gruppe der Formel R⁰-Q⁰-, worin
R⁰ ein Wasserstoffatom, (C₁-C₈)Alkyl, (C₃-C₈)Alkenyl oder (C₃-C₈)Alkinyl, wobei jeder der letztgenannten 3 Reste unsubstituiert oder durch einen oder mehrere Reste aus der Gruppe Halogen (C₁-C₄)Alkoxy, (C₁-C₄)Haloalkoxy, (C₁-C₄)Alkylthio, (C₁-C₄)Haloalkylthio, (C₁-C₄)Alkylsulfinyl, (C₁-C₄)Haloalkylsulfinyl, (C₁-C₄)Alkylsulfonyl, (C₁-C₄)Haloalkylsulfonyl, [(C₁-C₆)Alkoxy]carbonyl, CONR⁶R⁷, SO₂NR⁶R⁷, CN, OH, (C₃-C₆)Cycloalkyl, NR⁸R⁹ und Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylsulfinyl, (C₁-C₄)Alkylsulfonyl, NR⁸R⁹, [(C₁-C₄)Alkoxy]carbonyl, [(C₁-C₄)Alkyl]carbonyl, Phenyl, [(C₁-C₄)Alkyl]-carbonyl, CN und NO₂ substituiert ist, substituiert ist, oder Heterocyclyl mit 3 bis 6 Ringatomen, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylsulfinyl, (C₁-C₄)Alkylsulfonyl, NR⁸R⁹, [(C₁-C₄)Alkoxy]carbonyl, [(C₁-C₄)Alkyl]carbonyl, Phenyl, CN und NO₂ substituiert ist, oder (C₃-C₆)Cycloalkyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)-Haloalkoxy, [(C₁-C₄)Alkoxy]carbonyl, CN, OH und Phenyl substituiert ist, oder
(C₃-C₆)Cycloalkenyl, das unsubstituiert oder durch einen oder mehrere Reste aus der Gruppe Halogen, (C₁-C₄)Alkyl, (C₁-C₄)Haloalkyl, (C₁-C₄)Alkoxy und [(C₁-C₄)Alkoxy]carbonyl substituiert ist, oder
Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylsulfinyl, (C₁-C₄)Alkylsulfonyl, NR⁸R⁹, [(C₁-C₄)Alkoxy]carbonyl, [(C₁-C₄)Alkyl]carbonyl, Phenyl, CN und NO₂ substituiert ist, und
Q⁰ eine direkte Bindung oder eine divalente Gruppe der Formel -O- oder -NR^{#}, worin R^{#} ein Wasserstoffatom oder unsubstituiertes oder substituiertes (C₁-C₄)Alkyl ist, bedeutet,
R³ ein Wasserstoffatom, (C₁-C₈)Alkyl, (C₃-C₈)Alkenyl oder (C₃-C₈)Alkinyl, wobei jeder der letztgenannten 3 Reste unsubstituiert oder durch einen oder mehrere Reste aus der Gruppe Halogen (C₁-C₄)Alkoxy, (C₁-C₄)Haloalkoxy, (C₁-C₄)Alkylthio, (C₁-C₄)Haloalkylthio, (C₁-C₄)Alkylsulfinyl, (C₁-C₄)Haloalkylsulfinyl, (C₁-C₄)Alkylsulfonyl, (C₁-C₄)Haloalkylsulfonyl, [(C₁-C₆)Alkoxy]carbonyl, CONR⁶R⁷, SO₂NR⁶R⁷, CN, OH, (C₃-C₆)Cycloalkyl, NR⁸R⁹ und Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylsulfinyl, (C₁-C₄)Alkylsulfonyl, NR⁸R⁹, [(C₁-C₄)Alkoxy]carbonyl, [(C₁-C₄)Alkyl]carbonyl, Phenyl, [(C₁-C₄)Alkyl]carbonyl, CN und NO₂ substituiert ist, substituiert ist, oder Heterocyclyl, vorzugsweise mit 3 bis 6 Ringatomen, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylsulfinyl, (C₁-C₄)Alkylsulfonyl, NR⁸R⁹, [(C₁-C₄)Alkoxy]carbonyl, [(C₁-C₄)Alkyl]carbonyl, Phenyl, CN und NO₂ substituiert ist, substituiert ist, oder (C₃-C₆)Cycloalkyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, [(C₁-C₄)Alkoxy]carbonyl, CN, OH und Phenyl substituiert ist, oder
(C₃-C₆)Cycloalkenyl, das unsubstituiert oder durch einen oder mehrere Reste aus der Gruppe Halogen, (C₁-C₄)Alkyl, (C₁-C₄)Haloalkyl, (C₁-C₄)Alkoxy und [(C₁-C₄)Alkoxy]carbonyl substituiert ist, oder
Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Alkylsulfinyl, (C₁-C₄)Alkylsufonyl, NR⁸R⁹, [(C₁-C₄)Alkoxy]carbonyl, [(C₁-C₄)Alkyl]carbonyl, Phenyl, CN und NO₂ substituiert ist, und
R² und R³ gemeinsam mit dem N-Atom (N¹) einen Heterocyclylrest mit 3-6 Ringatomen bilden können, der gesättigt, ungesättigt oder heteroaromatisch ist und zusätzlich zum N-Atom (N¹) ein oder zwei Heteroatome aus der Gruppe N, O und S enthalten kann und unsubstituiert oder durch einen oder mehrere Reste aus der Gruppe (C₁-C₆)Alkyl, (C₁-C₃)Alkoxy, Halogen, [(C₁-C₃)Alkoxy]carbonyl, (C₁-C₃)Haloalkyl und Oxo, wobei sich der Oxo-Rest vorzugsweise nicht in Nachbarposition zum N-Atom (N¹) befindet, substituiert ist,
R⁴ Halogen, CN, (C₁-C₄)Alkyl, (C₁-C₄)Alkoxy, (C₂-C₆)Alkenyl, (C₂-C₆)-Alkinyl, (C₃-C₆)Alkenyloxy, (C₃-C₆)Alkinyloxy,
wobei jeder der letztgenannten 6 Reste unsubstituiert oder durch einen oder mehrere Reste aus der Gruppe Halogen, (C₁-C₆)Alkoxy, (C₁-Cₐ)Haloalkoxy, (C₁-C₆)Alkylthio, (C₁-C₆)Haloalkylthio, (C₁-C₆)Alkylsulfinyl, (C₁-C₆)Haloalkylsulfinyl, (C₁-C₆)Alkylsulfonyl, (C₁-C₆)Haloalkylsulfonyl, [(C₁-C₆)Alkoxy]carbonyl, [(C₁-C₆)Haloalkoxy]carbonyl, CONR⁶R⁷, SO₂NR⁶R⁷, CN, OH, (C₃-C₆)Cycloalkyl, NR⁸R⁹, unsubstituiertes oder substituiertes Phenyl, unsubstituiertes oder substituiertes Heterocyclyl substituiert ist, oder
unsubstituiertes oder substituiertes (C₃-C₆)Cycloalkyl, unsubstituiertes oder substituiertes (C₃-C₆)Cycloalkenyl, unsubstituiertes oder substituiertes Heterocyclyl mit 3 bis 6 Ringatomen, unsubstituiertes oder substituiertes Phenyl oder [(C₁-C₄)Alkyl]carbonyl oder [(C₁-C₄)Alkoxy]carbonyl bedeuten, wobei jeder der letztgenannten beiden Reste unsubstituiert oder im Alkylteil durch ein oder mehrere Halogenatome substituiert ist, oder ein Rest der Formel C(O)-NR'-R", C(S)-NR'-R", CR'=N-Q¹-R", NR'-Q¹-R" oder NR"'-N=CR'-R" bedeuten, wobei R', R" und R"' unabhängig voneinander ein Wasserstoffatom, einen Acylrest oder einen unsubstituierten oder substituierten (C₁-C₁₀)Kohlenstoffwasserstoffrest, und Q¹ und Q² unabhängig voneinander eine direkte Bindung oder eine divalente Gruppe der Formel -O- oder -N(R⁺)- bedeuten, wobei R⁺ ein Wasserstoffatom, ein Acylrest oder ein unsubstituierter oder substituierter (C₁-C₄)Alkylrest bedeutet, und R' mit R" oder R⁺ mit R' oder R⁺ mit R" jeweils einen Heterocyclylrest mit 3 bis 6 Ringatomen bilden kann, der unsubstituiert oder substituiert ist, vorzugsweise durch einen oder mehrere Reste aus der Gruppe Halogen, (C₁-C₆)Alkyl, (C₁-C₆)Alkoxy, [(C₁-C₆)Alkoxy]carbonyl, (C₁-C₆)Haloalkyl und Oxo,
I gleich 0 oder 1 ist,
R⁶ und R⁷ unabhängig voneinander H, (C₁-C₄)Alkyl, (C₃-C₄)Alkenyl, (C₃-C₄)Alkinyl oder Phenyl, das unsubstituiert oder durch einen oder mehrere Reste aus der Gruppe Halogen, (C₁-C₄)Alkyl, (C₁-C₄)Haloalkyl, (C₁-C₄)Alkoxy, (C₁-C₄)Alkylthio, (C₁-C₄)Alkylsulfonyl, [(C₁-C₄)Alkoxy]carbonyl, CN und NO₂ substituiert ist, oder
R⁶ und R⁷ gemeinsam mit dem N-Atom der NR⁶R⁷-Gruppe einen Heterocyclylrest mit 5 oder 6 Ringgliedern bilden können, der gegebenenfalls eines oder mehrere Heteroatome aus der Gruppe N, O und S enthalten kann und unsubstituiert oder ein- oder mehrfach durch Reste aus der Gruppe (C₁-C₄)Alkyl und Oxo substituiert ist,
R⁸und R⁹ (C₁-C₄)Alkylcarbonyl, (C₁-C₄)Haloalkylcarbonyl, (C₁-C₄)Alkoxycarbonyl oder (C₁-C₄)Alkylsulfonyl sind, oder gemeinsam mit dem N-Atom der NR⁸R⁹-Gruppe einen Heterocyclylrest mit 5 oder 6 Ringatomen bilden können, der gegebenenfalls eines oder mehrere Heteroatome aus der Gruppe N, O und S enthalten kann und unsubstituiert oder ein- oder mehrfach durch Reste aus der Gruppe (C₁-C₄)Alkyl und Oxo substituiert ist.

3. Verbindung der Formel (I) oder deren Salze nach einem der Ansprüche 1 oder 2, worin
R¹ (C₁-C₆)Alkyl, das unsubstituiert oder durch einen oder mehrere Reste aus der Gruppe Halogen und (C₁-C₄)Alkoxy substituiert ist, oder 3-Oxetanyl, (C₃-C₄)Alkenyl oder (C₃-C₄)Alkinyl,
R² H, (C₁-C₆)Alkyl, (C₃-C₆)Alkenyl, (C₃-C₆)Alkinyl, wobei jeder der letztgenannten drei Reste unsubstituiert oder durch einen oder mehrere Reste aus der Gruppe Halogen, (C₁-C₄)Alkoxy, (C₁-C₄)Alkylthio, (C₁-C₄)Alkylsulfonyl, [(C₁-C₄)Alkoxy]carbonyl, (C₃-C₆)Cycloalkyl, CN und OH substituiert ist, oder (C₃-C₆)Cycloalkyl, das unsubstituiert oder durch einen oder mehrere Reste aus der Gruppe Halogen, (C₁-C₄)Alkyl, (C₁-C₄)Alkoxy, [(C₁-C₄)Alkoxy]carbonyl, CN und OH substituiert ist, oder (C₃-C₆)Cycloalkenyl, (C₁-C₄)Alkoxy, (C₁-C₄)Alkenyloxy, (C₁-C₄)Alkylamino oder Di[(C₁-C₄)alkyl]amino und
R³ H, (C₁-C₆)Alkyl, (C₃-C₆)Alkenyl, (C₃-C₆)Alkinyl, wobei jeder der letztgenannten drei Reste unsubstituiert oder durch einen oder mehrere Reste aus der Gruppe Halogen, (C₁-C₄)Alkoxy, (C₁-C₄)Alkylthio, (C₁-C₄)Alkylsulfonyl, [(C₁-C₄)Alkoxy]carbonyl, (C₃-C₆)Cycloalkyl, CN und OH substituiert ist, oder (C₃-C₆)Cycloalkyl, das unsubstituiert oder durch einen oder mehrere Reste aus der Gruppe Halogen, (C₁-C₄)Alkyl, (C₁-C₄)Alkoxy, [(C₁-C₄)Alkoxy]carbonyl, CN und OH substituiert ist, oder (C₃-C₆)Cycloalkenyl oder
R² und R³ gemeinsam mit dem N-Atom (N¹) einen Heterocyclylrest mit 3-6 Ringatomen bilden können, der gesättigt, ungesättigt oder heteroaromatisch ist, zusätzlich zu dem N-Atom (N¹) ein oder zwei Heteroatome aus der Gruppe N, O und S enthalten kann und unsubstituiert oder durch einen oder mehrere Reste aus der Gruppe Halogen, (C₁-C₆)Alkyl, (C₁-C₃)Alkoxy, [(C₁-C₃)Alkoxy]carbonyl und Oxo, wobei sich der Oxo-Rest vorzugsweise nicht in Nachbarposition zum N-Atom (N¹) befindet, substituiert ist, und
R⁴ (C₁-C₄)Alkyl, (C₁-C₄)Haloalkyl, (C₁-C₄)Alkoxy oder Halogen,
I gleich 0 oder 1 ist, vorzugsweise 0 ist,
R⁵ H oder Methyl,
Q O oder NR*,
R* H oder (C₁-C₄)Alkyl,
X und Y unabhängig voneinander (C₁-C₄)Alkyl, (C₁-C₄)Alkoxy, wobei jeder der letztgenannten zwei Reste unsubstituiert oder durch einen oder mehrere Halogenatome substituiert ist, oder (C₁-C₄)Alkylthio, Halogen oder Mono- oder Di[(C₁-C₂)alkyl]amino bedeuten, und
W ein Sauerstoffatom bedeutet.

4. Verbindung der Formel (I) oder deren Salze nach einem der Ansprüche 1 bis 3, worin
R¹ (C₁-C₃)Alkyl, Allyl oder Propargyl,
R² H, (C₁-C₄)Alkyl, (C₃-C₅)Alkenyl, (C₃-C₅)Alkinyl, (C₃-C₆)Cycloalkyl oder (C₃-C₆)Cycloalkenyl,
R³ H, (C₁-C₄)Alkyl, (C₃-C₅)Alkenyl, (C₃-C₅)Alkinyl, (C₃-C₆)Cycloalkyl oder (C₃-C₆)Cycloalkenyl, oder
R² und R³ gemeinsam mit dem N-Atom (N¹) einen Heterocyclylrest mit 3-6 Ringatomen bilden können, der gesättigt, ungesättigt oder heteroaromatisch ist, zusätzlich zu dem N-Atom (N¹) ein oder zwei Heteroatome aus der Gruppe N, O und S enthalten kann und unsubstituiert oder durch einen oder mehrere (C₁-C₆)Alkylreste substituiert ist,
R⁴ (C₁-C₃)Alkyl oder Halogen,
I gleich 0 oder 1, vorzugsweise 0 ist,
Q O oder NR*,
R* (C₁-C₃)Alkyl,
X (C₁-C₂)Alkyl, (C₁-C₂)Alkoxy, (C₁-C₂)Alkylthio, (C₁-C₂)Haloalkyl oder (C₁-C₂)Haloalkoxy,
Y (C₁-C₂)Alkyl, (C₁-C₂)Alkoxy, Halogen, NHCH₃ oder N(CH₃)₂
V CH oder N, vorzugsweise N und
Z CH oder N bedeuten.

5. Verfahren zur Herstellung von Verbindungen der Formel (I) oder deren Salzen, wie in einem oder mehreren der Ansprüche 1 bis 4 definiert, worin
a) eine Verbindung der Formel (II) 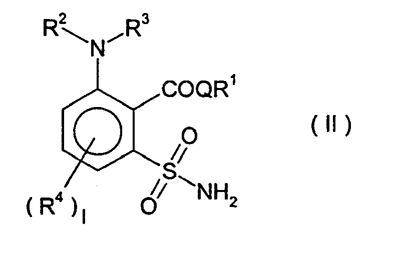 mit einem heterocyclischen Carbamat der Formel (III), 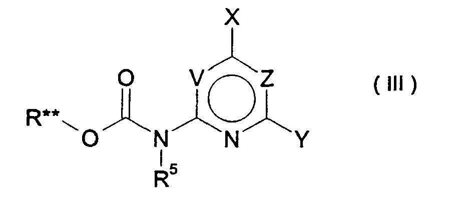 worin R** einen substituierten oder unsubstituierten C₁-C₂₀-Kohlenwasserstoffrest bedeutet, umgesetzt wird oder
b) ein Sulfonylcarbamat der Formel (IV), 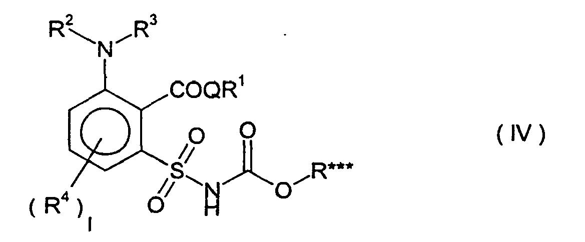 worin R*** einen substituierten oder unsubstituierten C₁-C₂₀-Kohlenwasserstoffrest bedeutet, mit einem Aminoheterocyclus der Formel (V) 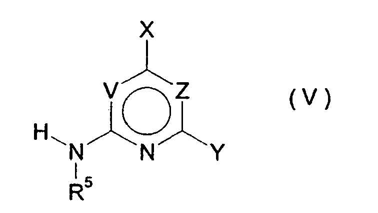 umgesetzt wird oder
c) ein Sulfonylisocyanat der Formel (VI) 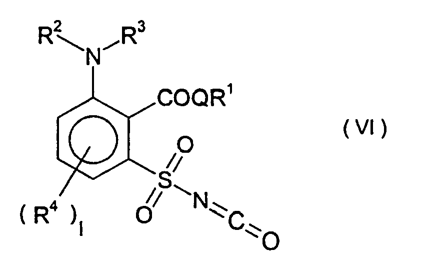 mit einem Aminoheterocyclus der Formel (V) umgesetzt wird oder
d) ein Sulfonamid der Formel (II) mit einem (Thio)-Isocyanat der Formel (VII) 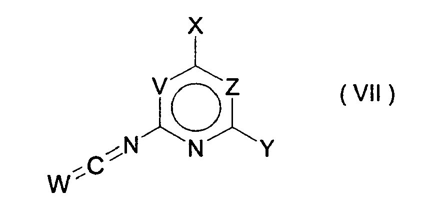 in Gegenwart einer Base umgesetzt wird oder
e) ein Aminoheterocyclus der Formel (V) zunächst basenkatalysiert mit einem Kohlensäureester, umgesetzt wird und das gebildete Intermediat in einer Eintopfreaktion mit einem Sulfonamid der Formel (11) (siehe Variante a) umgesetzt wird, oder
f) einen Phenylsulfonylharnstoff der Formel (VIII) 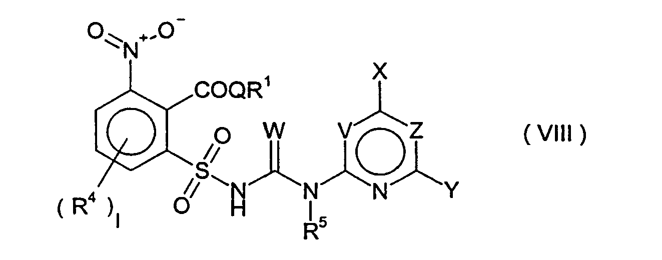 durch Reduktion der Nitrogruppe, und gegebenenfalls weiterer Umsetzung der freigesetzten Hydroxylamin- oder Aminfunktion, zu einem Sulfonylharnstoff der Formel (I) umgesetzt wird,
wobei in den Formeln (II)-(VIII) die Reste, Gruppen und Indizes R¹-R⁵, Q, V, W, X, Y, Z und I wie in Formel (I) nach Anspruch 1 definiert sind.

6. Herbizides oder pflanzenwachstumsregulierendes Mittel, enthaltend a) mindestens eine Verbindung der Formel (I) oder deren Salz nach einem der Ansprüche 1 bis 4 und b) im Pflanzenschutz übliche Formulierungshilfsmittel.

7. Verfahren zur Bekämpfung von Schadpflanzen oder zur Wachstumsregulierung von Pflanzen, wobei eine wirksame Menge von mindestens einer Verbindung der Formel (I) oder deren Salz nach einem der Ansprüche 1 bis 4 auf die Schadpflanzen bzw. Pflanzen, deren Pflanzensamen oder die Fläche, auf der sie wachsen, appliziert wird.

8. Verwendung der Verbindungen der Formel (I) oder deren Salze nach einem der Ansprüche 1 bis 4 als Herbizide oder Pflanzenwachstumsregulatoren.

9. Verwendung nach Anspruch 8, wobei die Verbindungen der Formel (I) oder deren Salze zur Bekämpfung von Schadpflanzen oder zur Wachstumsregulierung in Kulturen von Nutz- oder Zierpflanzen eingesetzt werden.

10. Verwendung nach Anspruch 9, wobei die Kulturpflanzen transgene Kulturpflanzen sind.

11. Verbindung der Formel (II*) 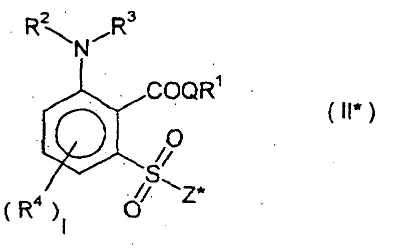 worin Z*= NH₂, NHCOOR***, NCO oder NH-tert.-Butyl bedeutet und R¹-R⁴, I und Q wie in Formel (1) nach Anspruch 1 und R*** wie in Formel (IV) nach Anspruch 5 definiert sind und wobei folgende Verbindungen a) b) und c) ausgenommen sind:
a) 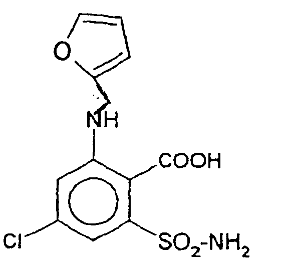
b) 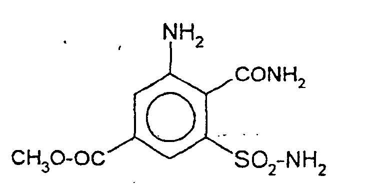
c)

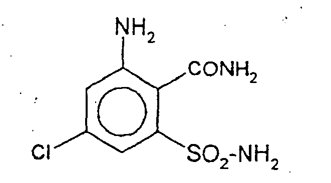

12. Verwendung einer Verbindung der Formel (II*), wie in Anspruch 11 definiert zur Herstellung einer Verbindung der Formel (I) gemäß Anspruch 1.

13. Verbindung der Formel (X*) 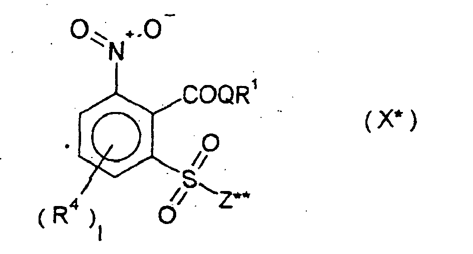 worin Z**= NH₂, NHCOOR***, NH-tert.-Butyl oder Cl bedeutet und R¹, R⁴, I und Q wie in Formel (I) nach Anspruch 1 und R*** wie in Formel (IV) nach Anspruch 5 definiert sind, und wobei Verbindungen ausgenommen sind, in denen Z** = Cl, I = 0, Q = O und R¹ = H, Methyl, Ethyl oder Allyl ist sowie Verbindungen in denen Z** = NH₂, I = 0, Q = O und R¹= H ist.

14. Verwendung einer Verbindung der Formel (X*) wie in Anspruch 13 definiert, worin Z** gleich NH₂, NHCOOR*** oder NH-tert.-Butyl ist und R*** wie in Formel (IV) nach Anspruch 5 definiert ist, zur Herstellung einer Verbindung der Formel (II*) wie in Anspruch 11 definiert, worin Z* gleich NH₂, NHCOOR*** oder NH-tert.-Butyl ist.

15. Verbindungen der Formel (VIII) 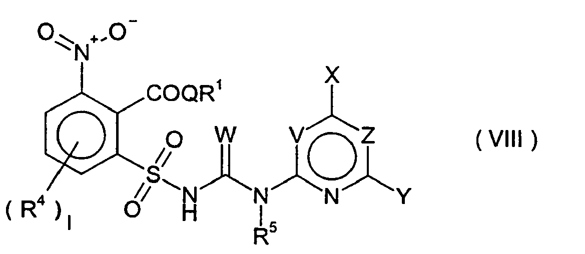 worin R¹, R⁴, R⁵, Q, V, W, X, Y, Z und 1 wie in Formel (1) nach Anspruch 1 definiert sind.

16. Verwendung einer Verbindung der Formel (VIII) wie in Anspruch 15 definiert, zur Herstellung einer Verbindung der Formel (1) wie in Anspruch 1 definiert.

## Claims

1. A compound of the formula (I) or a salt thereof 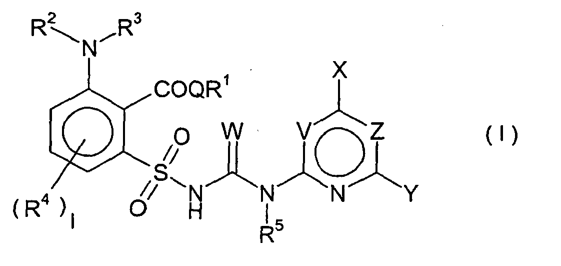 in which
R¹ is H, (C₁-C₆)alkyl, (C₃-C₆)alkenyl, (C₃-C₆)alkynyl, where each of the three last-mentioned radicals is unsubstituted or substituted by one or more radicals selected from the group consisting of halogen, unsubstituted and substituted phenyl, unsubstituted and substituted heterocyclyl having 3 to 6 ring atoms, unsubstituted and substituted (C₃-C₆)cycloalkyl, (C₁-C₄)alkoxy, (C₁-C₄)alkylthio, [(C₁-C₄)alkoxy]carbonyl and [(C₁-C₄)haloalkoxy]carbonyl, or is unsubstituted or substituted (C₃-C₆)cycloalkyl, substituted or unsubstituted (C₃-C₆)cycloalkenyl, unsubstituted or substituted phenyl, unsubstituted or substituted heterocyclyl having 3 to 6 ring atoms,
where substituted phenyl, substituted heterocyclyl, substituted cycloalkyl or substituted cycloalkenyl carry, as substituents, one or more radicals selected from the group consisting of halogen, (C₁-C₄)alkyl , (C₁-C₄)haloalkyl, (C₁-C₄)alkoxy, (C₁-C₄)alkyl, di[(C₁-C₄)alkoxy](C₁-C₄)alkyl, (C₁-C₄)haloalkoxy, (C₁-C₄)alkylthio, (C₁-C₄)alkylsulfinyl, (C₁-C₄)haloalkylsulfinyl, (C₁-C₄)alkylsulfonyl, (C₁-C₄)haloalkylsulfonyl, NR⁸R⁹, [(C₁-C₄)alkoxy]carbonyl, [(C₁-C₄)haloalkoxy]carbonyl, [(C₁-C₄)alkyl]carbonyl, OH, phenyl, CN and NO₂ and
R² is a group of the formula R⁰-Q⁰-,
in which R⁰ is a hydrogen atom, (C₁-C₁₂)alkyl, (C₂-C₁₂)alkenyl or (C₂-C₁₂)alkynyl, where each of the three last-mentioned radicals is unsubstituted or substituted by one or more radicals selected from the group consisting of halogen, (C₁-C₆)alkoxy, (C₁-C₆)haloalkoxy, (C₁-C₆)alkylthio, (C₁-C₆)haloalkylthio, (C₁-C₆)alkylsulfinyl, (C₁-C₆)haloalkylsulfinyl, (C₁-C₆)alkylsulfonyl, (C₁-C₆)haloalkylsulfonyl, [(C₁-C₆)alkoxy]carbonyl, [(C₁-C₆)haloalkoxy]carbonyl, CONR⁶R⁷, SO₂NR⁶R⁷, CN, OH, SH, (C₃-C₆)cycloalkyl, NR⁸R⁹, unsubstituted or substituted phenyl, unsubstituted or substituted heterocyclyl, or is (C₃-C₆)cycloalkyl, (C₃-C₆)cycloalkenyl, phenyl or heterocyclyl, preferably having 3 to 6 ring atoms, where the four last-mentioned radicals may be unsubstituted or substituted, and
in which Q⁰ is a direct bond or a divalent group of the formula -O- or -N(R^{#})- where R^{#} is a hydrogen atom, an acyl radical or (C₁-C₁₂)alkyl, (C₂-C₁₂)alkenyl or (C₂-C₁₂)alkynyl, where each of the 3 last-mentioned radicals is unsubstituted or substituted by one or more radicals selected from the group consisting of halogen, (C₁-C₆)alkoxy, (C₁-C₆)haloalkoxy, (C₁-C₆)alkylthio, (C₁-C₆)haloalkylthio, CN, OH, (C₃-C₆)cycloalkyl, unsubstituted or substituted phenyl, unsubstituted or substituted heterocyclyl, or is unsubstituted or substituted (C₃-C₆)cycloalkyl, unsubstituted or substituted (C₃-C₆)cycloalkenyl or
unsubstituted or substituted phenyl, and R⁰ and R^{#} together with the nitrogen atom of the NR^{#}R⁰ group may form a heterocyclyl radical, preferably having 3 to 6 ring atoms, which is unsubstituted or substituted, preferably by one or more radicals selected from the group consisting of halogen, (C₁-C₆)alkyl, (C₁-C₆)alkoxy, [(C₁-C₆)alkoxy]carbonyl, (C₁-C₆)haloalkyl and oxo,
R³ is a hydrogen atom, (C₁-C₁₂)alkyl, (C₂-C₁₂)alkenyl or (C₂-C₁₂)alkynyl, where each of the 3 last-mentioned radicals is unsubstituted or substituted by one or more radicals selected from the group consisting of halogen, (C₁-C₆)alkoxy, (C₁-C₆)haloalkoxy, (C₁-C₆)alkylthio, (C₁-C₆)haloalkylthio, (C₁-C₆)alkylsulfinyl, (C₁-C₆)haloalkylsulfinyl, (C₁-C₆)alkylsulfonyl, (C₁-C₆)haloalkylsulfonyl, [(C₁-C₆)alkoxy]carbonyl, [(C₁-C₆)haloalkoxy]carbonyl, CONR⁶R⁷, SO₂NR⁶R⁷, CN, OH, (C₃-C₆)cycloalkyl, NR⁸R⁹, unsubstituted or substituted phenyl, unsubstituted or substituted heterocyclyl, or is unsubstituted or substituted (C₃-C₆)cycloalkyl, unsubstituted or substituted (C₃-C₆)cycloalkenyl, unsubstituted or substituted heterocyclyl, preferably having 3 to 6 ring atoms, or unsubstituted or substituted phenyl, and
R² and R³ together with the nitrogen atom of the NR²R³ group (N¹) may form a heterocyclyl radical, preferably having 3 to 6 ring atoms, which is unsubstituted or substituted, preferably by one or more radicals selected from the group consisting of halogen, (C₁-C₆)alkyl, (C₁-C₆)alkoxy, [(C₁-C₆)alkoxy]carbonyl, (C₁-C₆)haloalkyl and oxo, where the oxo radical is preferably not adjacent to the nitrogen atom (N¹), and
R⁴ independently of one another are halogen, CN, (C₁-C₄)alkyl, (C₁-C₄)alkoxy, (C₂-C₆)alkenyl, (C₂-C₆)alkynyl, (C₃-C₆)alkenyloxy, (C₃-C₆)alkynyloxy, where each of the 6 last-mentioned radicals is unsubstituted or substituted by one or more radicals selected from the group consisting of halogen, (C₁-C₆)alkoxy, (C₁-C₆)haloalkoxy, (C₁-C₆)alkylthio, (C₁-C₆)haloalkylthio, (C₁-C₆)alkylsulfinyl, (C₁-C₆)haloalkylsulfinyl, (C₁-C₆)alkylsulfonyl, (C₁-C₆)haloalkylsulfonyl, [(C₁-C₆)alkoxy]carbonyl, [(C₁-C₆)haloalkoxy]carbonyl, CONR⁶R⁷, SO₂NR⁶R⁷, CN, OH, (C₃-C₆)cycloalkyl, NR⁸R⁹, unsubstituted or substituted phenyl or unsubstituted or substituted heterocyclyl, or
is unsubstituted or substituted (C₃-C₆)cycloalkyl, unsubstituted or substituted (C₃-C₆)cycloalkenyl, unsubstituted or substituted heterocyclyl, preferably having 3 to 6 ring atoms, unsubstituted or substituted phenyl, [(C₁-C₄)alkyl]carbonyl or [(C₁-C₄)alkoxy]carbonyl, where each of the two last-mentioned radicals is unsubstituted or substituted in the alkyl moiety by one or more halogen atoms, or is a radical of the formula C(O)-NR'-R", C(S)-NR'-R", CR'=N-Q¹-R", S(O)ₘ-Q¹-R"", P(O)ₙ(-Q¹-R""Q²-R"), NR'-Q¹-R" or NR"'-N=CR'-R", where R', R" and R'" independently of one another are a hydrogen atom, an acyl radical or an unsubstituted or substituted (C₁-C₁₀)hydrocarbon radical, R"" is a carbon-containing acyl radical or an unsubstituted or substituted (C₁-C_{1O})hydrocarbon radical, and Q¹ and Q² independently of one another are a direct bond or a divalent group of the formula -O- or -N(R⁺)-, where R⁺ is a hydrogen atom, an acyl radical, or an unsubstituted or substituted (C₁-C₁₀)hydrocarbon radical, and m = 0, 1, 2 or 3, and n = 0, 1 or 2, and R' together with R", R⁺ together with R', R⁺ together with R" or R⁺ together with R"" may in each case form a heterocyclyl radical, preferably having 3 to 6 ring atoms,
which is unsubstituted or substituted, preferably by one or more radicals selected from the group consisting of halogen, (C₁-C₆)alkyl, (C₁-C₆)alkoxy, [(C₁-C₆)alkoxy]carbonyl, (C₁-C₆)haloalkyl and oxo,
I is 0, 1 or 2, preferably 0 or 1,
R⁵ is H or (C₁-C₄)alkyl which is unsubstituted or substituted,
R⁶ and R⁷ independently of one another are H, (C₁-C₆)alkyl, (C₃-C₆)alkenyl, (C₃-C₆)alkynyl, unsubstituted or substituted phenyl or unsubstituted or substituted heterocyclyl or
R⁶ and R⁷ together with the nitrogen atom of the NR⁶R⁷ group may form a heterocyclyl radical having 5 or 6 ring atoms which may optionally contain one or more additional heteroatoms selected from the group consisting of N, O and S and which is unsubstituted or mono- or polysubstituted by radicals selected from the group consisting of (C₁-C₄)alkyl and oxo, and
R⁸ and R⁹ independently of one another are (C₁-C₄)alkylcarbonyl, (C₁-C₄)haloalkylcarbonyl, (C₁-C₄)alkoxycarbonyl or (C₁-C₄)alkylsulfonyl or together with the nitrogen atom of the NR⁸R⁹ group may form a heterocyclyl radical having 5 or 6 ring atoms which may optionally contain one or more additional- heteroatoms selected from the group consisting of N, O and S and which is unsubstituted or mono- or polysubstituted by radicals selected from the group consisting of (C₁-C₄)alkyl and oxo, and
Q is O, S or NR*,
R* is (C₁-C₄)alkyl, (C₃-C₄)alkenyl or (C₃-C₄)alkynyl, where each of the three last-mentioned radicals is unsubstituted or substituted by one or more radicals selected from the group consisting of halogen, (C₁-C₄)alkoxy and (C₁-C₄)alkylthio, and
R* and R¹ together with the nitrogen atom of the NR*R¹ group may form a heterocyclyl radical which is unsubstituted or substituted, preferably by one or more radicals selected from the group consisting of halogen, (C₁-C₆)alkyl, (C₁-C₆)alkoxy, [(C₁-C₆)alkoxy]carbonyl, (C₁-C₆)haloalkyl and oxo,
X, Y independently of one another are H, halogen, (C₁-C₄)alkyl, (C₁-C₄)alkoxy, (C₁-C₄)alkylthio, where each of the three last-mentioned radicals is unsubstituted or substituted by one or more radicals from the group consisting of halogen, (C₁-C₄)alkoxy and (C₁-C₄)alkylthio, are mono- or di[(C₁-C₄)alkyl]amino, (C₃-C₅)alkenyl, (C₃-C₅)alkenyloxy, (C₃-C₅)alkynyl or (C₃-C₅)alkynyloxy, and
where the radicals R¹, R², R³ and R⁴, including substituents, have up to 20 carbon atoms,
W is an oxygen or sulfur atom,
V, Z independently of one another are CH or N.

2. A compound of the formula (I) or a salt thereof as claimed in claim 1, in which
R¹ is H, (C₁-C₆)alkyl, (C₃-C₆)alkenyl or (C₃-C₆)alkynyl, where each of the three last-mentioned radicals is unsubstituted or substituted by one or more radicals selected from the group consisting of halogen, phenyl, (C₁-C₄)alkoxy, (C₁-C₄)alkylthio, (C₃-C₆)cycloalkyl, heterocyclyl having 3 to 6 ring atoms and [(C₁-C₄)alkoxy]carbonyl, or is (C₃-C₆)cycloalkyl or heterocyclyl having 3 to 6 ring atoms, where each of the two last-mentioned radicals is unsubstituted or substituted by one or more radicals selected from the group consisting of halogen, (C₁-C₄)alkyl and (C₁-C₄)alkoxy,
R² is a group of the formula R⁰-Q⁰- in which
R⁰ is a hydrogen atom, (C₁-C₈)alkyl, (C₃-C₈)alkenyl or (C₃-C₈)alkynyl, where each of the 3 last-mentioned radicals is unsubstituted or substituted by one or more radicals selected from the group consisting of halogen, (C₁-C₄)alkoxy, (C₁-C₄)haloalkoxy, (C₁-C₄)alkylthio, (C₁-C₄)haloalkylthio, (C₁-C₄)alkylsulfinyl, (C₁-C₄)haloalkylsulfinyl, (C₁-C₄)alkylsulfonyl, (C₁-C₄)haloalkylsulfonyl, [(C₁-C₆)alkoxy]carbonyl, CONR⁶R⁷, SO₂NR⁶R⁷, CN, OH, (C₃-C₆)cycloalkyl, NR⁸R⁹ and phenyl which is unsubstituted or substituted by one or more radicals selected from the group consisting of halogen, (C₁-C₄)alkyl, (C₁-C₄)haloalkyl, (C₁-C₄)alkoxy, (C₁-C₄)haloalkoxy, (C₁-C₄)alkylthio, (C₁-C₄)alkylsulfinyl, (C₁-C₄)alkylsulfonyl, NR⁸R⁹, [(C₁-C₄)alkoxy]carbonyl, [(C₁-C₄)alkyl]carbonyl, phenyl, [(C₁-C₄)alkyl]carbonyl, CN and NO₂, or
is heterocyclyl having 3 to 6 ring atoms which is unsubstituted or substituted by one or more radicals selected from the group consisting of halogen, (C₁-C₄)alkyl, (C₁-C₄)haloalkyl, (C₁-C₄)alkoxy, (C₁-C₄)haloalkoxy, (C₁-C₄)alkylthio, (C₁-C₄)alkylsulfinyl, (C₁-C₄)alkylsulfonyl, NR⁸R⁹, [(C₁-C₄)alkoxy]carbonyl, [(C₁-C₄)alkyl]carbonyl, phenyl, CN and NO₂, or
is (C₃-C₆)cycloalkyl which is unsubstituted or substituted by one or more radicals selected from the group consisting of halogen, (C₁-C₄)alkyl, (C₁-C₄)haloalkyl, (C₁-C₄)alkoxy, (C₁-C₄)haloalkoxy, [(C₁-C₄)alkoxy]carbonyl, CN, OH and phenyl, or
is (C₃-C₆)cycloalkenyl which is unsubstituted or substituted by one or more radicals selected from the group consisting of halogen, (C₁-C₄)alkyl, (C₁-C₄)haloalkyl, (C₁-C₄)alkoxy and [(C₁-C₄)alkoxy]carbonyl, or
is phenyl which is unsubstituted or substituted by one or more radicals selected from the group consisting of halogen, (C₁-C₄)alkyl, (C₁-C₄)haloalkyl, (C₁-C₄)alkoxy, (C₁-C₄)haloalkoxy, (C₁-C₄)alkylthio, (C₁-C₄)alkylsulfinyl, (C₁-C₄)alkylsulfonyl, NR⁸R⁹, [(C₁-C₄)alkoxy]carbonyl, [(C₁-C₄)alkyl]carbonyl, phenyl, CN and NO₂, and
Q⁰ is a direct bond or a divalent group of the formula -O- or -NR^{#}, in which R^{#} is a hydrogen atom or unsubstituted or substituted (C₁-C₄)alkyl,
R³ is a hydrogen atom, (C₁-C₈)alkyl, (C₃-C₈)alkenyl or (C₃-C₈)alkynyl, where each of the 3 last-mentioned radicals is unsubstituted or substituted by one or more radicals selected from the group consisting of halogen, (C₁-C₄)alkoxy, (C₁-C₄)haloalkoxy, (C₁-C₄)alkylthio, (C₁-C₄)haloalkylthio, (C₁-C₄)alkylsulfinyl, (C₁-C₄)haloalkylsulfinyl, (C₁-C₄)alkylsulfonyl, (C₁-C₄)haloalkylsulfonyl, [(C₁-C₆)alkoxy]carbonyl, CONR⁶R⁷, SO₂NR⁶R⁷, CN, OH, (C₃-C₆)cycloalkyl, NR⁸R⁹ and phenyl which is unsubstituted or substituted by one or more radicals selected from the group consisting of halogen, (C₁-C₄)alkyl, (C₁-C₄)haloalkyl, (C₁-C₄)alkoxy, (C₁-C₄)haloalkoxy, (C₁-C₄)alkylthio, (C₁-C₄)alkylsulfinyl, (C₁-C₄)alkylsufonyl, NR⁸R⁹, [(C₁-C₄)alkoxy]carbonyl, [(C₁-C₄)alkyl]carbonyl, phenyl, [(C₁-C₄)alkyl]carbonyl, CN and NO₂, or
is heterocyclyl preferably having 3 to 6 ring atoms which is unsubstituted or substituted by one or more radicals selected from the group consisting of halogen, (C₁-C₄)alkyl, (C₁-C₄)haloalkyl, (C₁-C₄)alkoxy, (C₁-C₄)haloalkoxy, (C₁-C₄)alkylthio, (C₁-C₄)alkylsulfinyl, (C₁-C₄)alkylsulfonyl, NR⁸R⁹, [(C₁-C₄)alkoxy]carbonyl, [(C₁-C₄)alkyl]carbonyl, phenyl, CN and NO₂, or
is (C₃-C₆)cycloalkyl which is unsubstituted or substituted by one or more radicals selected from the group consisting of halogen, (C₁-C₄)alkyl, (C₁-C₄)haloalkyl, (C₁-C₄)alkoxy, (C₁-C₄)haloalkoxy, [(C₁-C₄)alkoxy]carbonyl, CN, OH and phenyl, or
is (C₃-C₆)cycloalkenyl which is unsubstituted or substituted by one or more radicals selected from the group consisting of halogen, (C₁-C₄)alkyl, (C₁-C₄)haloalkyl, (C₁-C₄)alkoxy and [(C₁-C₄)alkoxy]carbonyl, or
is phenyl which is unsubstituted or substituted by one or more radicals selected from the group consisting of halogen, (C₁-C₄)alkyl, (C₁-C₄)haloalkyl, (C₁-C₄)alkoxy, (C₁-C₄)haloalkoxy, (C₁-C₄)alkylthio, (C₁-C₄)alkylsulfinyl, (C₁-C₄)alkylsulfonyl, NR⁸R⁹, [(C₁-C₄)alkoxy]carbonyl, [(C₁-C₄)alkyl]carbonyl, phenyl, CN and NO₂, and
R² and R³ together with the nitrogen atom (N¹) may form a heterocyclyl radical having 3-6 ring atoms which is saturated, unsaturated or heteroaromatic and may, in addition to the nitrogen atom (N¹), contain one or two heteroatoms selected from the group consisting of N, O and S and which is unsubstituted or substituted by one or more radicals selected from the group consisting of (C₁-C₆)alkyl, (C₁-C₃)alkoxy, halogen, [(C₁-C₃)alkoxy]carbonyl, (C₁-C₃)haloalkyl and oxo, where the oxo radical is preferably not adjacent to the nitrogen atom (N¹),
R⁴ are halogen, CN, (C₁-C₄)alkyl, (C₁-C₄)alkoxy, (C₂-C₆)alkenyl, (C₂-C₆)alkynyl, (C₃-C₆)alkenyloxy, (C₃-C₆)alkynyloxy,
where each of the 6 last-mentioned radicals is unsubstituted or substituted by one or more radicals selected from the group consisting of halogen, (C₁-C₆)alkoxy, (C₁-C₆)haloalkoxy, (C₁-C₆)alkylthio, (C₁-C₆)haloalkylthio, (C₁-C₆)alkylsulfinyl, (C₁-C₆)haloalkylsulfinyl, (C₁-C₆)alkylsulfonyl, (C₁-C₆)-haloalkylsulfonyl, [(C₁-C₆)alkoxy]carbonyl, [(C₁-C₆)haloalkoxy]-carbonyl, CONR⁶R⁷, SO₂NR⁶R⁷, CN, OH, (C₃-C₆)cycloalkyl, NR⁸R⁹, unsubstituted or substituted phenyl, unsubstituted or substituted heterocyclyl, or
are unsubstituted or substituted (C₃-C₆)cycloalkyl, unsubstituted or substituted (C₃-C₆)cycloalkenyl, unsubstituted or substituted heterocyclyl having 3 to 6 ring atoms, unsubstituted or substituted phenyl or [(C₁-C₄)alkyl]carbonyl or [(C₁-C₄)alkoxy]carbonyl, where each of the two last-mentioned radicals is unsubstituted or substituted in the alkyl moiety by one or more halogen atoms, or are radicals of the formula C(O)-NR'-R", C(S)-NR'-R", CR'=N-Q¹-R", NR'-Q¹-R" or NR"'-N=CR'-R" where R', R" and R'" independently of one another are a hydrogen atom, an acyl radical or an unsubstituted or substituted (C₁-C₁₀)hydrocarbon radical, and Q¹ and Q² independently of one another are a direct bond or a divalent group of the formula -O- or -N(R⁺)-, where R⁺ is a hydrogen atom, an acyl radical or an unsubstituted or substituted (C₁-C₄)alkyl radical and R' together with R" or R⁺ together with R' or R⁺ together with R" may in each case form a heterocyclyl radical having 3 to 6 ring atoms which is unsubstituted or substituted, preferably by one or more radicals selected from the group consisting of halogen, (C₁-C₆)alkyl, (C₁-C₆)alkoxy, [(C₁-C₆)alkoxy]carbonyl, (C₁-C₆)haloalkyl and oxo, is 0 or 1,
R⁶ and R⁷ independently of one another are H, (C₁-C₄)alkyl, (C₃-C₄)alkenyl, (C₃-C₄)alkynyl or phenyl which is unsubstituted or substituted by one or more radicals selected from the group consisting of halogen, (C₁-C₄)alkyl, (C₁-C₄)haloalkyl, (C₁-C₄)alkoxy, (C₁-C₄)alkylthio, (C₁-C₄)alkylsulfonyl, [(C₁-C₄)alkoxy]carbonyl, CN and NO₂, or
R⁶ and R⁷ together with the nitrogen atom of the NR⁶R⁷ group may form a heterocyclyl radical having 5 or 6 ring members which may contain one or more heteroatoms selected from the group consisting of N, O and S and which is unsubstituted or mono- or polysubstituted by radicals selected from the group consisting of (C₁-C₄)alkyl and oxo,
R⁸ and R⁹ are (C₁-C₄)alkylcarbonyl, (C₁-C₄)haloalkylcarbonyl, (C₁-C₄)alkoxycarbonyl or (C₁-C₄)alkylsulfonyl, or together with the nitrogen atom of the NR⁸R⁹ group may form a heterocyclyl radical having 5 or 6 ring atoms which may contain one or more heteroatoms selected from the group consisting of N, O and S and which is unsubstituted or mono- or polysubstituted by radicals selected from the group consisting of (C₁-C₄)alkyl and oxo.

3. A compound of the formula (I) or a salt thereof as claimed in either of claims 1 and 2 in which
R¹ is (C₁-C₆)alkyl which is unsubstituted or substituted by one or more radicals selected from the group consisting of halogen and (C₁-C₄)alkoxy, or is 3-oxetanyl, (C₃-C₄)alkenyl or (C₃-C₄)alkynyl,
R² is H, (C₁-C₆)alkyl, (C₃-C₆)alkenyl, (C₃-C₆)alkynyl, where each of the three last-mentioned radicals is unsubstituted or substituted by one or more radicals selected from the group consisting of halogen, (C₁-C₄)alkoxy, (C₁-C₄)alkylthio, (C₁-C₄)alkylsulfonyl, [(C₁-C₄)alkoxy]carbonyl, (C₃-C₆)cycloalkyl, CN and OH, or is (C₃-C₆)cycloalkyl which is unsubstituted or substituted by one or more radicals selected from the group consisting of halogen, (C₁-C₄)alkyl, (C₁-C₄)alkoxy, [(C₁-C₄)alkoxy]carbonyl, CN and OH, or is (C₃-C₆)cycloalkenyl, (C₁-C₄)alkoxy, (C₁-C₄)alkenyloxy, (C₁-C₄)alkylamino or di[(C₁-C₄)alkyl]amino and
R³ is H, (C₁-C₆)alkyl, (C₃-C₆)alkenyl, (C₃-C₆)alkynyl, where each of the three last-mentioned radicals is unsubstituted or substituted by one or more radicals selected from the group consisting of halogen, (C₁-C₄)alkoxy, (C₁₋C₄)alkylthio, (C₁-C₄)alkylsulfonyl, [(C₁-C₄)alkoxy]carbonyl, (C₃-C₆)cycloalkyl, CN and OH, or is (C₃-C₆)cycloalkyl which is unsubstituted or substituted by one or more radicals selected from the group consisting of halogen, (C₁-C₄)alkyl, (C₁-C₄)alkoxy, [(C₁-C₄)alkoxy]carbonyl, CN and OH, or is (C₃-C₆)cycloalkenyl or
R² and R³ together with the nitrogen atom (N¹) may form a heterocyclyl radical having 3-6 ring atoms which is saturated, unsaturated or heteroaromatic, which may, in addition to the nitrogen atom (N¹), contain one or two heteroatoms selected from the group consisting of N, O and S and which is unsubstituted or substituted by one or more radicals selected from the group consisting of halogen, (C₁-C₆)alkyl, (C₁-C₃)alkoxy, [(C₁-C₃)alkoxy]carbonyl and oxo, where the oxo radical is preferably not adjacent to the nitrogen atom (N¹), and
R⁴ are (C₁-C₄)alkyl, (C₁-C₄)haloalkyl, (C₁-C₄)alkoxy or halogen,
I is 0 or 1, preferably 0,
R⁵ is H or methyl,
Q is O or NR*,
R* is H or (C₁-C₄)alkyl,
X and Y independently of one another are (C₁-C₄)alkyl, (C₁-C₄)alkoxy, where each of the two last-mentioned radicals is unsubstituted or substituted by one or more halogen atoms, or are (C₁-C₄)alkylthio, halogen or mono- oder di[(C₁-C₂)alkyl]amino, and
W is an oxygen atom.

4. A compound of the formula (I) or a salt thereof as claimed in any of claims 1 to 3 in which
R¹ is (C₁-C₃)alkyl, allyl or propargyl,
R² is H, (C₁-C₄)alkyl, (C₃-C₅)alkenyl, (C₃-C₅)alkynyl, (C₃-C₆)cycloalkyl or (C₃-C₆)cycloalkenyl,
R³ is H, (C₁-C₄)alkyl, (C₃-C₅)alkenyl, (C₃-C₅)alkynyl, (C₃-C₆)cycloalkyl or (C₃-C₆)cycloalkenyl, or
R² and R³ together with the nitrogen atom (N¹) may form a heterocyclyl radical having 3-6 ring atoms which is saturated, unsaturated or heteroaromatic and may, in addition to the nitrogen atom (N¹), contain one or two heteroatoms selected from the group consisting of N,O and S and which is unsubstituted or substituted by one or more (C¹-C⁶)alkyl radicals,
R⁴ are (C₁-C₃)alkyl or halogen,
I is 0 or 1, preferably 0,
Q is O or NR*,
R* is (C₁-C₃)alkyl,
X is (C₁-C₂)alkyl, (C₁-C₂)alkoxy, (C₁-C₂)alkylthio, (C₁-C₂)haloalkyl or (C₁-C₂)haloalkoxy,
Y is (C₁-C₂)alkyl, (C₁-C₂)alkoxy, halogen, NHCH₃ or N(CH₃)₂,
V is CH or N, preferably N and
Z is CH or N.

5. A process for preparing compounds of the formula (I) or salts thereof as defined in one or more of claims 1 to 4 which comprises
a) reacting a compound of the formula (II) 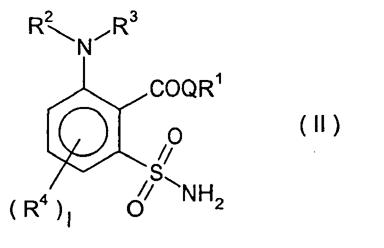 with a heterocyclic carbamate of the formula (III), 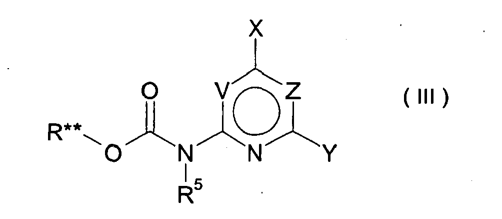 in which R** is a substituted or unsubstituted C₁-C₂₀-hydrocarbon radical, or
b) reacting a sulfonylcarbamate of the formula (IV), 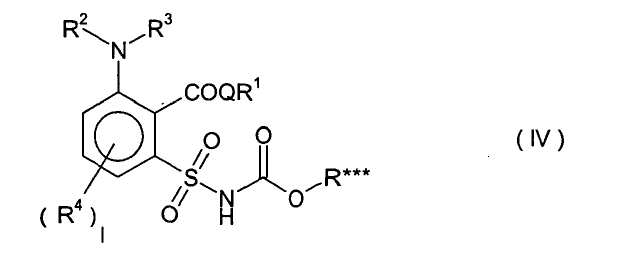 in which R*** is a substituted or unsubstituted C₁-C₂₀-hydrocarbon radical with an amino heterocycle of the formula (V) 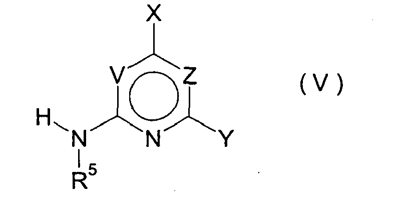 or
c) reacting a sulfonyl isocyanate of the formula (VI) 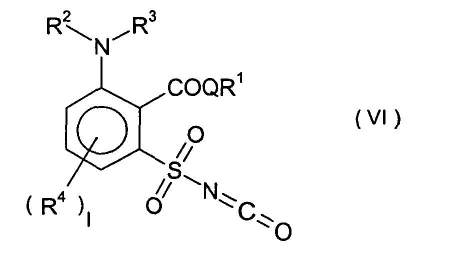 with an amino heterocycle of the formula (V) or
d) reacting a sulfonamide of the formula (II) with a (thio)isocyanate of the formula (VII) 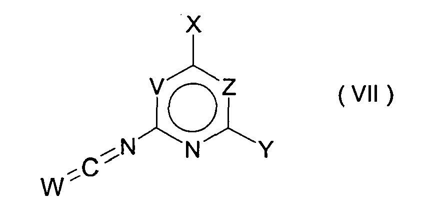 in the presence of a base or
e) reacting an amino heterocycle of the formula (V) initially under base catalysis with a carbonic ester and reacting the resulting intermediate in a one-pot reaction with a sulfonamide of the formula (II) (see variant a), or
f) reacting a phenylsulfonyl urea of the formula (VIII) 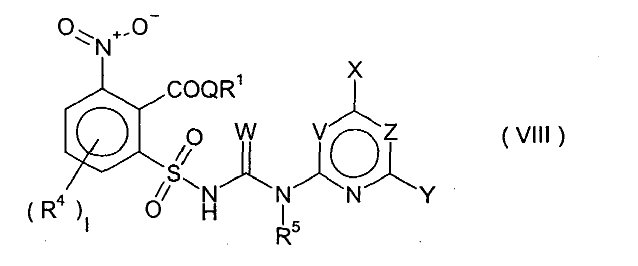 by reduction of the nitro group and, if appropriate, further conversion of the hydroxylamine or amine function that is released to give a sulfonyl urea of the formula (I),
where in the formulae (II)-(VIII) the radicals, groups and indices R¹-R⁵, Q, V, W, X, Y, Z and I are as defined in formula (I) of claim 1.

6. A herbicidal or plant-growth-regulating composition, comprising a) at least one compound of the formula (I) or a salt thereof as claimed in any of claims 1 to 4 and b) formulation auxiliaries which are customary in crop protection.

7. A method for controlling harmful plants or for regulating the growth of plants, which comprises applying an effective amount of at least one compound of the formula (I) or a salt thereof as claimed in any of claims 1 to 4 to the harmful plants or plants, to their plant seeds or to the area on which they grow.

8. The use of the compounds of the formula (I) or their salts as claimed in any of claims 1 to 4 as herbicides or plant growth regulators.

9. The use as claimed in claim 8, where the compounds of the formula (I) or their salts are employed for controlling harmful plants or for regulating the growth in crops of useful or ornamental plants.

10. The use as claimed in claim 9, where the crop plants are transgenic crop plants.

11. A compound of the formula (II*) 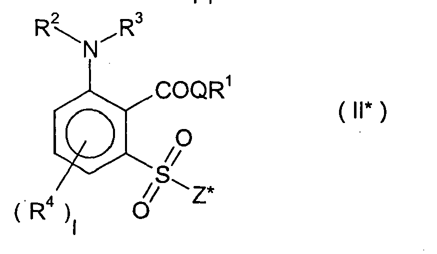 in which Z*= NH₂, NHCOOR***, NCO or NH-tert-butyl and R¹-R⁴, I and Q are as defined in formula (I) of claim 1 and R*** is as defined in formula (IV) of claim 5, and wherein the following compounds a), b) and c) are excepted:
a) 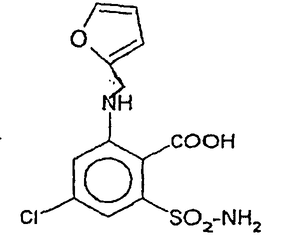
b). 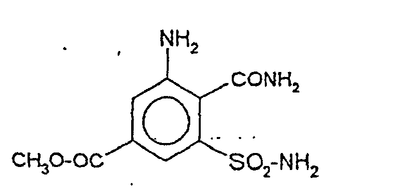
c)

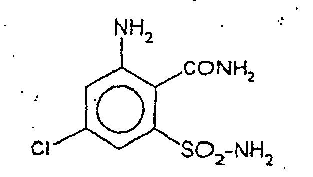

12. The use of a compound of the formula (II*) as defined in claim 11 for preparing a compound of the formula (I) as claimed in claim 1.

13. A compound of the formula (X*) 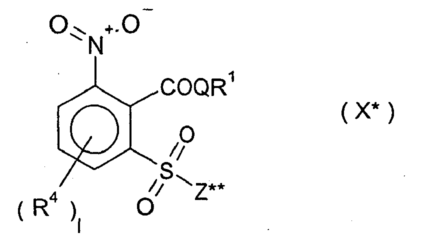 in which Z**= NH₂, NHCOOR***, NH-tert-butyl or Cl and R¹, R⁴, I and Q are as defined in formula (I) of claim 1 and R*** is as defined in formula (IV) of claim 5, except for compounds in which Z** = Cl, I = 0, Q = O and R¹ = H, methyl, ethyl or allyl, and compounds in which Z** = NH₂, I = 0, Q = O and R¹ = H.

14. The use of a compound of the formula (X*) as defined in claim 13 in which Z** is NH₂, NHCOOR*** or NH-tert-butyl and R*** is as defined in formula (IV) of claim 5 for preparing a compound of the formula (II*) as defined in claim 11 in which Z* is NH₂, NHCOOR*** or NH-tert-butyl.

15. A compound of the formula (VIII) 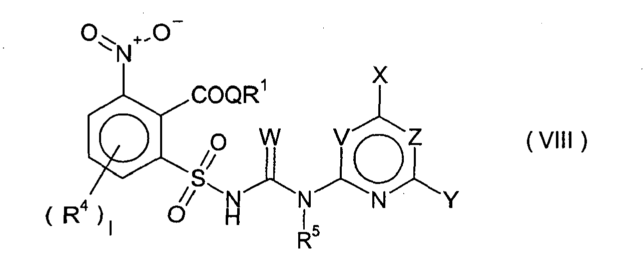 in which R¹, R⁴, R⁵, Q, V, W, X, Y, Z and I are as defined in formula (I) of claim 1.

16. The use of a compound of the formula (VIII) as defined in claim 15 for preparing a compound of the formula (I) as defined in claim 1.

## Revendications

1. Composé de formule I ou ses sels 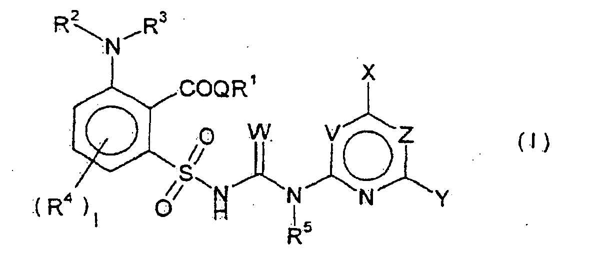 où
R¹ représente H, un radical alkyle en C₁-C₆, alcényle en C₃-C₆, alcynyle en C₃-C₆, chacun des trois derniers radicaux cités étant non substitué ou substitué par un ou plusieurs radicaux pris dans le groupe constitué par un atome d'halogène, un radical phényle non substitué ou substitué, hétérocyclyle non substitué ou substitué de 3 à 6 chaînons, cycloalkyle en C₃-C₆ non substitué ou substitué, alkoxy en C₁-C₄, (alkyl en C₁-C₄) thio, (alkoxy en C₁-C₄) carbonyle et halo (alkoxy en C₁-C₄)-carbonyle, ou cycloalkyle en C₃-C₆ non substitué ou substitué, cycloalcényle en C₃-C₆ non substitué ou substitué, phényle non substitué ou substitué, hétérocyclyle non substitué ou substitué ayant 3 à 6 chaînons,
phényle substitué, hétérocyclyle substitué, cycloalkyle substitué ou cycloalcényle substitué portent en tant que substituants un ou plusieurs radicaux pris dans le groupe constitué par un atome d'halogène, un radical alkyle en C₁-C₄, haloalkyle en C₁-C₄, (alkoxy en C₁-C₄)alkyle en C₁-C₄, di(alkoxy en C₁-C₄) alkyle en C₁-C₄, haloalkoxy en C₁-C₄, (alkyl en C₁-C₄)thio, (alkyl en C₁-C₄)sulfinyle, halo(alkyl en C₁-C₄)-sulfinyle, (alkyl en C₁-C₄) sulfonyle, halo(alkyl en C₁-C₄)sulfonyle, NR⁸R⁹, (alkoxy en C₁-C₄)-carbonyle, halo(alkoxy en C₁-C₄)-carbonyle, (alkyl en C₁-C₄) carbonyle, OH, phényle, CN et NO₂, et
R² représente un groupe de formule R⁰-Q⁰-,
où R° représente un atome d'hydrogène, un radical alkyle en C₁-C₁₂, alcényle en C₂-C₁₂ ou alcynyle en C₂-C₁₂, chacun des trois derniers radicaux cités étant non substitué ou substitué par un ou plusieurs radicaux pris dans le groupe constitué par un atome d'halogène, un radical alkoxy en C₁-C₆, haloalkoxy en C₁-C₆, (alkyl en C₁-C₆) thio, halo (alkyl en C₁-C₆) thio, (alkyl en C₁-C₆) -sulfinyle, halo(alkyl en C₁-C₆) sulfinyle, (alkyl en C₁-C₆)sulfonyle, halo (alkyl en C₁-C₆)-sulfonyle, (alkoxy en C₁-C₆)carbonyle, halo (alkoxy en C₁-C₆) carbonyle, CONR⁶R⁷, SO₂NR⁶R⁷, CN, OH, SH, cycloalkyle en C₃-C₆, NR⁸R⁹, phényle non substitué ou substitué, hétérocyclyle non substitué ou substitué, ou
cycloalkyle en C₃-C₆, cycloalcényle en C₃-C₆, phényle ou hétérocyclyle de préférence ayant 3 à 6 chaînons, les quatre derniers radicaux cités pouvant être non substitués ou substitués, et où Q⁰ représente une liaison directe ou un groupe divalent de formule -0- ou -N(R^{#}), R^{#} représente un atome d'hydrogène, un radical acyle ou alkyle en C₁-C₁₂, alcényle en C₂-C₁₂ ou alcynyle en C₂-C₁₂, chacun des 3 derniers radicaux cités étant non substitué ou substitué par un ou plusieurs radicaux pris dans le groupe constitué par un atome d'halogène, un radical alkoxy en C₁-C₆, haloalkoxy en C₁-C₆, (alkyl en C₁-C₆)thio, halo(alkyl en C₁-C₆)thio, CN, OH, cycloalkyle en C₃-C₆,
phényle non substitué ou substitué, hétérocyclyle non substitué ou substitué, ou cycloalkyle en C₃-C₆ non substitué ou substitué, cycloalcényle en C₃-C₆ non substitué ou substitué, ou
phényle non substitué ou substitué, et R⁰ et R^{#} peuvent former, conjointement avec l'atome de N du groupe NR^{#}R⁰, un radical hétérocyclyle de préférence de 3 à 6 chaînons, qui est non substitué ou substitué, de préférence par un ou plusieurs radicaux pris dans le groupe constitué par un atome d'halogène, un radical alkyle C₁-C₆, alkoxy C₁-C₆, (alkoxy C₁-C₆) carbonyle, haloalkyle C₁-C₆ et oxo,
R³ représente un atome d'hydrogène, un radical alkyle en C₁-C₁₂, alcényle en C₂-C₁₂ ou alcynyle en C₂-C₁₂, chacun des 3 derniers radicaux cités étant non substitué ou substitué par un ou plusieurs radicaux pris dans le groupe constitué par un atome d'halogène, un radical alkoxy en C₁-C₆, haloalkoxy en C₁-C₆, (alkyl en C₁-C₆) thio, halo (alkyl en C₁-C₆) thio, (alkyl en C₁-C₆)-sulfinyle, halo (alkyl en C₁-C₆) sulfinyle, (alkyl en C₁-C₆) sulfonyle, halo (alkyl en C₁-C₆)-sulfonyle,
(alkoxy en C₁-C₆) carbonyle, halo (alkoxy en C₁-C₆) carbonyle, CONR⁶R⁷, SO₂NR⁶R⁷, CN, OH, cycloalkyle en C₃-C₆, NR⁸R⁹, phényle non substitué ou substitué, hétérocyclyle non substitué ou substitué, ou cycloalkyle en C₃-C₆ non substitué ou substitué, cycloalcényle en C₃-C₆ non substitué ou substitué, hétérocyclyle non substitué ou substitué de préférence de 3 à 6 chaînons, ou phényle non substitué ou substitué, et
R² et R³ peuvent former, conjointement avec l'atome de N (N¹) du groupe NR²R³, un radical hétérocyclyle, de préférence de 3 à 6 chaînons, qui est non substitué ou substitué, de préférence par un ou plusieurs radicaux pris dans le groupe constitué par un atome d'halogène, un radical alkyle en C₁-C₆, alkoxy en C₁-C₆, (alkoxy en C₁-C₆)-carbonyle, haloalkyle en C₁-C₆ et oxo, le radical oxo ne se trouvant pas de préférence en position voisine de l'atome de N (N¹), et
R⁴ représentent, indépendamment les uns des autres, un atome d'halogène, un radical CN, alkyle en C₁-C₄, alkoxy en C₁-C₄, alcényle en C₂-C₆, alcynyle en C₂-C₆, alcényloxy en C₃-C₆, alcynyloxy en C₃-C₆, chacun des 6 derniers radicaux cités étant non substitué ou substitué par un ou plusieurs radicaux pris dans le groupe constitué par un atome d'halogène, un radical alkoxy en C₁-C₆, haloalkoxy en C₁-C₆, (alkyl en C₁-C₆)thio, halo (alkyl en C₁-C₆) thio, (alkyl en C₁-C₆) -sulfinyle, halo (alkyl en C₁-C₆) -sulfinyle, (alkyl en C₁-C₆) sulfonyle, halo (alkyl en C₁-C₆)-sulfonyle, (alkoxy en C₁-C₆) carbonyle, halo (alkoxy en C₁-C₆) carbonyle, CONR⁶R⁷, SO₂NR⁶R⁷, CN, OH, cycloalkyle en C₃-C₆, NR⁸R⁹, phényle non substitué ou substitué ou hétérocyclyle non substitué ou substitué, ou
cycloalkyle en C₃-C₆ non substitué ou substitué, cycloalcényle en C₃-C₆ non substitué ou substitué, hétérocyclyle non substitué ou substitué, de préférence de 3 à 6 chaînons, phényle non substitué ou substitué, (alkyl en C₁-C₄) carbonyle ou (alkoxy en C₁-C₄) carbonyle, chacun des deux derniers radicaux cités étant non substitué ou substitué dans la partie alkyle par un ou plusieurs atomes d'halogène, ou un radical de formule C(O)-NR'-R", C(S)-NR'-R", CR' =N-Q¹-R" , S(O)ₘ-Q¹-R" ", P (O)ₙ(-Q¹-R" "Q²-R"), NR'-Q¹-R" ou NR*'*"-N=CR*'*-R", R', R" et R*'*", indépendamment les uns des autres, représentent un atome d'hydrogène, un-radical acyle ou un radical hydrocarboné en C₁-C₁₀ non substitué ou substitué,
R"" représente un radical acyle carboné ou un radical hydrocarboné en C₁-C₁₀ non substitué ou substitué, et Q¹ et Q², indépendamment l'un de l'autre, représentent une liaison directe ou un groupe divalent de formule -0- ou -N(R⁺)-, R⁺ représente un atome d'hydrogène, un radical acyle ou un radical hydrocarboné en C₁-C₁₀ non substitué ou substitué, et m peut être = 0, 1, 2 ou 3, ainsi que n peut être = 0, 1 ou 2, et R' avec R", R⁺ avec R', R⁺ avec R" ou R⁺ avec R" " chacun peuvent former un radical hétérocyclyle, de préférence de 3 à 6 chaînons,
qui est non substitué ou substitué, de préférence par un ou plusieurs radicaux pris dans le groupe constitué par un atome d'halogène, un radical alkyle en C₁-C₆, alkoxy en C₁-C₆, (alkoxy en C₁-C₆) carbonyle, haloalkyle en C₁-C₆ et oxo,
1 est égal à 0, 1 ou 2, de préférence à 0 ou 1,
R⁵ représente H ou un radical alkyle en C₁-C₄ , qui est non substitué ou substitué,
R⁶ et R⁷, indépendamment l'un de l'autre, représentent H, un radical alkyle en C₁-C₆, alcényle en C₃-C₆, alcynyle en C₃-C₆, phényle non substitué ou substitué ou hétérocyclyle non substitué ou substitué ou
R⁶ et R⁷ peuvent former, conjointement avec l'atome de N du groupe NR⁶R⁷, un radical hétérocyclyle de 5 ou 6 chaînons, qui peut éventuellement contenir un ou plusieurs hétéroatomes supplémentaires pris dans le groupe constitué par les atomes de N, O et S et est non substitué ou substitué une ou plusieurs fois par des radicaux choisis dans le groupe constitué par un alkyle en C₁-C₄ et un oxo, et
R⁸ et R⁹, indépendamment l'un de l'autre, représentent un radical (alkyl en C₁-C₄) carbonyle, halo(alkyl en C₁-C₄)-carbonyle, (alkoxy en C₁-C₄)carbonyle ou (alkyl en C₁-C₄)sulfonyle, ou peuvent former, conjointement avec l'atome de N du groupe NR⁸R⁹, un radical hétérocyclyle de 5 ou 6 chaînons, qui peut éventuellement contenir un ou plusieurs hétéroatomes supplémentaires pris dans le groupe constitué par les atomes de N, 0 et S et est non substitué ou substitué une ou plusieurs fois par des radicaux pris dans le groupe constitué par les radicaux alkyle en C₁-C₄ et oxo, et
Q représente un atome de O, S ou un groupe NR*,
R* représente un radical alkyle en C₁-C_{4,} alcényle en C₃-C₄ ou alcynyle en C₃-C_{4,} chacun des trois derniers radicaux cités étant non substitué ou substitué par un ou plusieurs radicaux pris dans le groupe constitué par un atome d'halogène, un radical alkoxy en C₁-C₄ et (alkyl en C₁-C₄)-thio, et
R* et R¹ peuvent former, conjointement avec l'atome de N du groupe NR*R¹, un radical hétérocyclyle, qui est non substitué ou substitué par un ou plusieurs radicaux pris dans le groupe constitué par un atome d'halogène, alkyle en C₁-C_{6,} un radical alkoxy en C₁-C_{6,} (alkoxy en C₁-C₆)carbonyle, haloalkyle en C₁-C₆ et oxo,
X, Y, indépendamment l'un de l'autre, représentent H, un atome d'halogène, un radical alkyle en C₁-C₄, alkoxy en C₁-C₄, (alkyl en C₁-C₄) thio, chacun des trois derniers radicaux cités étant non substitué ou substitué par un ou plusieurs radicaux pris dans le groupe constitué par un atome d'halogène, un radical alkoxy en C₁-C₄ et (alkyl en C₁-C₄)thio, mono- ou di - (alkyl en C₁-C₄)amino, alcényle en C₃-C₅, alcényloxy en C₃-C₅, alcynyle en C₃-C₅ ou alcynyloxy en C₃-C₅, et les radicaux R¹, R² R³ et R⁴, y compris les substituants, présentent jusqu'à 20 atomes de carbone,
W représente un atome d'oxygène ou de soufre,
V, Z, indépendamment l'un de l'autre, représentent un groupe CH ou un atome de N,

2. Composé de formule (I) ou ses sels selon la revendication 1, où
R¹ représente H, un radical alkyle en C₁-C₆, alcényle en C₃-C₆ ou alcynyle en C₃-C₆, chacun des trois derniers radicaux cités étant non substitué ou substitué par un ou plusieurs radicaux pris dans le groupe constitué par un atome d'halogène, un radical phényle, alkoxy en C₁-C₄, (alkyl en C₁-C₄) thio, cycloalkyle en C₃-C₆, hétérocyclyle de 3 à 6 chaînons et (alkoxy en C₁-C₄)carbonyle, ou cycloalkyle en C₃-C₆ ou hétérocyclyle de 3 à 6 chaînons, chacun des deux derniers radicaux cités étant non substitué ou substitué par un ou plusieurs radicaux pris dans le groupe constitué par un atome d'halogène, un radical alkyle en C₁-C₄ et alkoxy en C₁-C₄,
R² représente un groupe de formule R⁰-Q⁰-, dans laquelle
R° représente un atome d'hydrogène, un radical alkyle en C₁-C₈, alcényle en C₃-C₈ ou alcynyle en C₃-C₈, chacun des 3 derniers radicaux cités étant non substitué ou substitué par un ou plusieurs radicaux pris dans le groupe constitué par un atome d'halogène, un radical alkoxy en C₁-C₄ , haloalkoxy en C₁-C₄ , (alkyl en C₁-C₄)thio, halo(alkyl en C₁-C₄)thio, (alkyl en C₁-C₄)-sulfinyle, halo (alkyl en C₁-C₄)-sulfinyle, (alkyl en C₁-C₄) sulfonyle, halo (alkyl en C₁-C₉)-sulfonyle, (alkoxy en C₁-C₆)-carbonyle, CONR⁶R⁷, SO₂NR⁶R⁷, CN, OH, cycloalkyle en C₃-C₆, NR⁸R⁹ et phényle, qui est non substitué ou substitué par un ou plusieurs radicaux pris dans le groupe constitué par un atome d'halogène, un radical alkyle en C₁-C₄, haloalkyle en C₁-C₄, alkoxy en C₁-C₄ , haloalkoxy en C₁-C₄ , (alkyl en C₁-C₄)thio, (alkyl en C₁-C₄)-sulfinyle, (alkyl en C₁-C₄)-sulfonyle, NR⁸R⁹, (alkoxy en C₁-C₄) carbonyle, (alkyl en C₁-C₄) -carbonyle, phényle, (alkyl en C₁-C₄) -carbonyle, CN et NO₂, ou hétérocyclyle de 3 à 6 chaînons, qui est non substitué ou substitué par un ou plusieurs radicaux pris dans le groupe constitué par un atome d'halogène, un radical alkyle en C₁-C₄, haloalkyle en C₁-C₄, alkoxy en C₁-C₄, haloalkoxy en C₁-C₄, (alkyl en C₁-C₄)thio, (alkyl en C₁-C₄)sulfinyle, (alkyl en C₁-C₄) sulfonyle, NR⁸R⁹, (alkoxy en C₁-C₄)-carbonyle, (alkyl en C₁-C₄)carbonyle, phényle, CN et NO₂, ou cycloalkyle en C₃-C₆, qui est non substitué ou substitué par un ou plusieurs radicaux pris dans le groupe constitué par un atome d'halogène, un radical alkyle en C₁-C₄, haloalkyle en C₁-C₄, alkoxy en C₁-C₄, haloalkoxy en C₁-C₄, (alkoxy en C₁-C₄)-carbonyle, CN, OH et phényle, ou
cycloalcényle en C₃-C₆, qui est non substitué ou substitué par un ou plusieurs radicaux pris dans le groupe constitué par un atome d'halogène, un radical alkyle en C₁-C₄, haloalkyle en C₁-C₄, alkoxy en C₁-C₄ et (alkoxy en C₁-C₄)carbonyle, ou phényle, qui est non substitué ou substitué par un ou plusieurs radicaux du groupe constitué par un atome d'halogène, un radical alkyle en C₁-C₄, haloalkyle en C₁-C₄, alkoxy en C₁-C₄, haloalkoxy en C₁-C₄, (alkyl en C₁-C₄)thio, (alkyl en C₁-C₄)-sulfinyle, (alkyl en C₁-C₄)-sulfonyle, NR⁸R⁹, (alkoxy en C₁-C₄)-carbonyle, (alkyl en C₁-C₄)-carbonyle, phényle, CN et NO₂, et
Q⁰ représente un liaison directe ou un groupe divalent de formule -O- ou -NR^{#}, où R^{#} représente un atome d'hydrogène ou un radical alkyle en C₁-C₄ non substitué ou substitué,
R³ représente un atome d'hydrogène, un radical alkyle en C₁-C₈, alcényle en C₃-C₈ ou alcynyle en C₃-C₈, chacun des 3 derniers radicaux cités étant non substitué ou substitué par un ou plusieurs radicaux pris dans le groupe constitué par un atome d'halogène, un radical alkoxy en C₁-C₄, haloalkoxy en C₁-C₄, (alkyl en C₁-C₄) thio, halo(alkyl en C₁-C₄) thio, (alkyl en C₁-C₄) sulfinyle, halo (alkyl en C₁-C₄) -sulfinyle, (alkyl en C₁-C₄)sulfonyle, halo (alkyl en C₁-C₄)-sulfonyle, (alkoxy en C₁-C₆) carbonyle, CONR⁶R⁷, SO₂NR⁶R⁷, CN, OH, cycloalkyle en C₃-C₆, NR⁸R⁹ et phényle, qui est non substitué ou substitué par un ou plusieurs radicaux pris dans le groupe constitué par un atome d'halogène, un radical alkyle en C₁-C₄, haloalkyle en C₁-C₄, alkoxy en C₁-C₄, haloalkoxy en C₁-C₄, (alkyl en C₁-C₄) thio, (alkyl en C₁-C₄)-sulfinyle, (alkyl en C₁-C₄) sulfonyle, NR⁸R⁹, (alkoxy en C₁-C₄)carbonyle, (alkyl en C₁-C₄) -carbonyle, phényle, (alkyl en C₁-C₄) carbonyle, CN et NO₂, ou hétérocyclyle, de préférence de 3 à 6 chaînons, qui est non substitué ou substitué par un ou plusieurs radicaux pris dans le groupe constitué par un atome d'halogène, un radical alkyle en C₁-C₄, haloalkyle en C₁-C₄, alkoxy en C₁-C₄, haloalkoxy en C₁-C₄, (alkyl en C₁-C₄) thio, (alkyl en C₁-C₄) sulfinyle, (alkyl en C₁-C₄) sulfonyle, NR⁸R⁹, (alkoxy en C₁-C₄) -carbonyle, (alkyl en C₁-C₄) carbonyle, phényle, CN et N0₂, ou cycloalkyle en C₃-C₆, qui est non substitué ou substitué par un ou plusieurs radicaux pris dans le groupe constitué par un atome d'halogène, un radical alkyle en C₁-C₄, haloalkyle en C₁-C₄, alkoxy en C₁-C₄, haloalkoxy en C₁-C₄, (alkoxy en C₁-C₄)-carbonyle, CN, OH et phényle, ou
cycloalcényle en C₃-C₆, qui est non substitué ou susbstitué par un ou plusieurs radicaux pris dans le groupe constitué par un atome d'halogène, un radical alkyle en C₁-C_{4,} haloalkyle en C₁-C_{4,} alkoxy en C₁-C₄ et (alkoxy en C₁-C₄)carbonyle, ou
phényle, qui est non substitué ou substitué par un ou plusieurs radicaux pris dans le groupe constitué par un atome d'halogène, un radical alkyle en C₁-C₄, haloalkyle en C₁-C₄, alkoxy en C₁-C₄, haloalkoxy en C₁-C₄ , (alkyl en C₁-C₄) thio, (alkyl en C₁-C₄) sulfinyle, (alkyl en C₁-C₄)-sufonyle, NR⁸R⁹, (alkoxy en C₁-C₄)-carbonyle, (alkyl en C₁-C₄) carbonyle, phényle, CN et NO₂, et
R² et R³ peuvent former, conjointement avec l'atome de N (N¹), un radical hétérocyclyle de 3-6 chaînons, qui est saturé, insaturé ou hétéroaromatique et peut contenir, en plus de l'atome de N (N¹), un ou deux hétéroatomes pris dans le groupe constitué par les atomes de N, 0 et S et est non substitué ou substitué par un ou plusieurs radicaux pris dans le groupe constitué par les radicaux alkyle en C₁-C₆, alkoxy en C₁-C₃, halogène, (alkoxy en C₁-C₃)-carbonyle, haloalkyle en C₁-C₃ et oxo, le radical oxo ne se trouvant de préférence pas en position voisine de l'atome N (N¹),
R⁴ représente un atome d'halogène, un radical CN, alkyle en C₁-C_{4,} alkoxy en C₁-C₄, alcényle en C₂-C₆, alcynyle en C₂-C₆, alcényloxy en C₃-C₆, alcynyloxy en C₃-C₆,
chacun des 6 derniers radicaux cités étant non substitué ou substitué par un ou plusieurs radicaux pris dans le groupe constitué par un atome d'halogène, un radical alkoxy en C₁-C₆, haloalkoxy en C₁-C₆, (alkyl en C₁-C₆) thio, halo (alkyl en C₁-C₆)thio, (alkyl en C₁-C₆)-sulfinyle, halo (alkyl en C₁-C₆)sulfinyle, (alkyl en C₁-C₆) sulfonyle, halo(alkyl en C₁-C₆)-sulfonyle, (alkoxy en C₁-C₆) carbonyle, halo (alkoxy en C₁-C₆) carbonyle, CONR⁶R⁷, SO₂NR⁶R⁷, CN, OH, cycloalkyle en C₃-C₆, NR⁸R⁹, phényle non substitué ou substitué, hétérocyclyle non substitué ou substitué, ou
cycloalkyle en C₃-C₆ non substitué ou substitué, cycloalcényle en C₃-C₆ non substitué ou substitué, hétérocyclyle non substitué ou substitué de 3 à 6 chaînons, phényle non substitué ou substitué ou (alkyl en C₁-C₄)-carbonyle ou (alkoxy en C₁-C₄) carbonyle, chacun des deux derniers radicaux cités étant non substitué ou substitué dans la partie alkyle par un ou plusieurs atomes d'halogène, ou un radical de formule C(O)-NR'-R", C(S)-NR'-R", CR'=N-Q¹-R" , NR' -Q¹-R" ou NR'"-N=CR'-R", R' , R" et R'", indépendamment les uns des autres, représentent un atome d'hydrogène, un radical acyle ou un radical hydrocarboné en C₁-C₁₀ non substitué ou substitué, et Q¹ et Q², indépendamment l'un de l'autre, représentent une liaison directe ou un groupe divalent de formule -O- ou -N(R⁺)-, R⁺ représente un atome d'hydrogène, un radical acyle ou un radical alkyle en C₁-C₄ non substitué ou substitué, et R' avec R" ou R⁺ avec R' ou R⁺ avec R" peuvent former chacun un radical hétérocyclyle de 3 à 6 chaînons, qui est non substitué ou substitué, de préférence par un ou plusieurs radicaux pris dans le groupe constitué par un atome d'halogène, un radical alkyle en C₁-C₆, alkoxy en C₁-C₆, (alkoxy en C₁-C₆) carbonyle, haloalkyle en C₁-C₆ et oxo,
l est égal à 0 ou 1,
R⁶ et R⁷, indépendamment l'un de l'autre, représentent H, un radical alkyle en C₁-C₄, alcényle en C₃-C₄, alcynyle en C₃-C₄ ou phényle, qui est non substitué ou substitué par un ou plusieurs radicaux pris dans le groupe constitué par un atome d'halogène, un radical alkyle en C₁-C₄, haloalkyle en C₁-C₄, alkoxy en C₁-C₄ , (alkyl en C₁-C₄)thio, (alkyl en C₁-C₄)-sulfonyle, (alkoxy en C₁-C₄) carbonyle, CN et NO₂, ou
R⁶ et R⁷ peuvent former, conjointement avec l'atome de N du groupe NR⁶R⁷, un radical hétérocyclyle de 5 ou 6 chaînons, qui peut éventuellement contenir un ou plusieurs hétéroatomes pris dans le groupe constitué par les atomes de N, O et S et est non substitué ou substitué une ou plusieurs fois par des radicaux pris dans le groupe constitué par un radical alkyle en C₁-C₄ et oxo,
R⁸ et R⁹ représentent un radical (alkyl en C₁-C₄)-carbonyle, halo(alkyl en C₁-C₄) carbonyle, (alkoxy en C₁-C₄)carbonyle ou (alkyl en C₁-C₄)sulfonyle, ou peuvent former, conjointement avec l'atome de N, un radical hétérocyclyle de 5 ou 6 chaînons, qui peut éventuellement contenir un ou plusieurs hétéroatomes pris dans le groupe constitué par les atomes de N, 0 et S et est non substitué ou substitué une ou plusieurs fois par des radicaux pris dans le groupe constitué par les radicaux alkyle en C₁-C₄ et oxo.

3. Composé de formule (I) ou ses sels selon l'une des revendications 1 ou 2, où
R¹ représente un radical alkyle en C₁-C₆, qui est non substitué ou substitué par un ou plusieurs radicaux choisis dans le groupe constitué par un atome d'halogène et un alkoxy en C₁-C₄, ou un radical 3-oxétanyle, alcényle en C₃-C₄ ou alcynyle en C₃-C₄,
R² représente H, un radical alkyle en C₁-C₆, alcényle en C₃-C₆, alcynyle en C₃-C₆, chacun des trois derniers radicaux cités étant non substitué ou substitué par un ou plusieurs radicaux pris dans le groupe constitué par un atome d'halogène, un radical alkoxy en C₁-C₄, (alkyl en C₁-C₄)thio, (alkyl en C₁-C₄)sulfonyle, (alkoxy en C₁-C₄)carbonyle, cycloalkyle en C₃-C₆, CN et OH, ou cycloalkyle en C₃-C₆, qui est non substitué ou substitué par un ou plusieurs radicaux pris dans le groupe constitué par un atome d'halogène, un radical alkyle en C₁-C₄, alkoxy en C₁-C₄, (alkoxy en C₁-C₄) carbonyle, CN et OH, ou cycloalcényle en C₃-C₆, alkoxy en C₁-C₄, alcényloxy en C₁-C₄, (alkyl en C₁-C₄) amino ou di(alkyl en C₁-C₄)amino et
R³ représente H, un radical alkyle en C₁-C₆, alcényle en C₃-C₆, alcynyle en C₃-C₆, chacun des trois derniers radicaux cités étant non substitué ou substitué par un ou plusieurs radicaux pris dans le groupe constitué par un atome d'halogène, un radical alkoxy en C₁-C₄, (alkyl en C₁-C₄)thio, (alkyl en C₁-C₄)sulfonyle, (alkoxy en C₁-C₄)carbonyle, cycloalkyle en C₃-C₆, CN et OH, ou cycloalkyle en C₃-C₆, qui est non substitué ou substitué par un ou plusieurs radicaux pris dans le groupe constitué par un atome d'halogène, un radical alkyle en C₁-C₄, alkoxy en C₁-C₄, (alkoxy en C₁-C₄) carbonyle, CN et OH, ou cycloalcényle en C₃-C₆ ou
R² et R³ peuvent former, conjointement avec l'atome de N (N¹), un radical hétérocyclyle de 3-6 chaînons, qui est saturé, insaturé ou hétéroaromatique, qui peut contenir en plus de l'atome de N (N¹) un ou deux hétéroatomes pris dans le groupe constitué par les atomes de N, 0 et S et est non substitué ou substitué par un ou plusieurs radicaux pris dans le groupe constitué par un atome d'halogène, un radical alkyle en C₁-C₆, alkoxy en C₁-C₃, (alkoxy en C₁-C₃)-carbonyle et oxo, le radical oxo ne se trouve pas de préférence en position voisine de l'atome de N (N¹), et
R⁴ représente un radical alkyle en C₁-C₄, haloalkyle en C₁-C₄, alkoxy en C₁-C₄ ou halogène,
1 est égal à 0 ou 1, de préférence à 0,
R⁵ représente H ou un méthyle,
Q représente O ou NR*,
R* représente H ou un alkyle en C₁-C₄,
X et Y, indépendamment l'un de l'autre, représentent un radical alkyle en C₁-C₄, alkoxy en C₁-C₄, chacun des deux derniers radicaux cités étant non substitué ou substitué par un ou plusieurs atomes d'halogènes, ou (alkyl en C₁-C₄)thio, halogènes ou mono- ou di(alkyl en C₁-C₂)amino, et
W représente un atome d'oxygène.

4. Composé de formule (I) ou ses sels selon l'une des revendications 1 à 3, où
R¹ représente un radical alkyle en C₁-C₃, allyle ou propargyle,
R² représente H, un radical alkyle en C₁-C₄, alcényle en C₃-C₅, alcynyle en C₃-C₅, cycloalkyle en C₃-C₆ ou cycloalcényle en C₃-C₆,
R³ représente H, un radical alkyle en C₁-C₄, alcényle en C₃-C₅, alcynyle en C₃-C₅, cycloalkyle en C₃-C₆ ou cycloalcényle C₃-C₆, ou
R² et R³ peuvent former, conjointement avec l'atome de N (N¹), un radical hétérocyclyle de 3-6 chaînons, qui est saturé, insaturé ou hétéroaromatique, qui peut contenir, en plus de l'atome de N (N¹), un ou deux hétéroatomes du groupe constitué par les atomes de N, O et S et est non substitué ou substitué par un ou plusieurs radicaux alkyle en C₁-C₆,
R⁴ représente un radical alkyle en C₁-C₃ ou un atome d'halogène,
1 est égal à 0 ou 1, de préférence à 0,
Q représente un atome de 0 ou un groupe NR*,
R* représente un radical alkyle en C₁-C₃,
X représente un radical alkyle en C₁-C₂, alkoxy en C₁-C₂, (alkyl en C₁-C₂)thio, haloalkyle en C₁-C₂ ou haloalkoxy en C₁-C₂,
Y représente un radical alkyle en C₁-C₂, alkoxy en C₁-C₂, halogène, NHCH₃ ou N(CH₃)₂,
V représente un groupe CH ou un atome de N, de préférence un atome de N et
Z représente un groupe CH ou un atome de N.

5. Procédé pour la préparation de composés de formule (I) ou leurs sels, tels que définis dans une ou plusieurs des revendications 1 à 4, où
a) on fait réagir un composé de formule (II) 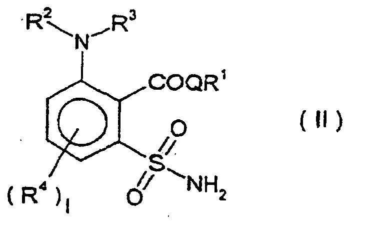 sur un carbamate hétérocyclique de formule (III) 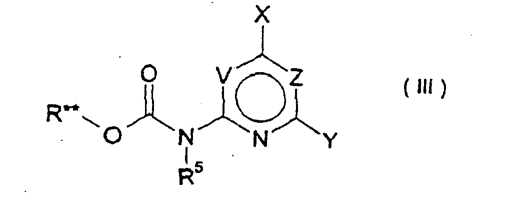 où R** représente un radical hydrocarboné en C₁-C₂₀ substitué ou non substitué, ou
b) on fait réagir un carbamate de sulfonyle de formule (IV) 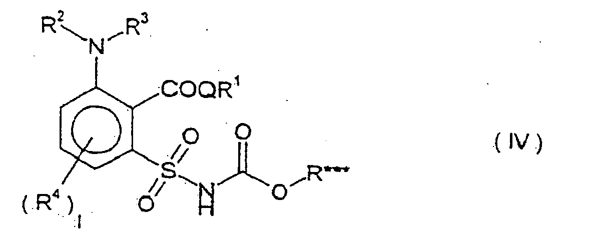 où R*** représente un radical hydrocarboné en C₁-C₂₀ substitué ou non substitué, sur un hétérocycle aminé de formule (V) 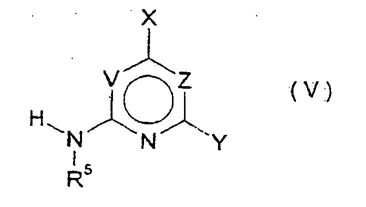 ou
c) on fait réagir un isocyanate de sulfonyle de formule (VI) 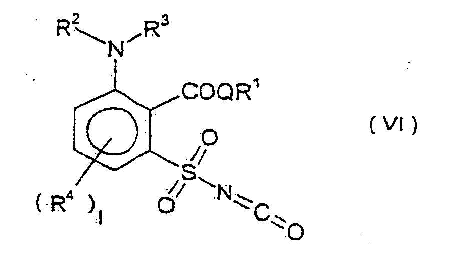 sur un hétérocycle aminé de formule (V) ou
d) on fait réagir un sulfonamide de formule (II) sur un (thio)-isocyanate de formule (VII) 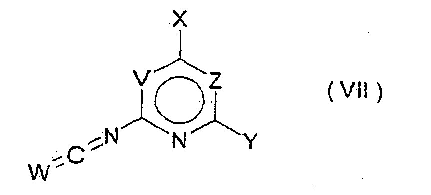 en présence d'une base ou
e) on fait réagir un hétérocycle aminé de formule (V) d'abord sous catalyse par une base sur un ester de l'acide carbonique, et on fait réagir l'intermédiaire formé dans une réaction en un pot sur un sulfonamide de formule (II) (voir variante a), ou
f) on fait réagir une phénysulfonylurée de formule (VIII) 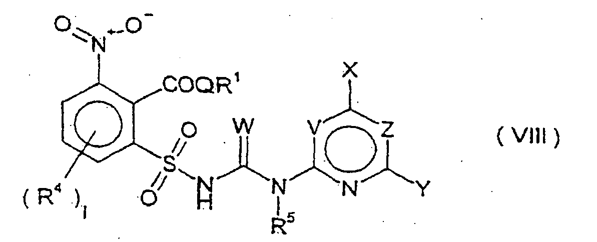 par réduction du groupe nitro, et éventuellement par une réaction ultérieure de la fonction libérée hydroxylamine ou amine, en une sulfonylurée de formule (I),
dans les formules (II)-(VIII) les radicaux, les groupes et les indices R¹-R⁵, Q, V, W, X, Y, Z et 1 sont définis comme dans la formule (I) selon la revendication 1.

6. Agents herbicides et régulateurs de croissance de plantes contenant a) au moins un composé de formule (I) ou son sel selon l'une des revendications 1 à 4 et b) des formulants usuels dans la phytoprotection.

7. Procédé pour la lutte contre les plantes adventices ou pour la régulation de la croissance de plantes, en appliquant une quantité efficace d'au moins un composé de formule (I) ou son sel selon l'une des revendications 1 à 4, aux plantes adventices ou aux plantes, à leurs germes ou à la surface de leur croissance.

8. Utilisation des composés de formule (I) ou leurs sels selon l'une des revendications 1 à 4 en tant qu'herbicides ou régulateurs de croissance de plantes.

9. Utilisation selon la revendication 8, en utilisant les composés de formule (I) ou leurs sels pour la lutte contre les plantes adventices ou pour la régulation de la croissance de plantes dans les cultures de plantes utiles ou de plantes ornementales.

10. Utilisation selon la revendication 9, les plantes de culture étant des plantes de culture transgéniques.

11. Composé de formule (II*) 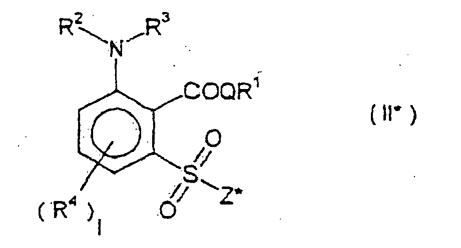 où Z* = NH₂, NHCOOR***, NCO ou NH-tert-butyle et R¹-R⁴, 1 et Q sont définis comme dans la formule (I) selon la revendication 1 et R*** est défini comme dans la formule (IV) selon la revendication 5, et les composés a), b) et c) suivants étant exclus :
a) 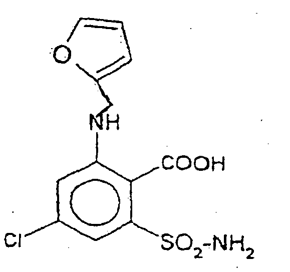
b) 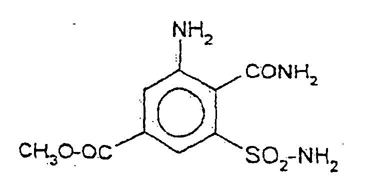
c)

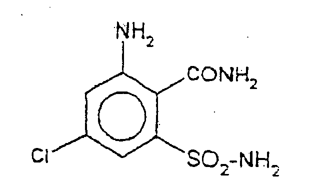

12. Utilisation d'un composé de formule (II*) comme défini dans la revendication 11 pour la préparation d'un composé de formule (I) selon la revendication 1.

13. Composé de formule (X*) 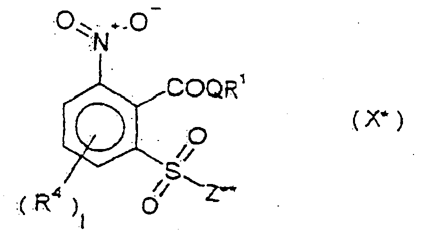 où Z** = NH₂, NHCOOR***, NH-tert-butyle ou Cl et R¹, R⁴, 1 et Q sont définis comme dans la formule (I) selon la revendication 1 et R*** comme dans la formule (IV) selon la revendication 5, et les composés dans lesquels Z** = Cl, 1 = 0, Q = 0 et R¹ = H, méthyle, éthyle ou allyle, ainsi que les composés dans lesquels Z** = NH₂, 1 = 0, Q = 0 et R¹ = H, sont exclus.

14. Utilisation d'un composé de formule (X*) défini comme dans la revendication 13, où Z** est égal à NH₂, NHCOOR*** ou NH-tert-butyle et R*** est défini comme dans la formule (IV) selon la revendication 5, pour la préparation d'un composé de formule (II*) défini comme dans la revendication 11, où Z* est égal à NH₂, NHCOOR*** ou NH-tert-butyle.

15. Composés de formule (VIII) 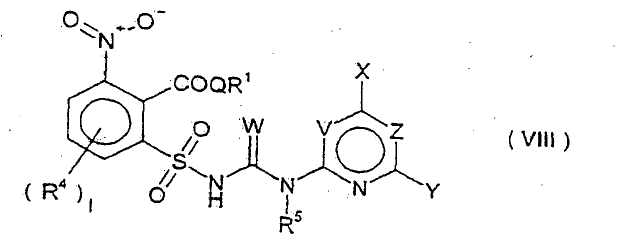 dans laquelle R¹, R⁴, R⁵, Q, V, W, X, Y, Z et 1 sont définis comme dans la revendication 1.

16. Utilisation d'un composé de formule (VIII) défini comme dans la revendication 15, pour la préparation d'un composé de formule (I) défini comme dans la revendication 1.
